# EUROPEAN PATENT APPLICATION

(11) **EP 4 640 178 A2**
(43) Date of publication of application: **29.10.2025**
(21) Application number: 25188121.5
(22) Date of filing: 01.05.2021
(51) Int. Cl.: A61C 7/28

(54) **METHODS FOR DESIGNING AN ORTHODONTIC APPLIANCE**

(30) Priority: 02.05.2020 US 202062704293 P; 02.05.2020 US 202062704295 P
(62) Divisional of application: 21727058.6
(71) Applicant: Brius Technologies, Inc., Carrollton, TX 75006 (US)
(72) Inventor: ROEIN PEIKAR, Seyed Mehdi, Addison, TX 75001 (US); WRATTEN, James Sylvester, Waterville, NY 13480 (US)
(74) Representative: Forresters IP LLP

(57) **Abstract**

Orthodontic appliances, systems, and methods are disclosed herein. In some examples, an orthodontic appliance can include an anchor configured to be adjacent to the patient's teeth and a plurality of arms extending away from and coupled to the anchor, the arms configured to be secured to the patient' s teeth. According to embodiments of the present technology, methods for determining a design of an orthodontic appliance can include virtually deforming an appliance digital model based on an anatomy digital model and, based on an output of the virtual deformation, modifying one or more digital models or producing a final design of an orthodontic appliance. If one or more digital models is modified, the method is repeated until a final design is produced.

## Description

### CROSS-REFERENCE TO RELATED APPLICATIONS

The present application claims the benefit of priority to U.S. Provisional Application No. 62/704,293, filed May 2, 2020, and U.S. Provisional Application No. 62/704,295, filed May 2, 2020, both of which are incorporated by reference herein in their entireties.

The present application is related to the following applications, each of which is incorporated by reference herein in its entirety: U.S. Provisional Patent Application No. 62/956,290, titled ORTHODONTIC APPLIANCES AND ASSOCIATED SYSTEMS AND METHODS OF USE, filed January 1, 2020; U.S. Patent Application No. 16/865,323, titled DENTAL APPLIANCES, SYSTEMS AND METHODS, filed May 2, 2020; International Patent Application No. PCT/US20/31211, titled DENTAL APPLIANCES, SYSTEMS AND METHODS, filed May 2, 2020; U.S. Patent Application No. 15/929,443, titled DENTAL APPLIANCES AND ASSOCIATED SYSTEMS AND METHODS OF USE, filed May 2, 2020; U.S. Patent Application No. 15/929,444, titled DENTAL APPLIANCES AND ASSOCIATED SYSTEMS AND METHODS OF USE, filed May 2, 2020; International Patent Application No. PCT/US20/70017, titled DENTAL APPLIANCES AND ASSOCIATED SYSTEMS AND METHODS OF USE, filed May 2, 2020; U.S. Patent Application No. 15/929,442, titled DENTAL APPLIANCES AND ASSOCIATED METHODS OF MANUFACTURING, filed May 2, 2020; and International Patent Application No. PCT/US20/70016, titled DENTAL APPLIANCES AND ASSOCIATED METHODS OF MANUFACTURING, filed May 2, 2020.

### TECHNICAL FIELD

The present technology relates to the field of orthodontics and, more particularly, to devices, systems, and methods for designing and manufacturing orthodontic appliances.

### BACKGROUND

A common objective in orthodontics is to move a patient's teeth to positions where the teeth function optimally and aesthetically. To move the teeth, the orthodontist begins by obtaining multiple scans and/or impressions of the patient's teeth to determine a series of corrective paths between the initial positions of the teeth and the desired ending positions. The orthodontist then fits the patient to one of two main appliance types: braces or aligners.

Traditional braces consist of brackets and an archwire placed across a front side of the teeth, with elastic ties or ligature wires to secure the archwire to the brackets. In some cases self-ligating brackets may be used in lieu of ties or wires. The shape and stiffness of the archwire as well as the archwire-bracket interaction governs the forces applied to the teeth and thus the direction and degree of tooth movement. To exert a desired force on the teeth, the orthodontist often manually bends the archwire. The orthodontist monitors the patient's progress through regular appointments, during which the orthodontist visually assesses the progress of the treatment and makes manual adjustments to the archwire (such as new bends) and/or replaces or repositions brackets. The adjustment process is both time consuming and tedious for the patient and more often than not results in patient discomfort for several days following the appointment. Moreover, braces are not aesthetically pleasing and make brushing, flossing, and other dental hygiene procedures difficult.

Aligners comprise clear, removable, polymeric shells having cavities shaped to receive and reposition teeth to produce a final tooth arrangement. Dubbed "invisible braces," aligners offer patients significantly improved aesthetics over braces. Aligners do not require the orthodontists to bend wires or reposition brackets and are generally more comfortable than braces. However, unlike braces, aligners cannot effectively treat all malocclusions. Certain tooth repositioning steps, such as extrusion, translation, and certain rotations, can be difficult or impossible to achieve with aligners. Moreover, because the aligners are removable, success of treatment is highly dependent on patient compliance, which can be unpredictable and inconsistent.

Lingual braces are an alternative to aligners and traditional (buccal) braces and have been gaining popularity in recent years. Two examples of existing lingual braces are the Incognito^{™} Appliance System (3M United States) and INBRACE^{®} (Swift Health Systems, Irvine, California, USA), each of which consists of brackets and an archwire placed on the lingual, or tongue side, of the teeth. In contrast to traditional braces, lingual braces are virtually invisible, and, unlike aligners, lingual braces are fixed to the patient's teeth and force compliance. These existing lingual technologies, however, also come with several disadvantages. Most notably, conventional lingual appliances still rely on a bracket-archwire system to move the teeth, thus requiring multiple office visits and painful adjustments. For example, lingual technologies have a relatively short inter-bracket distance, which generally makes compliance of the archwire stiffer. As a result, the overall lingual appliance is more sensitive to archwire adjustments and causes more pain for the patient. Moreover, the lingual surfaces of the appliance can irritate the tongue and impact speech, and make the appliance difficult to clean.

Therefore, a need exists for improved orthodontic appliances.

### SUMMARY

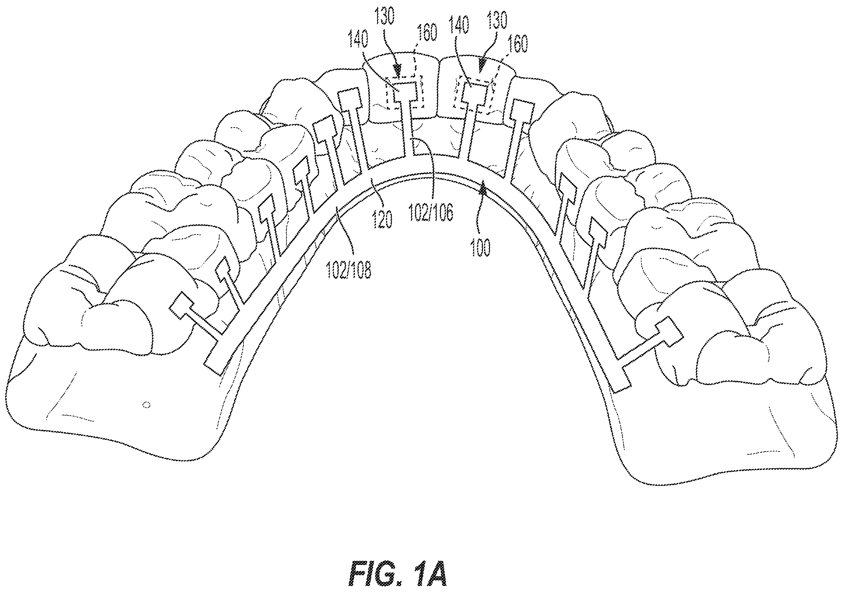

The subject technology is illustrated, for example, according to various aspects described below, including with reference to FIGS. 1A-25. Various examples of aspects of the subject technology are described as numbered clauses (1, 2, 3, etc.) for convenience. These are provided as examples and do not limit the subject technology.
Clause 1. A method for designing an orthodontic appliance comprising:
   obtaining an anatomy digital model representing a patient's gingiva and teeth in an arrangement;
   obtaining an appliance digital model representing an orthodontic appliance design configured to use with the patient's teeth;
   virtually deforming the appliance digital model into a configuration in which the appliance is coupled to the patient's teeth in the arrangement; and
   evaluating the deformed configuration of the appliance digital model.
Clause 2. The method of Clause Clause 1, wherein the orthodontic appliance comprises an appliance for repositioning one or more teeth of the patient.
Clause 3. The method of Clause 1 or Clause 2, wherein the orthodontic appliance comprises an anchor configured to be disposed adjacent the patient's teeth and one or more arms extending away from the anchor, each of the one or more arms being configured to couple to a respective one or more of the patient's teeth.
Clause 4. The method of any one Clause 1 to Clause 3, wherein the arrangement comprises an original tooth arrangement.
Clause 5. The method of any one Clause 1 to Clause 3, wherein the arrangement comprises an intermediate tooth arrangement.
Clause 6. The method of any one Clause 1 to Clause 3, wherein the arrangement comprises a final tooth arrangement.
Clause 7. The method of any one Clause 1 to Clause 6, wherein evaluating the deformed configuration comprises determining whether the deformed appliance digital model impinges on the gingiva.
Clause 8. The method of any one of Clause 1 to Clause 7, wherein evaluating the deformed configuration comprises evaluating relative positions of the appliance digital model and the gingiva.
Clause 9. The method of any one of Clause 1 to Clause 8, wherein evaluating the deformed configuration comprises determining whether appliance is spaced apart from gingiva by greater than a predetermined threshold.
Clause 10. The method of any one of Clause 1 to Clause 9, wherein evaluating the deformed configuration comprises determining whether any portion of the deformed appliance digital model exceeds an elastic strain limit.
Clause 11. The method of any one of Clause 1 to Clause 10, wherein evaluating the deformed configuration comprises determining a difference between a force and/or moment applied to the teeth by the deformed appliance and an intended force and/or moment.
Clause 12. The method of Clause 11, wherein evaluating the deformed configuration comprises determining whether the difference between a force and/or moment applied to the teeth by the deformed appliance and an intended force and/or moment exceeds a predetermined accuracy limit.
Clause 13. The method of any one Clause 1 to Clause 12, wherein evaluating the deformed configuration comprises determining if a force and/or moment applied to the teeth by the deformed appliance exceeds a predetermined maximum force and/or moment.
Clause 14. The method of any one of Clause 1 to Clause 13, further comprising, based on the evaluation, modifying the appliance digital model.
Clause 15. The method of Clause 14, wherein modifying the appliance digital model comprises changing a configuration of at least one arm of the appliance digital model.
Clause 16. The method of Clause 14 or Clause 15, wherein modifying the appliance digital model comprises changing a geometry of a shape-set configuration for the appliance digital model.
Clause 17. The method of any one of Clause 14 to Clause 16, wherein modifying the appliance digital model comprises changing a configuration of an anchor of the appliance digital model.
Clause 18. The method of any one of Clause 14 to Clause 17, further comprising, after modifying the appliance digital model:
   virtually deforming the modified appliance digital model into a configuration in which the appliance is mated to the patient's teeth; and
   evaluating the deformed configuration of the modified appliance digital model.
Clause 19. The method of any one of Clause 1 to Clause 18, wherein virtually deforming the appliance comprises performing a finite element analysis (FEA) using the appliance digital model.
Clause 20. A method for designing an orthodontic appliance for repositioning a tooth of a patient, the orthodontic appliance having an anchor and an arm extending away from the anchor, the method comprising:
   obtaining an anatomy digital model characterizing the patient's gingiva and teeth in an arrangement;
   obtaining an appliance digital model characterizing an orthodontic appliance design; and
   virtually deforming the appliance digital model based on the anatomy digital model.
Clause 21. The method of Clause 20, wherein virtually deforming the appliance model includes performing a finite element analysis (FEA).
Clause 22. The method of Clause 20 or Clause 21, further comprising obtaining an output from virtually deforming the appliance digital model based on the anatomy digital model.
Clause 23. The method of Clause 22, wherein the output is a deformed appliance digital model.
Clause 24. The method of Clause 22 or Clause 23, wherein the output comprises a position of a first portion of the appliance digital model corresponding to the anchor of the orthodontic appliance relative to a position of the patient's gingiva of the anatomy digital model.
Clause 25. The method of any one of Clause 22 to Clause 24, wherein the output comprises a measure of strain in the appliance digital model.
Clause 26. The method of any one of Clause 22 to Clause 25, further comprising determining if the output is greater than a predetermined threshold.
Clause 27. The method of any one of Clause 22 to Clause 26, further comprising determining if the output is less than a predetermined threshold.
Clause 28. The method of Clause Clause 26 or Clause 27, wherein the predetermined threshold is an elastic strain limit.
Clause 29. The method of Clause Clause 26 or Clause 27, wherein the predetermined threshold is a distance between the anatomy digital model and the appliance digital model.
Clause 30. The method of any one Clause 22 to Clause 29, further comprising modifying the appliance digital model based on the output.
Clause 31. The method of any one Clause 22 to Clause 30, further comprising modifying the anatomy digital model based on the output.
Clause 32. The method of any one of Clause 20 to Clause 31, wherein the arrangement is an original tooth arrangement.
Clause 33. The method of any one of Clause 20 to Clause 32, wherein the arrangement is a desired final tooth arrangement.
Clause 34. The method of any one of Clause 20 to Clause 33, wherein the arrangement is an intermediate tooth arrangement.
Clause 35. The method of any one of Clause 20 to Clause 34, wherein the appliance digital model comprises a planar appliance digital model virtually representing the orthodontic appliance in a substantially planar form.
Clause 36. The method of any one of Clause 20 to Clause 34, wherein the appliance digital model comprises an intended appliance digital model virtually representing a geometry of the orthodontic appliance in a shape-set form.
Clause 37. The method of any one of Clause 20 to Clause 34, wherein the appliance digital model comprises a deformed intended appliance digital model virtually representing the geometry of the orthodontic appliance in an installed form.
Clause 38. A method for designing an orthodontic appliance for repositioning a tooth of a patient, the orthodontic appliance having an anchor and at least one arm extending away from the anchor, the method comprising:
   obtaining a planar appliance digital model, the planar appliance digital model virtually representing the appliance in a substantially planar configuration;
   obtaining a heat treatment fixture digital model, the heat treatment fixture digital model characterizing a geometry of a heat treatment fixture for shape-setting an appliance;
   performing a first FEA using the planar appliance digital model and the heat treatment fixture digital model;
   obtaining an intended appliance digital model, the intended appliance digital model virtually representing the appliance in a three-dimensional configuration with a geometry based at least in part on the heat treatment fixture digital model;
   obtaining an original tooth arrangement (OTA) digital model, the OTA digital model virtually representing a patient's teeth and gingiva in an original arrangement;
   performing a second FEA using the intended appliance digital model and the OTA digital model; and
   obtaining a deformed intended appliance digital model and an analysis result.
Clause 39. The method of Clause 38, further comprising modifying the planar appliance digital model based on the analysis result.
Clause 40. The method of Clause 38 or Clause 39, further comprising modifying the heat treatment fixture digital model based on the analysis result.
Clause 41. The method of any one Clause 38 to Clause 40, wherein performing the first FEA comprises:
   discretizing at least one of the planar appliance digital model and the heat treatment fixture digital model into a plurality of finite elements and a plurality of nodes;
   assigning material properties to at least one of the planar appliance digital model and the heat treatment fixture digital model;
   defining a contact interaction between the planar appliance digital model and the heat treatment fixture digital model;
   assigning boundary conditions to at least one of the planar appliance digital model and the heat treatment fixture digital model;
   defining an analysis parameter; and
   running the FEA until an exit condition is reached.
Clause 42. The method of Clause 41, wherein assigning the boundary conditions includes assigning a non-zero displacement to an anchor portion of the planar appliance digital model.
Clause 43. The method of Clause 41 or Clause 42, wherein assigning the boundary conditions includes defining a relationship between an orientation of an arm of the planar appliance digital model and a base plane of a securing portion of the heat treatment fixture.
Clause 44. The method of Clause 43, wherein the arm of the planar appliance digital model is tangent to the base plane of the securing portion of the heat treatment fixture.
Clause 45. The method of any one Clause 41 to Clause 44, wherein assigning the boundary conditions includes assigning a displacement to an attachment portion of the planar appliance digital model.
Clause 46. The method of Clause 45, wherein the displacement assigned to the attachment portion has a magnitude of zero.
Clause 47. The method of Clause 45, wherein the displacement assigned to the attachment portion has a non-zero magnitude.
Clause 48. The method of any one of Clause 38 to Clause 47, wherein performing the second FEA comprises:
   discretizing at least one of the intended appliance digital model and the OTA digital model into a plurality of finite elements and a plurality of nodes;
   assigning material properties to at least one of the intended appliance digital model and the OTA digital model;
   defining a contact interaction between the intended appliance digital model and the OTA digital model;
   assigning boundary conditions to at least one of the intended appliance digital model and the OTA digital model;
   defining an analysis parameter; and
   running the FEA until an exit condition is reached.
Clause 49. A method for designing an orthodontic appliance for repositioning a tooth of a patient, the orthodontic appliance having an anchor and an arm extending away from the anchor, the method comprising:
   obtaining an OTA digital model of a patient's teeth and gingiva in an original arrangement, the OTA digital model comprising original position data of a tooth to be repositioned by the orthodontic appliance when installed in the patient's mouth;
   obtaining an FTA digital model characterizing the patient's teeth and gingiva in a desired final arrangement, the FTA digital model comprising final position data of the tooth;
   determining displacement data characterizing a displacement between the original position data of the tooth and the final position data of the tooth;
   obtaining a heat treatment fixture digital model based on the FTA digital model;
   obtaining a 3D template digital model based on the heat treatment fixture digital model comprising a first portion corresponding to the anchor of the orthodontic appliance in the treatment configuration and a second portion corresponding to the arm in the treatment configuration;
   obtaining a planar template digital model, wherein the planar template digital model is a substantially planar configuration of the 3D template digital model;
   obtaining a planar appliance digital model based on the planar template digital model;
   obtaining an intended appliance digital model, wherein the intended appliance digital model characterizes the orthodontic appliance in 3D configuration based on the heat treatment fixture digital model; and
   performing an FEA on the OTA and intended appliance digital models to deform the intended appliance digital model based on the displacement data.
Clause 50. The method of Clause 49, wherein obtaining the OTA digital model includes scanning the patient's teeth and gingiva.
Clause 51. The method of Clause 50, wherein scanning the patient's teeth and gingiva comprises optical scanning.
Clause 52. The method of Clause 50, wherein scanning the patient's teeth and gingiva comprises computed tomography scanning.
Clause 53. The method of Clause 50, wherein scanning the patient's teeth and gingiva comprises scanning an impression of the patient's teeth and gingiva.
Clause 54. The method of any one of Clause 49 to Clause 53, further comprising segmenting the OTA digital model into a plurality of digital models of each tooth and at least one gingiva.
Clause 55. The method of any one of Clauses Clause 49 to Clause 54, further comprising obtaining a securing member digital model representing a securing member, the securing member configured to be adhered to a surface of the tooth and detachably couple with a portion of the orthodontic appliance to secure the orthodontic appliance to the tooth.
Clause 56. The method of Clause 55, further comprising obtaining an OTA with securing member digital model comprising a combination of the OTA digital model and the securing member digital model, wherein the combination is based on a desired placement of the securing member on the patient's tooth when the orthodontic appliance is installed in the patient's mouth during treatment.
Clause 57. The method of Clause 55 or Clause 56, further comprising obtaining an FTA with securing member digital model comprising a combination of the FTA digital model and the securing member digital model, wherein the combination is based on a desired placement.
Clause 58. The method of Clause 56 or Clause 57, wherein the desired placement of the securing member is on a lingual surface of the patient's tooth.
Clause 59. The method of any one of Clause 49 to Clause 58, wherein the displacement data comprises three translations and three rotations.
Clause 60. The method of any one of Clause 49 to Clause 59, wherein obtaining the intended appliance digital model comprises performing an FEA with the planar appliance digital model and the heat treatment fixture digital model.
Clause 61. The method of any one of Clause 49 to Clause 60, wherein the method further comprises modifying the heat treatment fixture digital model based on the intended appliance digital model.
Clause 62. The method of Clause 59, wherein modifying the heat treatment fixture digital model comprises defining a tangent relationship between a gingival surface of the heat treatment fixture digital model and a gingival-facing surface of the intended appliance digital model.
Clause 63. The method of any one of Clause 61 to Clause 62, further comprising manufacturing the planar template digital model.
Clause 64. The method of any one of Clause 1 to Clause 63, further comprising manufacturing the heat treatment fixture digital model.
Clause 65. The method of any one of Clause 1 to Clause 64, further comprising manufacturing the intended appliance digital model.
Clause 66. An orthodontic appliance manufactured in accordance with a method of any one of the Clauses herein.
Clause 67. A heat treatment fixture manufactured in accordance with a method of any one of the Clauses herein.
Clause 68. A tangible, non-transitory computer-readable medium configured to store instructions that, when executed by one or more processors, cause the one or more processors to perform the method of any one of the Clauses herein.
Clause 69. A device comprising:
   one or more processors; and
   a tangible, non-transitory computer-readable medium configured to store instructions that, when executed by one or more processors, cause the one or more processors to perform the method of any one of the Clauses herein.
Clause 70. A method for determining an arrangement of an orthodontic device, the method comprising:
   obtaining position data corresponding to an original tooth arrangement (OTA) of a patient;
   obtaining position data corresponding to a first final tooth arrangement (FTA) of the patient, the first FTA differing from the OTA; and
   determining position data corresponding to a second FTA, the second FTA being based at least in part on the first FTA and a predetermined parameter, the second FTA differing from the first FTA,
   wherein the second FTA can be used to form a fixture and/or an orthodontic appliance, the appliance being configured to move teeth of the patient from the OTA toward the first FTA or the second FTA.
Clause 71. The method of Clause 70, further comprising manufacturing the fixture and/or the appliance according to at least the data corresponding to the second FTA.
Clause 72. The method of Clause 70 or Clause 71, wherein the appliance is configured to move teeth of the patient generally from the OTA to the first FTA or to the second FTA.
Clause 73. The method of any one of Clause 70 to Clause 72, wherein the appliance is configured to have an arrangement generally corresponding to the second FTA in which the appliance is in a substantially unloaded state.
Clause 74. The method of any one of Clauses 70 to 73, wherein the appliance is configured to have a first arrangement generally corresponding to the second FTA and a second arrangement generally corresponding to the OTA, the first arrangement corresponding to a substantially unloaded state and the second arrangement corresponding to a loaded state.
Clause 75. The method of any one of Clauses 70 to 74, wherein the predetermined parameter is associated with an expected movement of at least one tooth of the patient after repositioning of the at least one tooth via the appliance to the second FTA.
Clause 76. The method of Clause 75, wherein the expected movement is in at least one of the mesial-distal direction, lingual-facial direction, or occlusal-gingival direction.
Clause 77. The method of Clause 75 or Clause 76, wherein the expected movement is a rotation about an axis defined by at least one of the mesial-distal direction, lingual-facial direction, or occlusal-gingival direction.
Clause 78. The method of any one of Clause 70 to Clause 77, further comprising manufacturing the appliance such that the appliance in a substantially unloaded configuration generally corresponds to the second FTA, wherein the first FTA corresponds to a predetermined desired position of the patient's teeth.
Clause 79. The method of any one of Clause 70 to Clause 78, wherein the expected relapse corresponds to a positional difference between the first FTA and the second FTA.
Clause 80. A method for determining an arrangement of an orthodontic device, the method comprising:
   obtaining data corresponding to an original tooth arrangement (OTA) of a patient; and
   determining data corresponding to a final tooth arrangement (FTA) based on the OTA and a predetermined parameter,
   wherein the FTA can be used to form a fixture and/or an orthodontic appliance, the appliance being configured to move a patient's teeth from the OTA toward the FTA, and
   wherein the predetermined parameter is based at least in part on an expected relapse after repositioning the patient's teeth from the OTA.
Clause 81. The method of Clause 80, wherein a minimum threshold force is needed to move at least one tooth of the patient via the appliance, and wherein the predetermined parameter is associated with the minimum threshold force.
Clause 82. The method of Clause 80 or Clause 81, wherein the appliance has a configuration in an unloaded state that generally corresponds to the second FTA.
Clause 83. The method of any one of Clause 80 to Clause 82, wherein the appliance has a configuration in an unloaded state that generally corresponds to the second FTA, and wherein the appliance is configured to move the patient's teeth to the first FTA.
Clause 84. The method of any one of Clause 80 to Clause 83, wherein the appliance has a configuration in an unloaded state that generally corresponds to the second FTA, and wherein the appliance is configured to move the patient's teeth to the first FTA and not to the second FTA.
Clause 85. The method of any one of Clause 80 to Clause 84, wherein:
   a minimum threshold force is needed to move at least one tooth of the patient via the appliance;
   the predetermined parameter is associated with the minimum threshold force; and the appliance is configured to provide a non-zero force greater than the minimum threshold along a path defined by at least the OTA and the first FTA.
Clause 86. The method of any one of Clause 80 to Clause 85, wherein:
   a minimum threshold force is needed to move at least one tooth of the patient via the appliance;
   the predetermined parameter is associated with the minimum threshold force; and the appliance, when in a configuration generally corresponding to the first FTA, is configured to provide a non-zero force less than the minimum threshold.
Clause 87. A method for determining an arrangement of an orthodontic device, the method comprising:
   obtaining data corresponding to an original tooth arrangement (OTA) of a patient; and
   determining data corresponding to a final tooth arrangement (FTA) based on the OTA and a predetermined parameter,
   wherein the FTA can be used to form a fixture and/or an orthodontic appliance, the appliance being configured to move a patient's teeth from the OTA toward the FTA, and
   wherein a minimum threshold force is needed to move at least one tooth of the patient via the appliance, and
   wherein the predetermined parameter is associated with the minimum threshold force.
Clause 88. The method of Clause 87, wherein the appliance is configured to be coupled to a securing member fixed to a patient's tooth, and wherein the predetermined parameter is associated with an expected free play between the appliance and the securing member.
Clause 89. The method of Clause 87 or Clause 88, wherein the appliance includes an attachment portion configured to be coupled to a securing member fixed to a patient's tooth, and wherein the predetermined parameter is associated with an expected free play between the attachment portion and the securing member.
Clause 90. The method of any one of the Clauses herein, wherein:
   the appliance includes an arm having an attachment portion configured to be coupled to a securing member fixed to a patient's tooth,
   the predetermined parameter is associated with a free play between the attachment portion and the securing member, the free play corresponding to an angle of rotation in which the attachment portion is able to rotate relative to the securing member, and
   the second FTA differs from the first FTA at least by the angle of rotation.
Clause 91. The method of clause Clause 90, wherein the angle of rotation is in a direction corresponding to at least one of the mesial, distal, occlusal, gingival, facial, and/or lingual directions.
Clause 92. The method of any one of the clauses herein, wherein:
   the appliance includes an arm having an attachment portion configured to be coupled to a securing member fixed to a patient's tooth,
   the predetermined parameter is associated with a free play between the attachment portion and the securing member, the free play corresponding to a dimension in which the attachment portion is able to move relative to the securing member, and
   the second FTA differs from the first FTA at least by the dimension.
Clause 93. The method of clause Clause 92, wherein the dimension extends in a direction corresponding to at least one of the mesial-distal, occlusal-gingival, and/or facial-lingual directions.
Clause 94. The method of any one of the clauses herein, wherein an arm of the appliance is configured to be coupled to a securing member fixed to a patient's tooth, and wherein the predetermined parameter is associated with an expected free play between the arm and the securing member.
Clause 95. A method for determining an arrangement of an orthodontic device, the method comprising:
   obtaining data corresponding to an original tooth arrangement (OTA) of a patient; and
   determining data corresponding to a final tooth arrangement (FTA) based on the OTA and a predetermined parameter,
   wherein the FTA can be used to form a fixture and/or an orthodontic appliance, the appliance having a plurality of arms that, when coupled to a patient's teeth via corresponding securing members, are configured to be move a patient's teeth from the OTA toward the FTA, and
   wherein the predetermined parameter is based at least in part on an expected free play between at least one of the arms and corresponding securing member.
Clause 96. The method of any one of the clauses herein, wherein the predetermined parameter is associated with a positional difference between the first FTA and the second FTA.
Clause 97. The method of any one of the clauses herein, wherein the appliance is configured to have a first arrangement corresponding to the first FTA and the fixture is configured to have a second configuration corresponding to the second FTA, and wherein the predetermined parameter is associated with the difference between the first and second arrangements.
Clause 98. The method of any one of the clauses herein, further comprising:
   manufacturing the fixture to have an arrangement corresponding to the second FTA;
   treating the appliance disposed over the fixture, thereby causing the appliance to have an arrangement corresponding to the first FTA.
Clause 99. The method of any one of the clauses herein, further comprising:
   manufacturing the fixture to have an arrangement corresponding to the second FTA;
   manufacturing the appliance to have a 2D configuration;
   coupling the appliance over the fixture;
   treating the appliance disposed over the fixture, thereby causing the appliance to assume an arrangement corresponding to the second FTA; and
   decoupling the appliance from the fixture, thereby causing the appliance to assume an arrangement corresponding to the first FTA.
Clause 100. A method for determining an arrangement of an orthodontic device, the method comprising:
   obtaining data corresponding to an original tooth arrangement (OTA) of a patient; and
   determining data corresponding to a final tooth arrangement (FTA) based on the OTA and a predetermined parameter,
   wherein the FTA can be used to form a fixture and/or an orthodontic appliance, the appliance being configured to move a patient's teeth from the OTA toward the FTA, and
   wherein the predetermined parameter is associated with an expected plastic deformation threshold of the appliance.
Clause 101. The method of any one of the clauses herein, wherein the predetermined parameter is associated with a stress experienced by the appliance when in the OTA.
Clause 102. The method of any one of the clauses herein, wherein the predetermined parameter is associated with a material property of the appliance.
Clause 103. The method of any one of the clauses herein, wherein the appliance comprises a superelastic material, and wherein the predetermined parameter is associated with plastic deformation associated with the superelastic material.
Clause 104. The method of any one of the clauses herein, wherein the appliance comprises nitinol, and wherein the predetermined parameter is associated with plastic deformation associated with nitinol.
Clause 105. The method of any one of the clauses herein, wherein the appliance comprises nitinol, and wherein the predetermined parameter is associated with hysteresis of nitinol.
Clause 106. The method of any one of the clauses herein, wherein the predetermined parameter is associated with a stress experienced by the appliance when in a configuration corresponding to at least one of the OTA or the FTA.
Clause 107. The method of any one of the clauses herein, wherein:
   the predetermined parameter is associated with an expected plastic deformation threshold of the appliance,
   the appliance includes an anchor portion and an arm extending from the anchor portion, and
   the plastic deformation threshold is associated with the arm of the appliance.
Clause 108. The method of any one of the clauses herein, wherein:
   the predetermined parameter is associated with an expected plastic deformation threshold of the appliance,
   the appliance includes an anchor portion and an arm extending from the anchor portion, the arm including a biasing portion, and
   the plastic deformation threshold is associated with the biasing portion of the appliance.
Clause 109. The method of any one of the clauses herein, wherein the appliance, when coupled to the patient's teeth, is configured to transition from a first configuration corresponding to the OTA, and wherein determining the data corresponding to the FTA comprises determining whether a portion of the appliance in the first configuration exceeds a yield strength of a material of the appliance.
Clause 110. The method of any one of the clauses herein, wherein:
   the appliance, when coupled to the patient's teeth, is configured to transition from a first configuration corresponding to the OTA, and
   determining the data corresponding to the FTA comprises determining whether a portion of the appliance in the first configuration exceeds a yield strength of a material of the appliance.
Clause 111. The method of any one of the clauses herein, wherein the appliance, when coupled to the patient's teeth, is configured to transition from a first configuration corresponding to the OTA to a second configuration corresponding to the FTA, and wherein determining the data corresponding to the FTA comprises determining whether a portion of the appliance in the first or second configuration exceeds a yield strength of a material of the appliance.
Clause 112. A method for determining an arrangement of an orthodontic device, the method comprising:
   obtaining data corresponding to an original tooth arrangement (OTA) of a patient; and
   determining data corresponding to a final tooth arrangement (FTA) based on the OTA and a predetermined parameter,
   wherein the FTA can be used to form a fixture and/or an orthodontic appliance, the appliance being configured to move teeth of the patient from the OTA toward the FTA.
Clause 113. The method of any one of the clauses herein, wherein the predetermined parameter is that of any one of the clauses herein.
Clause 114. A method of fabricating an orthodontic appliance, the method comprising:
   obtaining position data corresponding to an original tooth arrangement (OTA) of a patient;
   obtaining position data corresponding to a desired final tooth arrangement (FTA) of the patient;
   fabricating an orthodontic appliance that, when installed within a mouth of the patient, is configured to urge teeth of the patient from the OTA to the FTA, wherein, when the appliance is coupled to the teeth of the patient in the FTA, the appliance exerts a non-zero force on one or more teeth of the patient, the non-zero force falling below a minimum threshold force.
Clause 115. A method of fabricating an orthodontic appliance, the method comprising:
   obtaining position data corresponding to an original tooth arrangement (OTA) of a patient;
   obtaining position data corresponding to a desired final tooth arrangement (FTA) of the patient;
   fabricating an orthodontic appliance configured to move teeth of the patient from the OTA toward the FTA; and
   shape-setting the appliance by applying the appliance to a treatment fixture such that the appliance assumes a first configuration, the fixture having a shape that deviates from the FTA such that, after the appliance is removed from the fixture, the appliance assumes a second configuration in which at least a portion of the appliance is deflected away from the first configuration.
Clause 116. A tangible, non-transitory computer-readable medium storing instructions that, when executed by one or more processors, cause the one or more processors to perform a method of any one of the clauses herein.
Clause 117. A device comprising:
   one or more processors; and
   a tangible, non-transitory computer-readable medium storing instructions that, when executed by the one or more processors, cause the one or more processors to perform the method of any one of the clauses herein.
Clause 118. An orthodontic appliance manufactured according to a method of any one of the clauses herein.
Clause 119. A heat treatment fixture manufactured according to a method of any one of the clauses herein.
Clause 120. 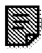 nethod for manufacturing an orthodontic appliance for repositioning a tooth of a patient, the orthodontic appliance having an anchor and at least one arm extending away from the anchor, the arm comprising a proximal portion at the anchor and a distal portion configured to be secured to an orthodontic bracket, the method comprising:
   obtaining first position data characterizing a first position of the patient's tooth prior to repositioning of the tooth by the appliance;
   obtaining second position data characterizing a second position of the patient's tooth after repositioning of the tooth by the appliance;
   obtaining third position data characterizing a desired position of the patient's tooth after an anticipated movement of the tooth after repositioning of the tooth by the appliance; and
   forming a three-dimensional configuration of the appliance such that the distal portion of the arm of the appliance is located at the second position,
   wherein the appliance is configured to reposition the tooth from the first position to the second position such that, after the tooth moves according to the anticipated movement, the tooth is positioned at the desired position.
Clause 121. The method of Clause 120, wherein the appliance is configured to reposition the tooth from the first position to the second position along a path in a first direction.
Clause 122. The method of Clause 120 or Clause 121, wherein the anticipated movement of the tooth is along the path in a second direction opposite of the first direction.
Clause 123. A method for designing an orthodontic appliance for repositioning a tooth of a patient, the method comprising:
   obtaining first position data characterizing an initial position of the patient's tooth;
   obtaining second position data characterizing an intended position of the patient's tooth;
   obtaining deformation data characterizing an anticipated deformation of the appliance releasing the appliance from a shape-setting fixture; and
   based on the first position data, the second position, and the deformation data, obtaining appliance data characterizing a three-dimensional (3D) configuration of the appliance such that the appliance is configured to reposition the tooth from the initial position to the intended position.
Clause 124. The method of Clause 123, wherein the anticipated deformation is due to a superelastic property of the appliance.
Clause 125. The method of Clause 123 of Clause 124, wherein the orthodontic appliance has an anchor and at least one arm extending away from the anchor, the arm comprising a proximal portion at the anchor and a distal portion configured to be secured to an orthodontic bracket that is configured to be secured to the patient's tooth, and wherein a position of the distal portion of the arm in the 3D configuration is different than the intended position of the tooth.
Clause 126. The method of any one of Clause 123 to Clause 125, wherein resilience data characterizes an anticipated deformation of the appliance after setting a shape of the appliance while the appliance is secured to the shape-setting fixture.
Clause 127. A method for designing an orthodontic appliance for repositioning a tooth of a patient, the method comprising:
   obtaining first position data characterizing an initial position of the patient's tooth; obtaining second position data characterizing an intended position of the patient's tooth;
   obtaining appliance data characterizing a pre-installation configuration of the appliance;
   obtaining deformation data characterizing an anticipated deformation of the appliance from the pre-installation configuration to an installed configuration; and
   based on the first position data, the second position, and the deformation data, obtaining modified appliance data characterizing a modified pre-installation configuration of the appliance.
Clause 128. The method of Clause 127, wherein the deformation data characterizes a stress and/or a strain in one or more portions of the appliance.
Clause 129. The method of Clause 127 or Clause 128, further comprising determining whether plastic deformation is expected to occur at one or more portions of the appliance due to the anticipated deformation of the appliance from the pre-installation configuration to the installed configuration.
Clause 130. The method of Clause 129, wherein determining whether plastic deformation is expected to occur comprises comparing the deformation data to at least one of a yield stress or a yield strain of a material of the appliance.
Clause 131. The method of any one of Clause 127 to Clause 130, wherein the modified pre-installation configuration is a first modified pre-installation configuration, the method further comprising, after obtaining the modified appliance data:
   obtaining second deformation data characterizing an anticipated deformation of the appliance from the first modified pre-installation configuration to an installed configuration; and
   based on the first position data, the second position, and the deformation data, obtaining second modified appliance data characterizing a second modified pre-installation configuration of the appliance.
Clause 132. 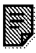 nethod for designing an orthodontic appliance for repositioning a tooth of a patient, the orthodontic appliance having an anchor and at least one arm extending away from the anchor, the arm comprising a proximal portion at the anchor and a distal portion configured to be received within a securing portion of an orthodontic bracket, the method comprising:
   obtaining first position data characterizing an initial position of the patient's tooth prior to repositioning of the tooth by the appliance;
   obtaining second position data characterizing an intended position of the patient's tooth after repositioning of the tooth by the appliance;
   obtaining arm data characterizing a dimension of the distal portion of the arm of the appliance;
   obtaining bracket data characterizing a dimension of the securing portion of the orthodontic bracket;
   obtain play data characterizing a difference between the arm data and the bracket data;
   based on the play data, obtaining force data characterizing an anticipated force to be applied to the bracket by the appliance; and
   based on the force data, obtaining third position data characterizing a passive position of the distal portion of the arm of the appliance after the appliance has been shape-set, the passive position being different than the intended position of the tooth and/or the original position of the tooth.
Clause 133. The method of Clause 132, further comprising forming a three-dimensional configuration of the appliance such that the distal portion of the arm of the appliance is located at the second position.
Clause 134. The method of Clause 132 or Clause 133, wherein obtaining the play data comprises determining an anticipated maximum angular displacement between a plane of the distal portion of the arm and a plane of the securing portion of the bracket.
Clause 135. The method of any one of Clause 132 to Clause 134, wherein obtaining the force data comprises determining an anticipated torque loss parameter associated with a connection between the distal portion of the arm and the securing portion of the bracket.
Clause 136. The method of any one of Clause 132 to Clause 135, wherein the arm data characterizes at least two of an occlusogingival dimension of the distal portion of the arm, a buccolingual dimension of the distal portion of the arm, or a mesiodistal dimension of the distal portion of the arm.
Clause 137. The method of any one of Clause 132 to Clause 136, wherein the bracket data characterizes at least two of an occlusogingival dimension of the securing portion of the bracket, a buccolingual dimension of the securing portion of the bracket, or a mesiodistal dimension of the securing portion of the bracket.
Clause 138. The method of any one of Clause 132 to Clause 137, wherein obtaining the play data comprises calculating an anticipated maximum distance between the distal portion of the arm and the securing portion of the bracket.
Clause 139. A method for manufacturing an orthodontic appliance for repositioning a tooth of a patient, the orthodontic appliance having an anchor and at least one arm extending away from the anchor, the arm comprising a proximal portion at the anchor and a distal portion configured to be secured to an orthodontic bracket that is secured to the patient's tooth, the method comprising:
   obtaining first position data characterizing an original position of the patient's tooth prior to repositioning of the tooth by the appliance;
   obtaining second position data characterizing an intended position of the patient's tooth after repositioning of the tooth by the appliance; and
   setting a shape of the appliance such that, when the distal portion of the arm is secured to the bracket that is secured to the tooth and the appliance has repositioned the tooth to its intended position, the appliance applies a force to the tooth, the force having a magnitude greater than a predetermined threshold.
Clause 140. The method of Clause 139, wherein the predetermined threshold is greater than zero.
Clause 141. The method of Clause 139 or Clause 140, wherein the predetermined threshold is between about 5 grams and about 150 grams.
Clause 142. The method of any one of Clause 139 to Clause 141, wherein, after setting a shape of the appliance, the distal portion of the arm is located at a passive position, the passive position being different than the intended position of the tooth and/or the original position of the tooth.
Clause 143. The method of any one of Clause 139 to Clause 142, wherein the predetermined threshold is unique to the tooth.
Clause 144. A method for designing an orthodontic appliance for repositioning a tooth of a patient, the method comprising:
   obtaining an appliance digital model characterizing the orthodontic appliance in an initial configuration;
   obtaining a fixture digital model characterizing a fixture for setting a shape of the appliance; and
   performing a finite element analysis (FEA) to virtually deform the appliance digital model based on the fixture digital model.
Clause 145. The method of Clause 144, wherein the fixture digital model comprises:
   a gingival portion having a shape substantially corresponding to a surface of the patient's gingiva; and
   at least one securing portion carried by the gingival portion and configured to retain a portion of the appliance.
Clause 146. The method of Clause 144 or Clause 145, wherein performing the FEA comprises causing at least one portion of the appliance digital model to substantially conform to the fixture digital model.
Clause 147. The method of any one of Clauses 144 to 146, wherein the appliance comprises an anchor and an arm extending away from the anchor, the arm comprising a proximal portion at the anchor and a distal portion configured to be secured to an orthodontic bracket, and wherein performing the FEA comprises positioning a distal portion of an arm of the appliance digital model at or within the securing portion of the fixture digital model.
Clause 148. The method of any one of Clauses 144 to 147, wherein the appliance comprises an anchor and an arm extending away from the anchor, and wherein performing the FEA comprises applying a non-zero displacement to an anchor of the appliance digital model.
Clause 149. The method of any one of Clauses 144 to 148, wherein the appliance is substantially planar in the initial configuration.
Clause 150. A method for designing an orthodontic appliance for repositioning a tooth of a patient, the method comprising:
   obtaining an appliance digital model characterizing the orthodontic appliance in a pre-installation configuration;
   obtaining an anatomy digital model characterizing a patient's teeth and gingiva in an original arrangement; and
   performing an FEA to virtually deform the appliance digital model based on the anatomy digital model.
Clause 151. The method of Clause 150, the appliance comprises an anchor and an arm extending away from the anchor, the arm comprising a proximal portion at the anchor and a distal portion configured to be secured to an orthodontic bracket, and wherein performing the FEA comprises causing the distal portion of the arm to be positioned at or adjacent to one of the patient's teeth.
Clause 152. The method of Clause 150 or Clause 151, wherein the appliance has a substantially three-dimensional (3D) shape in the pre-installation configuration.
Clause 153. The method any one of Clauses 150 to 152, further comprising evaluating the deformed appliance digital model.
Clause 154. The method of Clause 153, wherein evaluating the deformed appliance digital model comprises determining whether the deformed appliance digital model impinges on the gingiva or is spaced apart from the gingiva by greater than a predetermined threshold.
Clause 155. The method of Clause 153 or Clause 154, wherein evaluating the deformed configuration comprises determining whether any portion of the deformed appliance digital model exceeds an elastic strain limit.
Clause 156. The method of any one of Clauses 153 to 155, wherein evaluating the deformed configuration comprises determining a difference between a force and/or moment applied to the teeth by the deformed appliance and an intended force and/or moment.
Clause 157. The method of any one of Clauses 153 to 156, further comprising, based on the evaluation, modifying the appliance digital model, wherein modifying the appliance digital model comprises changing at least one of a shape of an arm of the appliance, a shape of an anchor of the appliance, or a shape of the appliance in the pre-installation configuration.
Clause 158. A method for designing an orthodontic appliance for repositioning a tooth of a patient, the method comprising:
   obtaining a preliminary appliance digital model virtually representing the appliance in a preliminary configuration;
   obtaining a heat treatment fixture digital model, the heat treatment fixture digital model characterizing a geometry of a heat treatment fixture for shape-setting an appliance, wherein the heat treatment fixture comprises a gingival surface having a shape substantially corresponding to a shape of a gingival surface of the patient and a securing portion configured to releasably retain a portion of the appliance;
   performing a first FEA to virtually deform the preliminary appliance digital model based on the heat treatment fixture digital model;
   obtaining an intended appliance digital model virtually representing the appliance in a three-dimensional configuration with a geometry based at least in part on the heat treatment fixture digital model;
   obtaining an original tooth arrangement (OTA) digital model virtually representing a patient's teeth and gingiva in an original arrangement;
   performing a second FEA to virtually deform the intended appliance digital model based on the OTA digital model; and
   obtaining a deformed intended appliance digital model and an analysis result.
Clause 159. The method of Clause 158, wherein the appliance is substantially planar in the preliminary configuration.
Clause 160. The method of Clause 158 or Clause 159, wherein performing the first FEA comprises:
   discretizing at least one of the preliminary appliance digital model and the heat treatment fixture digital model into a plurality of finite elements and a plurality of nodes;
   assigning material properties to at least one of the preliminary appliance digital model and the heat treatment fixture digital model;
   defining a contact interaction between the preliminary appliance digital model and the heat treatment fixture digital model;
   assigning boundary conditions to at least one of the preliminary appliance digital model and the heat treatment fixture digital model;
   defining an analysis parameter; and
   running the FEA until an exit condition is reached.
Clause 161. The method of Clause 160, wherein assigning the boundary conditions includes at least one of assigning a non-zero displacement a portion of the planar appliance digital model or defining a relationship between an orientation of a portion of the planar appliance digital model and a base plane of a securing portion of the heat treatment fixture.
Clause 162. The method of Clause 158, wherein performing the second FEA comprises:
   discretizing at least one of the intended appliance digital model and the OTA digital model into a plurality of finite elements and a plurality of nodes;
   assigning material properties to at least one of the intended appliance digital model and the OTA digital model;
   defining a contact interaction between the intended appliance digital model and the OTA digital model;
   assigning boundary conditions to at least one of the intended appliance digital model and the OTA digital model;
   defining an analysis parameter; and
   running the FEA until an exit condition is reached.
Clause 163. The method of Clause 162, wherein assigning the boundary conditions comprises assigning a displacement to a portion of the intended appliance digital model, the displacement based at least in part on a movement of the patient's tooth from the original arrangement to a desired final arrangement.
Clause 164. The method of any one of Clauses 158 to 163, wherein the analysis result comprises at least one of a strain in the deformed intended appliance digital model or a distance between the deformed intended appliance digital model and the gingival surface of the patient.
Clause 165. The method of any one of Clauses 158 to 164 wherein the orthodontic appliance comprises an anchor and at least one arm extending away from the anchor, the arm comprising a proximal portion at the anchor and a distal portion configured to be secured to an orthodontic bracket.
Clause 166. The method of Clause 165, wherein performing the first FEA causes the anchor of the appliance to be positioned at or adjacent to the gingival surface of the heat treatment fixture digital model.
Clause 167. The method of Clause 165, wherein performing the second FEA causes the distal portion of the arm of the appliance to be positioned at or adjacent to one of the patient's teeth.
Clause 168. A method for designing an orthodontic appliance for repositioning a tooth of a patient, the method comprising:
   obtaining an OTA digital model of a patient's teeth and gingiva in an original arrangement, the OTA digital model comprising original position data of a tooth to be repositioned by the orthodontic appliance when installed in the patient's mouth;
   obtaining an FTA digital model characterizing the patient's teeth and gingiva in a desired final arrangement, the FTA digital model comprising final position data of the tooth;
   determining displacement data characterizing a displacement between the original position data of the tooth and the final position data of the tooth;
   obtaining a heat treatment fixture digital model based on at least one of the OTA digital model or the FTA digital model;
   obtaining a 3D template digital model based on the heat treatment fixture digital model;
   obtaining a planar template digital model, wherein the planar template digital model is a substantially planar configuration of the 3D template digital model;
   obtaining a planar appliance digital model based on the planar template digital model;
   obtaining an intended appliance digital model, wherein the intended appliance digital model characterizes the orthodontic appliance in 3D configuration based on the heat treatment fixture digital model;
   performing an FEA on the OTA and intended appliance digital models to deform the intended appliance digital model based on the displacement data; and
   evaluating an analysis result of the virtual deformation.
Clause 169. The method of Clause 168, wherein the displacement data comprises three translations and three rotations.
Clause 170. The method of Clause 168 or Clause 169, further comprising modifying the heat treatment fixture digital model based on the intended appliance digital model.
Clause 171. The method of Clause 170, wherein modifying the heat treatment fixture digital model comprises defining a tangent relationship between a gingival surface of the heat treatment fixture digital model and a gingival-facing surface of the intended appliance digital model.
Clause 172. The method of any one of Clauses 168 to 171, further comprising manufacturing at least one of the planar template digital model, the heat treatment fixture digital model, or the intended appliance digital model.
Clause 173. The method of any one of Clauses 168 to 172, wherein the orthodontic appliance comprises an anchor and an arm extending away from the anchor, the arm comprising a proximal portion at the anchor and a distal portion configured to be secured to an orthodontic bracket.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure.
FIG. 1A shows the schematic representation of an orthodontic appliance configured in accordance with the present technology installed in a patient's mouth adjacent the patient's dentition.
FIG. 1B is a schematic depiction of connection configuration options configured in accordance with embodiments of the present technology.
FIG. 1C is a schematic depiction of a portion of an appliance configured in accordance with embodiments of the present technology.
FIGS. 2A and 2B are elevation views of an appliance configured in accordance with several embodiments of the present technology installed in an upper and lower jaw of a patient's mouth with the patient's teeth in an original tooth arrangement and a final tooth arrangement, respectively.
FIG. 3 is a flow diagram of an example process for manufacturing an orthodontic appliance in accordance with the present technology.
FIG. 4 is a schematic block diagram of a system for manufacturing an orthodontic appliance in accordance with the present technology.
FIG. 5 is a flow diagram of a process for designing an orthodontic appliance in accordance with the present technology.
FIG. 6 illustrates scanning a patient's teeth to obtain original tooth arrangement data.
FIG. 7 illustrates an example of a digital model of a patient's teeth and gingiva in an original tooth arrangement.
FIG. 8 illustrates an example of a digital model of a patient's teeth and gingiva in a final tooth arrangement.
FIG. 9 illustrates an example of a digital model of a securing member.
FIG. 10 illustrates an example of a digital model of a patient's teeth and gingiva and a plurality of securing members in an original tooth arrangement.
FIG. 11 illustrates an example of a digital model of a patient's teeth and gingiva and a plurality of securing members in a final tooth arrangement.
FIG. 12 illustrates an example of a digital model of a heat treatment fixture.
FIG. 13 illustrates an example of a digital model of a three-dimensional appliance template that is based on the heat treatment fixture model.
FIG. 14 illustrates an example of a digital model of a substantially planar appliance template.
FIG. 15 illustrates an example of a digital model of a substantially planar appliance with unique arm geometry based on determined displacement of each tooth.
FIG. 16 illustrates a perspective view of an orthodontic appliance in accordance with embodiments of the present technology.
FIG. 17 illustrates a perspective view off a heat treatment fixture for an appliance in accordance with the present technology.
FIG. 18 is a perspective view of an orthodontic appliance fastened to a heat treatment fixture in accordance with the present technology.
FIG. 19 is a flow diagram of an example process for determining a design of an orthodontic appliance.
FIG. 20 is a flow diagram of an example process for determining a design of an orthodontic appliance.
FIG. 21 illustrates an example of an intended appliance digital model obtained by performing a finite element analysis with a planar appliance digital model and a heat treatment fixture digital model.
FIG. 22 illustrates an example of a deformed intended appliance digital model obtained by performing a finite element analysis with an intended appliance digital model and an OTA digital model.
FIG. 23 illustrates an example of a result of a finite element analysis.
FIG. 24 illustrates another example of an analysis result.
FIG. 25 illustrates an example of results from iterative finite element analyses.
FIG. 26 is a plot showing the relationship between force applied to a patient's teeth and positioning of the patient's teeth.
FIG. 27 is a flow diagram of a method for determining data corresponding to an arrangement of an orthodontic device in accordance with embodiments of the present technology.
FIG. 28A is a perspective view of a securing member, FIG. 28B is a perspective view of a portion of an arm of an orthodontic appliance coupled to the securing member shown in FIG. 28A, and FIG. 28C is an enlarged side view of the securing member and appliance shown in FIG. 28B, in accordance with embodiments of the present technology.
FIG. 29A is a perspective view of a securing member, FIG. 29B is a perspective view of a portion of an arm of an orthodontic appliance coupled to the securing member shown in FIG. 29A, and FIG. 29C is an enlarged side view of the securing member and appliance shown in FIG. 23B, in accordance with embodiments of the present technology.
FIG. 30 is a flow diagram of a method for determining data corresponding to an arrangement of an orthodontic device, in accordance with embodiments of the present technology.
FIG. 31 is a flow diagram of a method for determining data corresponding to an arrangement of an orthodontic device, in accordance with embodiments of the present technology.
FIG. 32 is a side perspective view of an orthodontic appliance, configured in accordance with embodiments of the present technology, in accordance with embodiments of the present technology.
FIG. 33 is a flow diagram of a method for determining data corresponding to an arrangement of an orthodontic device, in accordance with embodiments of the present technology.
FIG. 34 is a flow diagram of a method for determining data corresponding to an arrangement of an orthodontic device, in accordance with embodiments of the present technology.

### DETAILED DESCRIPTION

The present technology relates generally to orthodontic appliances and associated systems configured to reposition one or more of a patient's teeth. In particular embodiments, the present technology relates to devices, systems, and methods for attaching or securing orthodontic appliances to the teeth, and associated methods for designing and fabricating such appliances. Specific details of several embodiments of the technology are described below with reference to FIGS. 1A-34.

### I. Definitions

Terms used herein to provide anatomical direction or orientation are intended to encompass different orientations of the appliance as installed in the patient's mouth, regardless of whether the structure being described is shown installed in a mouth in the drawings. For example, "mesial" means in a direction toward the midline of the patient's face along the patient's curved dental arch; "distal" means in a direction away from the midline of the patient's face along the patient's curved dental arch; "occlusal" means in a direction toward the chewing surfaces of the patient's teeth; "gingival" means in a direction toward the patient's gums or gingiva; "facial" means in a direction toward the patient's lips or cheeks (used interchangeably herein with "buccal" and "labial"); and "lingual" means in a direction toward the patient's tongue.

As used herein, the terms "proximal" and "distal" refer to a position that is closer and farther, respectively, from a given reference point. In many cases, the reference point is a certain connector, such as an anchor, and "proximal" and "distal" refer to a position that is closer and farther, respectively, from the reference connector along a line passing through the centroid of the cross-section of the portion of the appliance branching from the reference connector.

As used herein, the terms "generally," "substantially," "about," and similar terms are used as terms of approximation and not as terms of degree, and are intended to account for the inherent variations in measured or calculated values that would be recognized by those of ordinary skill in the art.

As used herein, the term "operator" refers to a clinician, practitioner, technician or any person or machine that designs and/or manufactures an orthodontic appliance or portion thereof, and/or facilitates the design and/or manufacture of the appliance or portion thereof, and/or any person or machine associated with installing the appliance in the patient's mouth and/or any subsequent treatment of the patient associated with the appliance.

As used herein, the term "force" refers to the magnitude and/or direction of a force, a torque, or a combination thereof.

### II. Overview of Orthodontic Appliances of the Present Technology

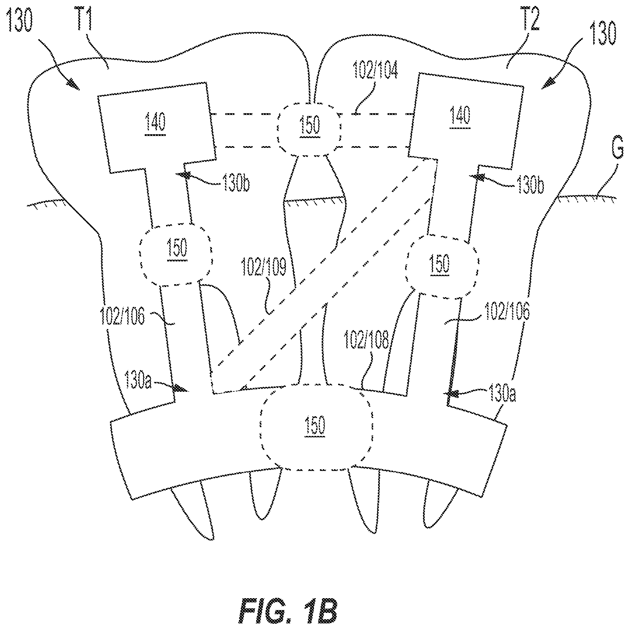

FIG. 1A is a schematic representation of an orthodontic appliance 100 (or "appliance 100") configured in accordance with embodiments of the present technology, shown positioned in a patient's mouth adjacent the patient's teeth. FIG. 1B is an enlarged view of a portion of the appliance 100. The appliance 100 is configured to be installed within a patient's mouth to impart forces on one or more of the teeth to reposition all or some of the teeth. In some cases, the appliance 100 may additionally or alternatively be configured to maintain a position of one or more teeth. As shown schematically in FIGS. 1A and 1B, the appliance 100 can comprise a deformable member that includes one or more attachment portions 140 (each represented schematically by a box), each configured to be secured to a tooth surface directly or indirectly via a securing member 160. The appliance 100 may further comprise one or more connectors 102 (also depicted schematically), each extending directly between attachment portions 140 ("first connectors 104"), between an attachment portion 140 and one or more other connectors 102 ("second connectors 106"), or between two or more other connectors 102 ("third connectors 108"). Only two attachment portions 140 and two connectors 102 are labeled in FIG. 1A for ease of illustration. As discussed herein, the number, configuration, and location of the connectors 102 and attachment portions 140 may be selected to provide a desired force on one or more of the teeth when the appliance 100 is installed.

The attachment portions 140 may be configured to be detachably coupled to a securing member 160 that is bonded, adhered, or otherwise secured to a surface of one of the teeth to be moved. In some embodiments, one or more of the attachment portions 140 may be directly bonded, adhered, or otherwise secured to a corresponding tooth without a securing member or other connection interface at the tooth. The different attachment portions 140 of a given appliance 100 may have the same or different shape, same or different size, and/or same or different configuration. The attachment portions 140 may comprise any of the attachment portions, bracket connectors, and/or male connector elements disclosed in U.S. Patent Publication No. 2017/0156823 A1, which is incorporated by reference herein in its entirety.

The appliance 100 may include any number of attachment portions 140 suitable for securely attaching the appliance 100 to the patient's tooth or teeth in order to achieve a desired movement. In some examples, multiple attachment portions 140 may be attached to a single tooth. The appliance 100 may include an attachment portion for every tooth, fewer attachment portions than teeth, or more attachment portions 140 than teeth. In these and other embodiments, the appliance 100 one or more of the attachment portions 140 may be configured to be coupled to one, two, three, four, five or more connectors 102.

As previously mentioned, the connectors 102 may comprise one or more first connectors 104 that extend directly between attachment portions 140. The one or more first connectors 104 may extend along a generally mesiodistal dimension when the appliance 100 is installed in the patient's mouth. In these and other embodiments, the appliance 100 may include one or more first connectors 104 that extend along a generally occlusogingival and/or buccolingual dimension when the appliance 100 is installed in the patient's mouth. In some embodiments, the appliance 100 does not include any first connectors 104.

Additionally or alternatively, the connectors 102 may comprise one or more second connectors 106 that extend between one or more attachment portions 140 and one or more connectors 102. The one or more second connectors 106 can extend along a generally occlusogingival dimension when the appliance 100 is installed in the patient's mouth. In these and other embodiments, the appliance 100 may include one or more second connectors 106 that extend along a generally mesiodistal and/or buccolingual dimension when the appliance 100 is installed in the patient's mouth. In some embodiments, the appliance 100 does not include any second connectors 106. In such embodiments, the appliance 100 would only include first connectors 104 extending between attachment portions 140. A second connector 106 and the attachment portion 140 to which it is attached may comprise an "arm," as used herein (such as arm 130 in FIGS. 1A and 1B). In some embodiments, multiple second connectors 106 may extend from the same location along the appliance 100 to the same attachment portion 140. In such cases, the multiple second connectors 106 and the attachment portion 140 together comprise an "arm," as used herein. The use of two or more connectors to connect two points on the appliance 100 enables application of a greater force (relative to a single connector connecting the same points) without increasing the strain on the individual connectors. Such a configuration is especially beneficial given the spatial constraints of the fixed displacement treatments herein.

Additionally or alternatively, the connectors 102 may comprise one or more third connectors 108 that extend between two or more other connectors 102. The one or more third connectors 108 may extend along a generally mesiodistal dimension when the appliance 100 is installed in the patient's mouth. In these and other embodiments, the appliance 100 may include one or more third connectors 108 that extend along a generally occlusogingival and/or buccolingual dimension when the appliance 100 is installed in the patient's mouth. In some embodiments, the appliance 100 does not include any third connectors 108. One, some, or all of the third connectors 108 may be positioned gingival to one, some, or all of the first connectors 104. In some embodiments, the appliance 100 includes a single third connector 108 that extends along at least two adjacent teeth and provides a common attachment for two or more second connectors 106. In several embodiments, the appliance 100 includes multiple non-contiguous third connectors 108, each extending along at least two adjacent teeth.

As shown in FIG. 1A, in some embodiments the appliance 100 may be configured such that all or a portion of one, some, or all of the connectors 102 disposed proximate the patient's gingiva when the appliance 100 is installed within the patient's mouth. For example, one or more third connectors 108 may be configured such that all or a portion of the one or more third connectors 108 is positioned below the patient's gum line and adjacent to but spaced apart from the gingiva. In many cases it may be beneficial to provide a small gap (e.g., 0.5 mm or less) between the third connector(s) 108 and the patient's gingiva, as contact between the third connector(s) 108 (or any portion of the appliance 100) and the gingiva can cause irritation and patient discomfort. In some embodiments, all or a portion of the third connector(s) 108 is configured to be in direct contact with the gingiva when the appliance 100 is disposed in the patient's mouth. Additionally or alternatively, all or a portion of one or more first connectors 104 and/or second connectors 106 may be configured to be disposed proximate the gingiva.

According to some embodiments, one or more connectors 102 may extend between an attachment portion 140 or connector 102 and a joint comprising (a) two or more connectors 102, (b) two or more attachment portions 140, or (c) at least one attachment portion 140 and at least one connector 102. According to some embodiments, one or more connectors 102 may extend between a first joint comprising (a) two or more connectors 102, (b) two or more attachment portions 140, or (c) at least one attachment member and at least one connector 102, and a second joint comprising (a) two or more connectors 102, (b) two or more attachment portions 140, or (c) at least one attachment portion 140 and at least one connector 102. An example of a connector 102 extending between (a) a joint between a second and third connector 106, 108, and (b) a joint between a second connector 106 and an attachment portion 140 is depicted schematically and labeled 109 in FIG. 1B.

Each of the connectors 102 may be designed to have a desired stiffness so that an individual connector 102 or combination of connectors 102 imparts a desired force on one or more of the teeth. In many cases, the force applied by a given connector 102 may be governed by Hooke's Law, or *F = k* × *x*, where F is the restoring force exerted by the connector 102, *k* is the stiffness coefficient of the connector 102, and *x* is the displacement. In the most basic example, if a connector 102 does not exist between two points on the appliance 100, then the stiffness coefficient along that path is zero and no forces are applied. In the present case, the individual connectors 102 of the present technology may have varying non-zero stiffness coefficients. For example, one or more of the connectors 102 may be rigid (i.e., the stiffness coefficient is infinite) such that the connector 102 will not flex or bend between its two end points. In some embodiments, one or more of the connectors 102 may be "flexible" (i.e., the stiffness coefficient is non-zero and positive) such that the connector 102 can deform to impart (or absorb) a force on the associated tooth or teeth or other connector 102.

In some embodiments it may be beneficial to include one or more rigid connectors between two or more teeth. A rigid connector 102 is sometimes referred to herein as a "rigid bar" or an "anchor." Each rigid connector 102 may have sufficient rigidity to hold and maintain its shape and resist bending. The rigidity of the connector 102 can be achieved by selecting a particular shape, width, length, thickness, and/or material. Connectors 102 configured to be relatively rigid may be employed, for example, when the tooth to be connected to the connector 102 or arm is not to be moved (or moved by a limited amount) and can be used for anchorage. Molar teeth, for example, can provide good anchorage as molar teeth have larger roots than most teeth and thus require greater forces to be moved. Moreover, anchoring one or more portions of the appliance 100 to multiple teeth is more secure than anchoring to a single tooth. As another example, a rigid connection may be desired when moving a group of teeth relative to one or more other teeth. Consider, for instance, a case in which the patient has five teeth separated from a single tooth by a gap, and the treatment plan is to close the gap. The best course of treatment is typically to move the one tooth towards the five teeth, and not vice versa. In this case, it may be beneficial to provide one or more rigid connectors between the five teeth. For all of the foregoing reasons and many others, the appliance 100 may include one or more rigid first connectors 104, one or more rigid second connectors 106, and/or one or more rigid third connectors 108.

In these and other embodiments, the appliance 100 may include one or more flexible first connectors 104, one or more flexible second connectors 106, and/or one or more flexible third connectors 108. Each flexible connector 102 may have a particular shape, width, thickness, length, material, and/or other parameters to provide a desired degree of flexibility. According to some embodiments of the present technology, the stiffness of a given connector 102 may be tuned via incorporation of a one or more resiliently flexible biasing portions 150. As shown schematically in FIG. 1B, one, some, or all of the connectors 102 may include one or more biasing portion 150, such as springs, each configured to apply a customized force specific to the tooth to which it is attached.

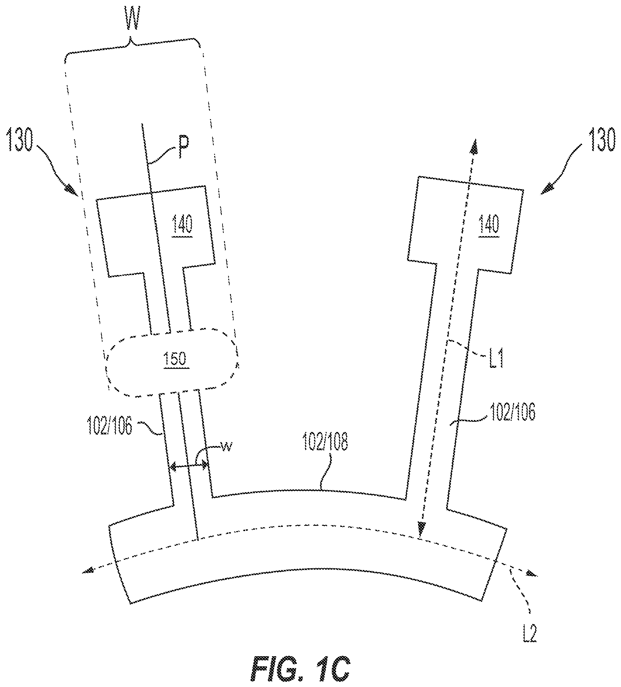

As depicted in the schematic shown in FIG. 1C, the biasing portion(s) 150 may extend along all or a portion of the longitudinal axis L1 of the respective connector 102 (only the longitudinal axis L1 for second connector 106 and the longitudinal axis L2 for third connector 108 is labeled in FIG. 1C). The direction and magnitude of the force and torque applied on a tooth by a biasing portion 150 depends, at least in part, on the shape, width, thickness, length, material, shape set conditions, and other parameters of the biasing portion 150. As such, one or more aspects of the biasing portion 150 (including the aforementioned parameters) may be varied so that the corresponding arm 130, connector 102, and/or biasing portion 150 produces a desired tooth movement when the appliance 100 is installed in the patient's mouth. Each arm 130 and/or biasing portion 150 may be designed to move one or more teeth in one, two, or all three translational directions (i.e., mesiodistal, buccolingual, and occlusogingival) and/or in one, two, or all three rotational directions (i.e., buccolingual root torque, mesiodistal angulation and mesial out-in rotation).

The biasing portions 150 of the present technology can have any length, width, shape, and/or size sufficient to move the respective tooth towards a desired position. In some embodiments, one, some, or all of the connectors 102 may have one or more inflection points along a respective biasing portion 150. The connectors 102 and/or biasing portions 150 may have a serpentine configuration such that the connector 102 and/or biasing portion 150 doubles back on itself at least one or more times before extending towards the attachment portion 140. For example, in some embodiments the second connectors 106 double back on themselves two times along the biasing portion 150, thereby forming first and second concave regions facing in generally different directions relative to one another. The open loops or overlapping portions of the connector 102 corresponding to the biasing portion 150 may be disposed on either side of a plane P (FIG. 1C) bisecting an overall width W (FIG. 1C) of the arm 130 and/or connector 102 such that the extra length of the arm 130 and/or connector 102 is accommodated by the space medial and/or distal to the arm 130 and/or connector 102. This allows the arm 130 and/or connector 102 to have a longer length (as compared to a linear arm) to accommodate greater tooth movement, despite the limited space in the occlusal-gingival or vertical dimension between any associated third connector 108 and the location at which the arm 130 attaches to the tooth.

It will be appreciated that the biasing portion 150 may have other shapes or configurations. For example, in some embodiments the connector 102 and/or biasing portion 150 may include one or more linear regions that zig-zag towards the attachment portion 140. One, some, or all of the connectors 102 and/or biasing portions 150 may have only linear segments or regions, or may have a combination of curved and linear regions. In some embodiments, one, some, or all of the connectors 102 and/or biasing portions 150 do not include any curved portions.

According to some examples, a single connector 102 may have multiple biasing portions 150 in series along the longitudinal axis of the respective connector 102. In some embodiments, multiple connectors 102 may extend between two points along the same or different paths. In such embodiments, the different connectors 102 may have the same stiffness or different stiffnesses.

In those embodiments where the appliance 100 has two or more connectors 102 with biasing portions 150, some, none, or all of the connectors 102 may have the same or different lengths, the same or different widths, the same or different thicknesses, the same or different shapes, and/or may be made of the same or different materials, amongst other properties. In some embodiments, less than all of the connectors 102 have biasing portions 150. Connectors 102 without biasing portions 150 may, for example, comprise one or more rigid connections between a rigid third connector 108 and the attachment portion 140. In some embodiments, none of the connectors 102 of the appliance 100 have a biasing portion 150.

According to some embodiments, for example as depicted schematically in FIG. 1A, the appliance 100 may include a single, continuous, substantially rigid third connector (referred to as "anchor 120") and a plurality of flexible arms 130 extending away from the anchor 120. When the appliance 100 is installed in the patient's mouth, each of the arms 130 may connect to a different one of the teeth to be moved and exerts a specific force on its respective tooth, thereby allowing an operator to move each tooth independently. Such a configuration provides a notable improvement over traditional braces in which all of the teeth are connected by a single archwire, such that movement of one tooth can cause unintentional movement of one or more nearby teeth. As discussed in greater detail herein, the independent and customized tooth movement enabled by the appliances of the present technology allows the operator to move the teeth from an original tooth arrangement ("OTA") to a final tooth arrangement ("FTA") more efficiently, thereby obviating periodic adjustments, reducing the number of office visits, and reducing or eliminating patient discomfort, and reducing the overall treatment time (i.e., the length of time the appliance is installed in the patient's mouth) by at least 50% relative to the overall treatment time for traditional braces.

The anchor 120 may comprise any structure of any shape and size configured to comfortably fit within the patient's mouth and provide a common support for one or more of the arms 130. In many embodiments, the anchor 120 is disposed proximate the patient's gingiva when the appliance 100 is installed within the patient's mouth, for example as shown in FIG. 1B. For instance, the appliance may be designed such that, when installed in the patient's mouth, all or a portion of the anchor 120 is positioned below the patient's gum line and adjacent but spaced apart from the gingiva. In many cases it may be beneficial to provide a small gap (e.g., 0.5 mm or less) between the anchor 120 (or any portion of the appliance 100) and the patient's gingiva as contact between the anchor 120 and the gingiva can cause irritation and patient discomfort. In some embodiments, all or a portion of the anchor 120 is configured to be in contact with the gingiva when the appliance 100 is disposed in the patient's mouth.

The anchor 120 may be significantly more rigid than the arms 130 such that the equal and opposite forces experienced by each of the arms 130 when exerting a force on its respective tooth are countered by the rigidity of the anchor 120 and the forces applied by the other arms 130, and do not meaningfully affect the forces on other teeth. As such, the anchor 120 effectively isolates the forces experienced by each arm 130 from the rest of the arms 130, thereby enabling independent tooth movement.

According to some embodiments, for example as shown schematically in FIG. 1A and 1B, the anchor 120 comprises an elongated member having a longitudinal axis L2 (see FIG. 1C) and forming an arched shape configured to extend along a patient's jaw when the appliance 100 is installed. In these and other embodiments, the anchor 120 may be shaped and sized to span two or more of the patient's teeth when positioned in the patient's mouth. In some examples, the anchor 120 includes a rigid, linear bar, or may comprise a structure having both linear and curved segments. In these and other embodiments, the anchor 120 may extend laterally across all or a portion of the patient's mouth (e.g., across all or a portion of the palate, across all or a portion of the lower jaw, etc.) and/or in a generally anterior-posterior direction. Moreover, the appliance 100 may comprise a single anchor or multiple anchors. For example, the appliance 100 may comprise multiple, discrete, spaced apart anchors, each having two or more arms 130 extending therefrom. In these and other embodiments, the appliance 100 may include one or more other connectors extending between adjacent arms 130.

Any and all of the features discussed above with respect to anchor 120 may apply to any of the third connectors 108 disclosed herein.

As shown in FIG. 1B, each of the arms 130 may extend between a proximal or first end portion 130a and a distal or second end portion 130b, and may have a longitudinal axis L extending between the first end portion 130a and the second end portion 130b. The first end portion 130a of one, some, or all of the arms 130 may be disposed at the anchor 120. In some embodiments, one, some, or all of the arms 130 are integral with the anchor 120 such that the first end portion 130a of such arms are continuous with the anchor 120. The arms 130 may extend from the anchor 120 at spaced intervals along the longitudinal axis L2 of the anchor 120, as shown in FIG. 1A. In some embodiments, the arms 130 may be spaced at even intervals relative to each other, or at uneven intervals relative to each other, along the longitudinal axis L2 of the anchor 120.

One, some, or all of the arms 130 may include an attachment portion 140 at or near the second end portion 130b. In some embodiments, for example as shown in FIGS. 1A-1C, one or more of the arms 130 is cantilevered from the anchor 120 such that the second end portion 130b of the cantilevered arm(s) 130 has a free distal end portion 130b. In these and other embodiments, a distal terminus of the attachment portion 140 may coincide with a distal terminus of the arm 130. The attachment portion 140 may be configured to detachably couple the respective arm 130 to a securing member (e.g., a bracket) that is bonded, adhered, or otherwise secured to a surface of one of the teeth to be moved. In some embodiments, the attachment portion 140 may be directly bonded, adhered, or otherwise secured to a corresponding tooth without a securing member or other connection interface at the tooth.

Referring to still to FIGS. 1A and 1B, one, some, or all of the arms 130 may include one or more resiliently flexible biasing portions 150, such as springs, each configured to apply a customized force, torque or combination of force and torque specific to the tooth to which it is attached. The biasing portion(s) 150 may extend along all or a portion of the longitudinal axis L1 of the respective arm 130 between the anchor 120 and the attachment portion 140. The direction and magnitude of the force and torque applied on a tooth by a biasing portion 150 depends, at least in part, on the shape, width, thickness, length, material, shape set conditions, and other parameters of the biasing portion 150. As such, one or more aspects of the arm 130 and/or biasing portion 150 (including the aforementioned parameters) may be varied so that the arm 130 and/or biasing portion 150 produce a desired tooth movement when the appliance 100 is installed in the patient's mouth. Each arm 130 and/or biasing portion 150 may be designed to move one or more teeth in one, two, or all three translational directions (i.e., mesiodistal, buccolingual, and occlusogingival) and/or in one, two, or all three rotational directions (i.e., buccolingual root torque, mesiodistal angulation and mesial out-in rotation).

The biasing portions 150 of the present technology can have any length, width, shape, and/or size sufficient to move the respective tooth towards a desired FTA. In some embodiments, one, some, or all of the arms 130 may have one or more inflection points along a respective biasing portion 150. The arms 130 and/or biasing portions 150 may have a serpentine configuration such that the arm 130 and/or biasing portion 150 doubles back on itself at least one or more times before extending towards the attachment portion 140. In FIG. 1B, the arm 130 doubles back on itself two times along the biasing portion 150, thereby forming first and second concave regions facing in generally different directions relative to one another. The open loops or overlapping portions of the arm 130 corresponding to the biasing portion 150 may be disposed on either side of a plane P bisecting an overall width W of the arm 130 such that the extra length of the arm 130 is accommodated by the space medial and/or distal to the arm 130. This allows the arm 130 to have a longer length (as compared to a linear arm) to accommodate greater tooth movement, despite the limited space in the occlusal-gingival or vertical dimension between the anchor 120 and the location at which the arm 130 attaches to the tooth.

It will be appreciated that the biasing portion 150 may have other shapes or configurations. For example, in some embodiments the arm 130 and/or biasing portion 150 may include one or more linear regions that zig-zag towards the attachment portion 140. One, some, or all of the arms 130 and/or biasing portions 150 may have only linear segments or regions, or may have a combination of curved and linear regions. In some embodiments, one, some, or all of the arms 130 and/or biasing portions 150 do not include any curved portions.

According to some examples, a single arm 130 may have multiple biasing portions 150. The multiple biasing portions 150 may be in series along the longitudinal axis L1 of the respective arm 120. In some embodiments, multiple arms 130 may extend in parallel between two points along the same path or along different paths. In such embodiments, the different arms 130 may have the same stiffness or different stiffnesses.

In those embodiments where the appliance 100 has two or more arms 130 with biasing portions 150, some, none, or all of the arms 130 may have the same or different lengths, the same or different widths, the same or different thicknesses, the same or different shapes, and/or may be made of the same or different materials, amongst other properties. In some embodiments, less than all of the arms 130 have biasing portions 150. Arms 130 without biasing portions 150 may, for example, comprise one or more rigid connections between the anchor 120 and the attachment portion 140. In some embodiments, none of the arms 130 of the appliance 100 have a biasing portion 150.

The appliances of the present technology may include any number of arms 130 suitable for repositioning the patient's teeth while taking into account the patient's comfort. Unless explicitly limited to a certain number of arms in the specification, the appliances of the present technology may comprise a single arm, two arms, three arms, five arms, ten arms, sixteen arms, etc. In some examples, one, some, or all of the arms 130 of the appliance may be configured to individually connect to more than one tooth (i.e., a single arm 130 may be configured to couple to two teeth at the same time). In these and other embodiments, the appliance 100 may include two or more arms 130 configured to connect to the same tooth at the same time.

Any portion of the appliances of the present technology may include a biasing portion 150. For example, in some embodiments, portions thereof (e.g., the anchor(s), the arm(s), the biasing portion(s), the attachment portion(s), the link(s), etc.) may comprise one or more superelastic materials.

Additional details related to the individual directional force(s) applied via the biasing portion 150 or, more generally the arm 130, are described in U.S. Patent Publication No. 2017/0156823 A1, the disclosure of which is incorporated by reference herein in its entirety.

The appliances disclosed herein and/or any portion thereof (e.g., the anchor(s), the arm(s), the biasing portion(s), the attachment portion(s), the link(s), etc.) may comprise one or more superelastic materials. The appliances disclosed herein and/or any portion thereof (e.g., the anchor(s), the arm(s), the biasing portion(s), the attachment portion(s), the link(s), etc.) may comprise Nitinol, stainless steel, beta-titanium, cobalt chrome, MP35N, 35N LT, one or more metal alloys, one or more polymers, one or more ceramics, and/or combinations thereof.

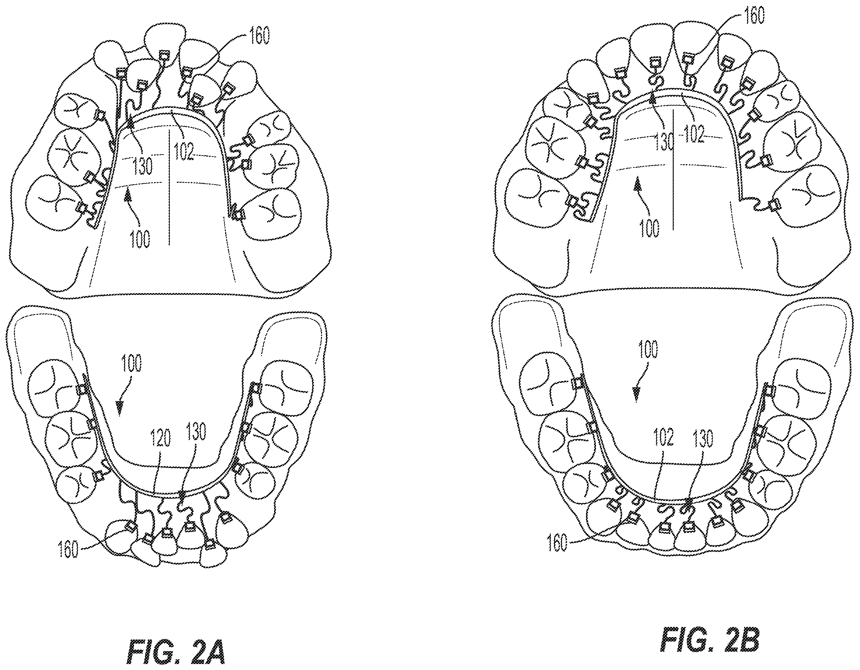

FIGS. 2A and 2B are elevation views of the appliance 100 installed on both the upper and lower arches of a patient's mouth M with the arms 130 coupled to securing members 160 attached to the lingual surfaces of the teeth. It will be appreciated that the appliance 100 of one or both of the upper and lower arches may be positioned proximate a buccal side of a patient's teeth, and that the securing elements 160 and/or arms 130 may alternatively be coupled to the buccal surface of the teeth.

FIG. 2A shows the teeth in an OTA with the arms 130 in a deformed or loaded state, and FIG. 2B shows the teeth in the FTA with the arms 130 in a substantially unloaded state. When the arms 130 are initially secured to the securing members 160 when the teeth are in the OTA, the arms 130 are forced to take a shape or path different than their "as designed" configurations. Because of the inherent memory of the resilient biasing portions 150, the arms 130 impart a continuous, corrective force on the teeth to move the teeth towards the FTA, which is where the biasing portions 150 are in their as-designed or unloaded configurations. As such, tooth repositioning using the appliances of the present technology can be accomplished in a single step, using a single appliance. In addition to enabling fewer office visits and a shorter treatment time, the appliances of the present technology greatly reduce or eliminate the pain experienced by the patient as the result of the teeth moving as compared to braces. With traditional braces, every time the orthodontist makes an adjustment (such as installing a new archwire, bending the existing archwire, repositioning a bracket, etc.), the affected teeth experience a high force which is very painful for the patient. Over time, the applied force weakens until eventually a new wire is required. The appliances of the present technology, however, apply a movement-generating force on the teeth continuously while the appliance is installed, which allows the teeth to move at a slower rate that is much less painful (if painful at all) for the patient. Even though the appliances disclosed herein apply a lower and less painful force to the teeth, because the forces being applied are continuous and the teeth can move independently (and thus more efficiently), the appliances of the present technology arrive at the FTA faster than traditional braces or aligners, as both alternatives require intermediate adjustments.

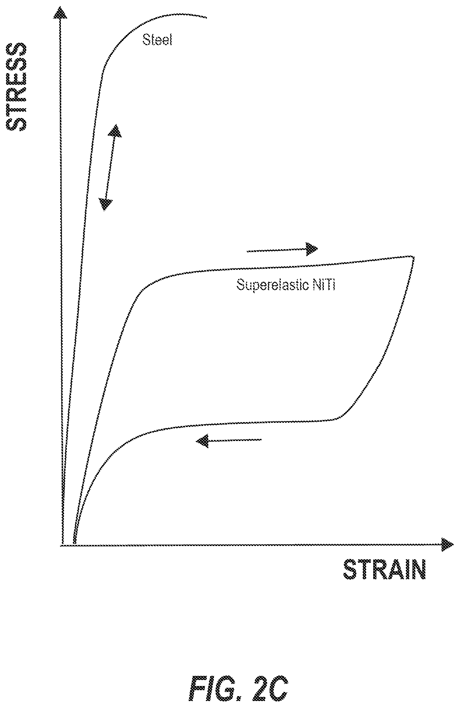

In many embodiments, the movement-generating force is lower than that applied by traditional braces. In those embodiments in which the appliance comprises a superelastic material (such as nitinol), the superelastic material behaves like a constant force spring for certain ranges of strain, and thus the force applied does not drop appreciably as the tooth moves. For example, as shown in the stress-strain curves of nitinol and steel in FIG. 2C, the curve for nitinol is relatively flat compared to that of steel. Thus, the superelastic connectors, biasing portions, and/or arms of the present technology apply essentially the same stress for many different levels of strain (e.g., deflection). As a result, the force applied to a given tooth stays constant as the teeth move during treatment, at least up until the teeth are very close or in the final arrangement. The appliances of the present technology are configured to apply a force just below the pain threshold, such that the appliance applies the maximum non-painful force to the tooth (or teeth) at all times during tooth movement. This results in the most efficient (i.e., fastest) tooth movement without pain.

In some embodiments, tooth repositioning may involve multiple steps performed progressively, by using multiple appliances. Embodiments involving multiple steps (or multiple appliances, or both) may include one or more intermediate tooth arrangements (ITAs) between an original tooth arrangement (OTA) and a desired final tooth arrangement (FTA). Likewise, the appliances disclosed herein may be designed to be installed after a first or subsequently used appliance had moved the teeth from an OTA to an ITA (or from one ITA to another ITA) and was subsequently removed. Thus, the appliances of the present technology may be designed to move the teeth from an ITA to an FTA (or to another ITA). Additionally or alternatively, the appliances may be designed to move the teeth from an OTA to an ITA, or from an OTA to an FTA without changing appliances at an ITA.

In some embodiments, the appliances disclosed herein may be configured such that, once installed on the patient's teeth, the appliance cannot be removed by the patient. In some embodiments, the appliance may be removable by the patient.

Any of the example appliances or appliance portions described herein may be made of any suitable material or materials, such as, but not limited to nitinol, stainless steel, beta-titanium, cobalt chrome or other metal alloy, polymers or ceramics, and may be made as a single, unitarily-formed structure or, alternatively, in multiple separately formed components connected together in single structure. However, in particular examples, the rigid bars, bracket connectors and loop or curved features of an appliance (or portion of an appliance) described in those examples are made by cutting a two dimensional (2D) form of the appliance from a 2D sheet of material and bending the 2D form into a desired 3D shape of the appliance, according to processes as described in more detail below. Additionally or alternatively, such appliances (or portions of appliances) can be formed using any suitable techniques, including those described in U.S. Patent Publication No. 2017/0156823 A1, which is hereby incorporated by reference in its entirety.

### III. Selected Methods for Manufacturing Orthodontic Appliances and Fixtures

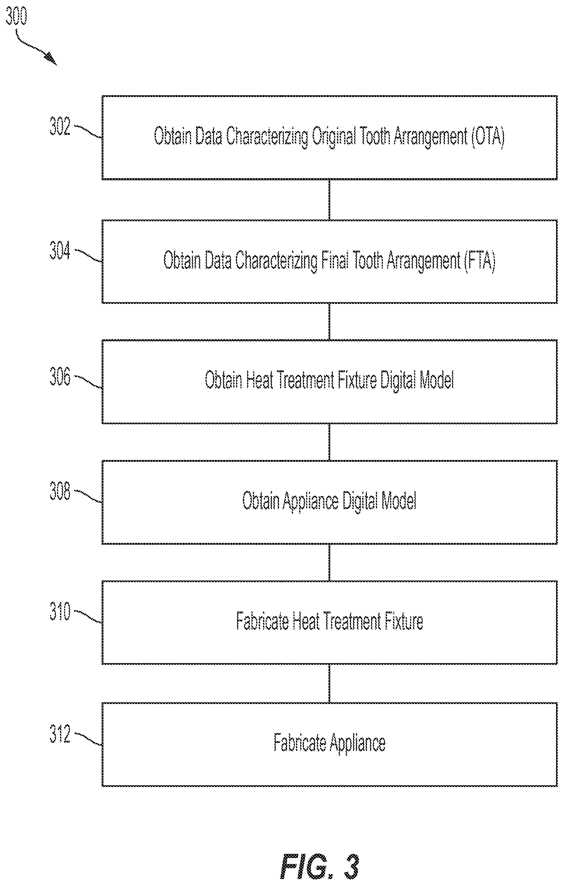

FIG. 3 illustrates an example process 300 for designing and fabricating an orthodontic appliance as described elsewhere herein. The particular processes described herein are exemplary only, and may be modified as appropriate to achieve the desired outcome (e.g., the desired force applied to each tooth by the appliance, the desired material properties of the appliance, etc.). In various embodiments, other suitable methods or techniques can be utilized to fabricate an orthodontic appliance. Moreover, although various aspects of the methods disclosed herein refer to sequences of steps, in various embodiments the steps can be performed in different orders, two or more steps can be combined together, certain steps may be omitted, and additional steps not expressly discussed can be included in the process as desired.

As noted above, in some embodiments an orthodontic appliance is configured to be coupled to a patient's teeth while the teeth are in an original tooth arrangement (OTA). In this position, elements of the appliance exert customized loads on individual teeth to urge them toward a desired final tooth arrangement (FTA). For example, an arm 130 of the appliance 100 can be coupled to a tooth and configured to apply a force so as to urge the tooth in a desired direction toward the FTA. In one example, an arm 130 of the appliance 100 can be configured to apply a tensile force that urges the tooth lingually along the facial-lingual axis. By selecting the appropriate dimensions, shape, shape set, material properties, and other aspects of the arms 130, a customized load can be applied to each tooth to move each tooth from its OTA toward its FTA. In some embodiments, the arms 130 are each configured such that little or no force is applied once the tooth to which the arm 130 is coupled has achieves its FTA. In other words, the appliance 100 can be configured such that the arms 130 are at rest in the FTA state.

As shown in FIG. 3, the process 300 can begin at block 302 with obtaining data (e.g., position data) characterizing the patient's original tooth arrangement (OTA). In some embodiments, the operator may obtain a digital representation of the patient's OTA, for example using optical scanning, cone beam computed tomography (CBCT), scanning of patient impressions, or other suitable imaging technique to obtain position data of the patient's teeth, gingiva, and optionally other adjacent anatomical structures while the patient's teeth are in the original or pre-treatment condition.

The process 300 continues at block 304 with obtaining data (e.g., position data) characterizing the patient's intended or desired final tooth arrangement (FTA). The data characterizing the FTA can include coordinates (e.g., X,Y,Z coordinates) for each of the patient's teeth and the gingiva, Additionally or alternatively, such data can include positioning of each of the patient's teeth relative to other ones of the patient's teeth and/or the gingiva. In some embodiments, the operator can obtain a digital representation of the patient's FTA, for example, an FTA digital model generated using segmentation software (e.g., iROK Digital Dentistry Studio) to create individual virtual teeth and gingiva from the OTA data. In some embodiments, digital models of the securing members 160 can be added to the segmented OTA digital model (e.g., by an operator selecting positions on the lingual surface (or other suitable surface) for placement of securing members 160 thereon). Suitable software can be used to move the virtual teeth with the attached securing members 160 from the OTA to a desired final position (e.g., the FTA), with or without the securing members digital models included.

At block 306, a heat treatment fixture digital model can be obtained. In some embodiments, the heat treatment fixture digital model can correspond to and/or be derived from one or more anatomical digital models (e.g., the OTA digital model, the FTA digital model, combinations thereof, etc.). For example, the anatomical digital model can be modified (e.g., using MeshMixer or other suitable modeling software) in a variety of ways to render a model suitable for manufacturing a heat treatment fixture. In some embodiments, multiple anatomical digital models can be combined to form the heat treatment fixture digital model. For example, a gingival portion of the OTA digital model can be combined with a teeth portion and/or a securing member portion of the FTA digital model to form the heat treatment fixture digital model. In some embodiments, the anatomical digital model can be modified to replace the securing members 160 (which are configured to couple to arms 130 of an appliance 100 (FIGS. 2A and 2B)) with hook-like members (which can be configured to facilitate temporary coupling of the heat treatment fixture to the appliance for shape-setting). Additionally or alternatively, the anatomical digital model can be modified to enlarge or thicken the gingiva, to remove one or more of the teeth, and/or to add structural components for increased rigidity. In some embodiments, enlarging or thickening the gingiva may be done to ensure portions (e.g., the anchor) of the fabricated appliance, which is based in part on the anatomical digital model, does not engage or contact the patient's gingiva when the appliance is installed. As a result, modifying the anatomical digital model as described herein may be done to provide a less painful teeth repositioning experience for the patient.

The process 300 continues at block 308 with obtaining an appliance digital model. As used herein, the term "digital model" and "model" are intended to refer to a virtual representation of an object or collection of objects. For example, the term "appliance digital model" refers to the virtual representation of the structure and geometry of the appliance, including its individual components (e.g., the anchor, arms, biasing portions, attachment portions, etc.). In some embodiments, a substantially planar digital model of the appliance is generated based at least in part on the heat treatment fixture digital model (and/or the FTA digital model). According to some examples, a contoured or 3D appliance digital model generally corresponding to one or more portions of the FTA and/or the OTA can first be generated that conforms to the surface and attachment features of the heat treatment fixture digital model. In some embodiments, the 3D appliance digital model can include generic arm portions and securing members, without particular geometries, dimensions, or other properties of the arms being selected or defined by a particular patient. The 3D appliance digital model may then be flattened to generate a substantially planar or substantially 2D appliance digital model. In some embodiments, the particular configuration of the arms 130 (e.g., the geometry of biasing portions 150, the position along the anchor 120 (FIG. 1B), etc.) can then be selected so as to apply the desired force to urge the corresponding tooth (to which the arm 130 is attached) from its OTA toward its FTA. As noted previously, in some embodiments the arms are configured so as to be substantially at rest or in a substantially unstressed state when at the FTA. The selected arm configurations can then be substituted or otherwise incorporated into the planar appliance digital model.

At block 310, the heat treatment fixture can be fabricated. For example, using the heat treatment fixture digital model (block 306), the heat treatment fixture can be cast, molded, 3D printed, or otherwise fabricated using suitable materials configured to withstand heating for shape setting of an appliance thereon.

At block 312, the appliance can be fabricated. In some embodiments, fabricating the appliance includes first fabricating the appliance in a planar configuration based on the planar appliance digital model. For example, the planar appliance can be cut out of a sheet of metal or other suitable material. In some embodiments, the appliance is cut out of a sheet of Nitinol or other metal using laser cutting, water jet, stamping, chemical etching, machining, or other suitable technique. The thickness of the material can be varied across the appliance, for example by electropolishing, etching, grinding, depositing, or otherwise manipulating the material of the appliance to achieve the desired material properties.

According to some examples, the planar member (e.g., as cut out from a sheet of metal) can be bent or otherwise manipulated into the desired arrangement (e.g., substantially corresponding to one or more portions of the FTA and/or the OTA) to form a contoured appliance. In some embodiments, the planar appliance can be bent into position by coupling the planar appliance to the heat treatment fixture fabricated at block 310. For example, the arms of the appliance can be removably coupled to hook members of the heat treatment fixture, and optionally ligature wire or other temporary fasteners can be used to secure the arms or other portions of the appliance to the heat treatment fixture. The resulting assembly (i.e., the appliance fastened to the heat treatment fixture) can then be heated to shape-set the appliance into its final form. In some embodiments, the final form of the appliance can correspond or substantially correspond to the FTA and/or the OTA. For example, when the appliance is in its final form, the anchor portion of the appliance can substantially correspond to the gingiva from the OTA while the arms of the appliance substantially correspond to the teeth in the FTA. As a result, the appliance is configured to be in an unstressed, or nearly unstressed, state in the FTA. In operation, the appliance can then be installed in the patient's mouth (e.g., by bending or otherwise manipulating arms of the appliance to be coupled to brackets of the patient's teeth while in the OTA). Due to the shape set of the appliance and the geometry of the arms and anchor, the arms will tend to urge each tooth away from its OTA and toward the FTA.

Additional details and further examples of processes for designing and fabricating appliances and heat treatment fixtures are described below. The particular processes disclosed herein are exemplary, and may be modified as needed to achieve the desired outcome (e.g., the desired force applied to each tooth by the appliance, the desired material properties of the final appliance, etc.). Moreover, although various aspects of the methods disclosed herein refer to sequences of steps, in various embodiments the steps can be performed in different orders, two or more steps can be combined together, certain steps may be omitted, and additional steps not expressly discussed can be included in the process as desired.

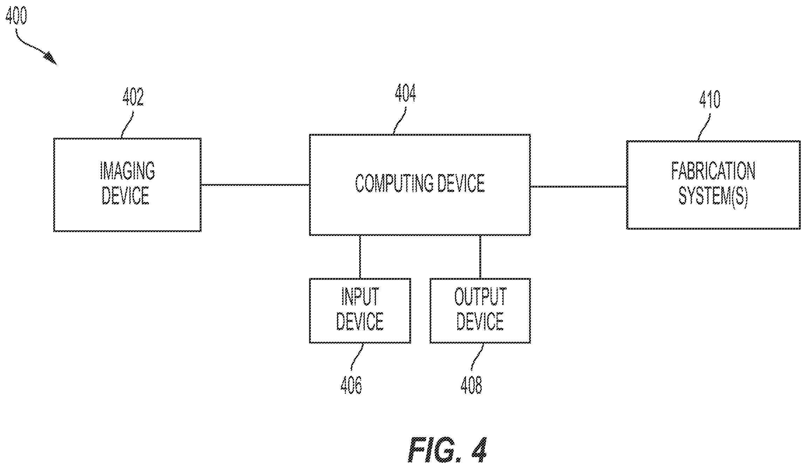

Several of the methods disclosed herein can be performed using one or more aspects of a manufacturing system 400 shown schematically in FIG. 4. The system 400 can include an imaging device 402 communicatively coupled to a computing device 404. The imaging device 402 can include any suitable device or collection of devices configured to obtain image data or other digital representation of a patient's teeth, gingiva, and other dental anatomy. For example, the imaging device 402 can include an optical scanning device (e.g., as commercially sold by ITERO, 3SHAPE, and others), a cone-beam computed tomography scanner, or any other suitable imaging device. In some embodiments, the imaging device 402 can be any suitable device for obtaining a digital representation of a patient's anatomy (e.g., the OTA), even if such digital representation is not based on and does not result in a graphical representation of the patient's anatomy.

The computing device 404 can be any suitable combination of software and hardware. For example, the computing device 404 can include a special purpose computer or data processor that is specifically programmed, configured, or constructed to perform one or more of the computer-executable instructions explained in detail herein. Additionally or alternatively, the computing device 404 can include a distributed computing environment in which tasks or modules are performed by remote processing devices, which are linked through a communication network (e.g., a wireless communication network, a wired communication network, a cellular communication network, the Internet, a short-range radio network (e.g., via Bluetooth)). In a distributed computing environment, program modules may be located in both local and remote memory storage devices.

Computer-implemented instructions, data structures, and other data under aspects of the technology may be stored or distributed on computer-readable storage media, including magnetically or optically readable computer disks, as microcode on semiconductor memory, nanotechnology memory, organic or optical memory, or other portable and/or non-transitory data storage media. In some embodiments, aspects of the technology may be distributed over the Internet or over other networks (e.g. a Bluetooth network) on a propagated signal on a propagation medium (e.g., an electromagnetic wave(s), a sound wave) over a period of time, or may be provided on any analog or digital network (packet switched, circuit switched, or other scheme).

The system 400 can also include one or more input devices 406 (e.g., touch screen, keyboard, mouse, microphone, camera, etc.) and one or more output devices 408 (e.g., display, speaker, etc.) coupled to the computing device 404. In operation, a user can provide instructions to the computing device 404 and receive output from the computing device 404 via the input and output devices 406 and 408.

As shown in FIG. 4, the computing device 404 may be connected to one or more fabricating systems 410 (including fabricating machines) for fabricating appliances, heat treatment fixtures, and any other components thereof and associated tools, as described herein. The computing device 404 can be connected to the fabricating system(s) 410 by any suitable communication connection including, but not limited to a direct electronic connection, network connection, or the like. Alternatively, or in addition, the connection may be provided by delivery to the fabricating system 410 of a physical, non-transient storage medium on which data from the computing device 404 has been stored.

### Methods of Designing Orthodontic Appliances and Fixtures

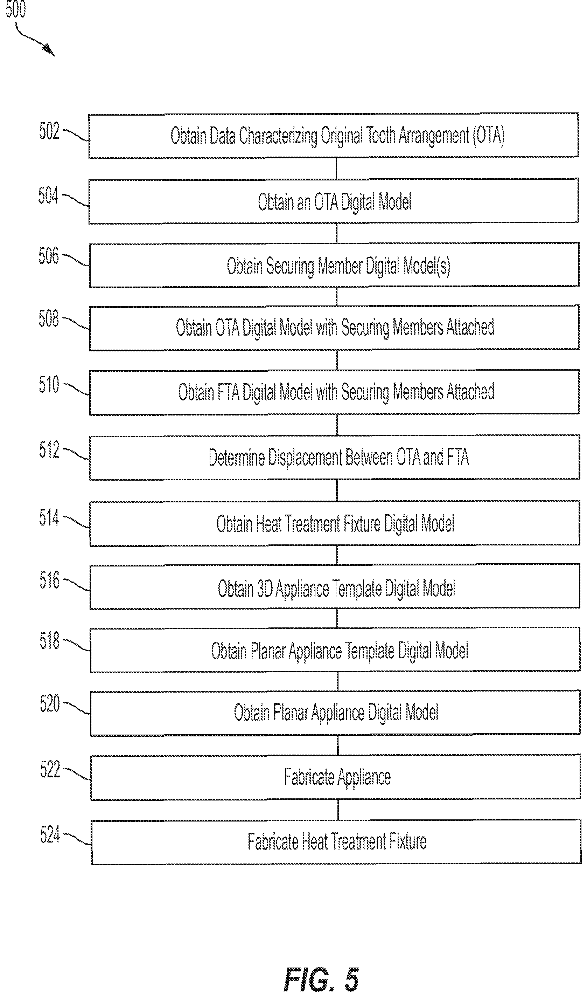

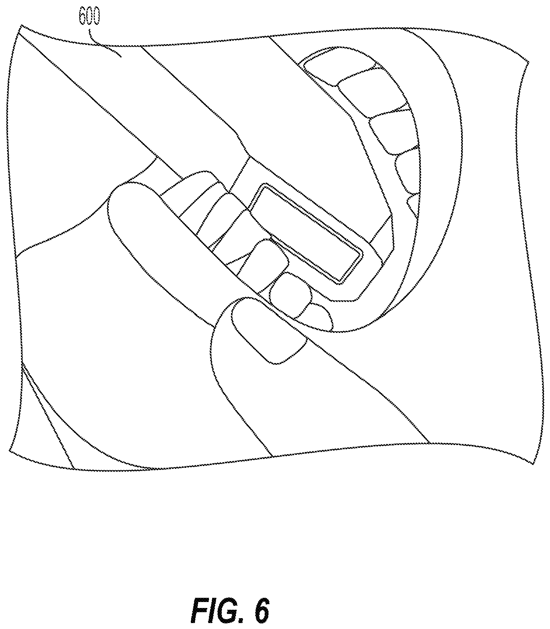

FIG. 5 is a flow diagram of a process 500 for designing an orthodontic appliance. The process 500 begins at block 502 with obtaining data characterizing an original tooth arrangement (OTA). For example, as shown in FIG. 6, the OTA data can be obtained by scanning the patient's teeth using an intraoral optical scanner 600. Such a scanner 600 can be used to scan both the patient's upper and lower teeth to generate a 3D model of each. The scanning can be performed using any suitable technique, for example a dental cone beam CT scanner, or magnetic resonance imaging (MRI), or similar device or technique. In various examples, the OTA data can include data associated with the roots of the teeth as well as the exposed portions, which may be advantageous in designing an appropriate orthodontic appliance. In some examples, the OTA data can be obtained using an impression made of the patient's upper and lower jaws (e.g., using polyvinyl siloxane or any other suitable impression material). The impression can then be scanned to create 3D data, which can include the relationship between the upper and lower jaw (e.g., to record the patient's bite). In examples in which impressions are used, the relationship between the teeth in the upper and lower arches (inter-arch relationship) can be obtained by taking a wax bite of the patient in the centric position. In various embodiments, the OTA data can be obtained directly (e.g., by imaging the patient's mouth using an appropriate imaging device) or indirectly (e.g., by receiving pre-existing OTA data from an operator or another source).

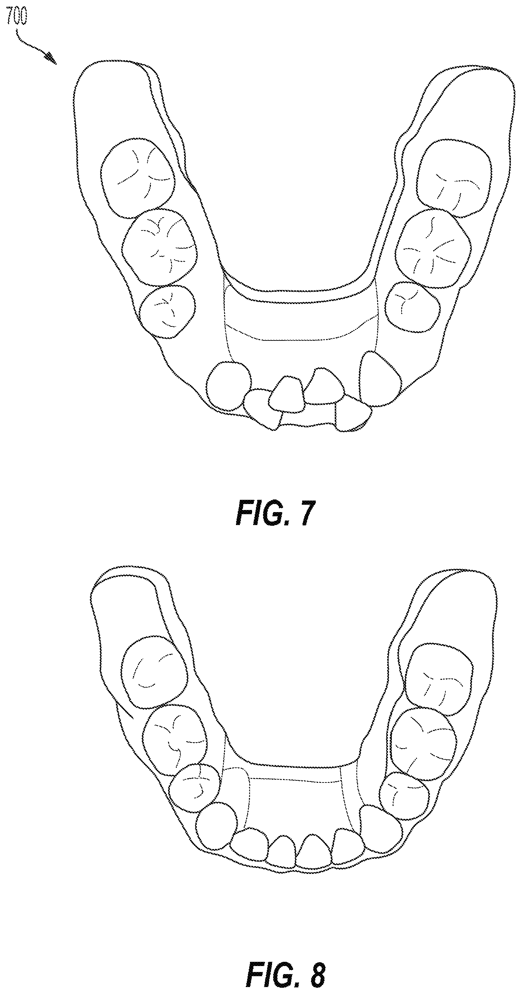

Returning to FIG. 5, the process 500 continues with obtaining an OTA digital model at block 504. FIG. 7 is a graphical representation of an example of an OTA digital model 700. The digital model 700 can virtually represent or characterize the arrangement of the patient's teeth and gingiva in the original tooth arrangement. As seen in FIG. 7, the teeth in the OTA may be maloccluded, mis-aligned, crowded, or otherwise in need of orthodontic correction. In some embodiments, one or more teeth present in the OTA may be designated for extraction prior to use of the orthodontic appliance.

In some embodiments, obtaining the OTA digital model corresponding to the OTA data can include first obtaining a single complex 3D database of the patient's jaw, which is then segmented to separate the patient's teeth into separate 3D bodies (e.g., individual teeth or blocks of multiple teeth) that can then be manipulated virtually by an operator. Such segmentation can be performed using any suitable techniques or software, for example using iROK Digital Dentistry Studio or other suitable software. Following segmentation, the resulting 3D databases upper and lower teeth can include a model of the gingiva and independent models of each tooth. As a result, the OTA data can be manipulated by an operator to virtually move teeth relative to the gingiva. As described in more detail elsewhere herein, the teeth can be manipulated from the OTA towards a final tooth arrangement (FTA). FIG. 8 illustrates an example final tooth arrangement (FTA). As seen in FIG. 8, the teeth in the FTA may be more aligned, less mal-occluded, and otherwise aesthetically and functionally improved relative to the OTA (e.g., as reflected in the digital model 700). In some embodiments, the FTA can have desired or favorable inter-arch and intra-arch arrangements, for example, based on an operator's prescription. For example, one or more (or all) teeth from the upper or lower jaws (or both) are moved until their cusps have a good interdigitation and fit.

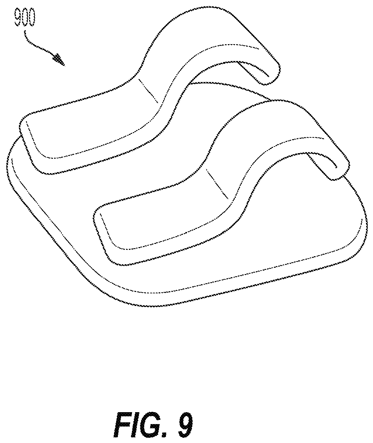

Referring back to FIG. 5, the process 500 continues in block 506 with obtaining securing member digital model(s). As discussed previously, securing members (e.g., securing members 160, brackets, etc.) can be coupled to the patient's teeth to allow for an orthodontic appliance (e.g., appliance 10) to be mated thereto. The securing member digital models can include virtual representation of the geometry and/or other structural characteristics of the securing member(s). In various embodiments, the securing member digital models can be identical for each securing member, or may vary among the securing members. For example, different securing members may be used for molars than for incisors. FIG. 9 illustrates an example securing member digital model 900.

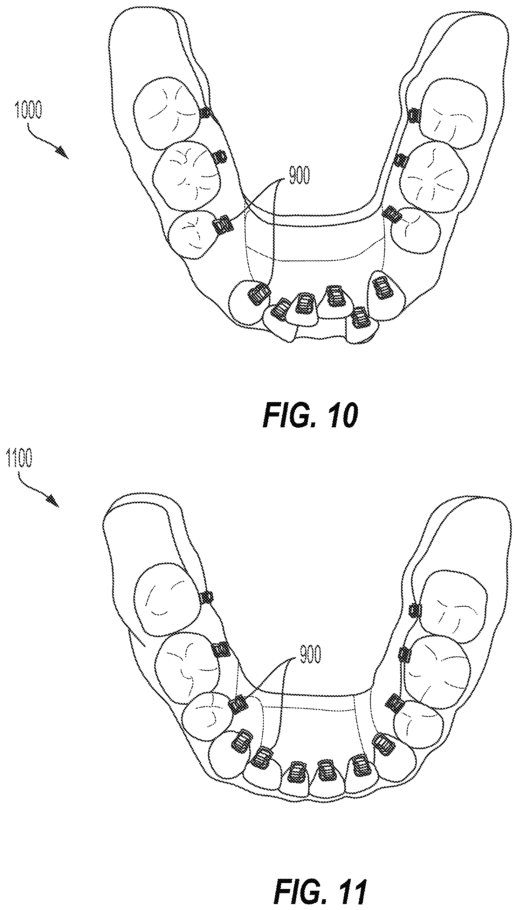

With continued reference to FIG. 5, the process 500 continues in block 508 with obtaining an OTA digital model with securing members attached. For example, the securing member digital model 900 (FIG. 9) can be applied to appropriate locations on the patient's teeth within the OTA digital model 700 (FIG. 7). The resulting digital model 1000 is shown in FIG. 10, in which a plurality of digital models of securing members 900 are disposed along lingual surfaces of the patient's teeth. In some embodiments, in the digital model 1000, each of the patient's teeth can have a securing member coupled thereto. As noted previously, an orthodontic appliance can include a plurality of arms having attachment portions configured to be coupled to securing members (e.g., brackets) that are attached to the patient's teeth.

In some examples, the digital models 900 of the securing members can be virtually positioned on the teeth in the OTA using appropriate software (e.g., iROK Digital Dentistry Studio). In some embodiments, virtually positioning the securing members can include selecting virtual models of particular securing members from a library of available securing members, and then positioning the selected securing members on one or more teeth. In some embodiments, the bracket positioning can be assigned automatically (e.g., by automatically positioning the bracket in a central or the pre-defined portion of the tooth) or manually (e.g., by an operator selecting and/or manipulating the attachment location for each securing member). In some embodiments, the position of each securing member can be refined by the operator as desired. For example, it may be desirable to position the securing members as close to the gingiva as possible so as to avoid interference with securing members on the other jaw or interference with the teeth from the other jaw when the mouth is closed.

In some embodiments, the digital model 1000 with the teeth in the OTA and securing members attached thereto can be used to determine a configuration of a bonding tray, which may then be used to physically attach securing members to the patient's teeth by an operator. For example, the bonding tray can be configured to fit over the patient's teeth similar to an aligner, and can include recesses on a side of each tooth that are sized and configured to receive an appropriate securing member (e.g., bracket) therein. In various embodiments, such recesses can be positioned on the lingual, buccal, mesial/distal, occlusal, root, or any suitable surface of a tooth to which a corresponding bracket is intended to be bonded. In operation, an appropriate securing member can be placed in each recess and then an adhesive (e.g., an adhesive that cures when illuminated by ultraviolet light) can be applied to the bonding surface of each securing member. The tray can then be placed over the patient's teeth and the adhesive cured to bond all the securing members to the appropriate location on each tooth.

To generate such a bonding tray, the digital model 1000 can be used, which characterizes the teeth in the OTA with securing members attached. The digital model 1000 may be further manipulated, for example, to remove excess virtual gingiva to limit the size of the tray to only what is necessary to hold the securing members in position against the patient's teeth. The trimmed digital model can then be used to generate a physical 3D model of the patient's teeth with the securing members disposed thereon, for example using 3D printing in a polymer resin or other suitable technique.

In some embodiments, a suitable material (e.g., a clear polymer resin) can then be formed over (e.g., thermoformed over) the physical model of the patient's teeth with securing members in the OTA. This can create the aligner-like tray with recesses shaped and configured to receive securing members therein. The securing members can then be placed into corresponding recesses of the tray, and the tray can be applied to the patient's teeth with a curable adhesive to attach the securing members to the patient's teeth in the OTA. The tray may then be removed, leaving the securing members in place.

In some embodiments, the bonding tray can be 3D printed directly, without the need for a physical model of the patient's teeth and without the use of thermoforming. For example, a digital model of a bonding tray can be derived from the digital model 1000 characterizing the teeth in the OTA with securing members attached. In some embodiments, a negative of the digital model 1000 can be generated, and can be trimmed to provide a general tray-like structure with a surface corresponding to the teeth and securing members in the digital model 1000. This resulting model can be manipulated to provide features for retaining brackets in the corresponding recesses. Finally, the bonding tray can be 3D printed based on this digital model, for example using 3D printable polymer resins or other suitable materials or deposition techniques.

Alternatively, the operator may attach securing members to the patient's teeth directly, without the assistance of a tray.

Referring back to FIG. 5, the process 500 continues at block 510 with obtaining an FTA digital model 1100 (FIG. 11) with securing members 900 attached. For example, the digital model 1000 (FIG. 10) of the teeth in OTA with models of the securing members 900 attached thereto can be used to generate the FTA digital model 1100 (FIG. 11). In some embodiments, the digital model 1000 can be manipulated to place the teeth in the FTA.

The FTA digital model 1100 can be derived based at least in part on data characterizing the teeth in the FTA. Such FTA data can include a digital representation of the desired final positions and orientations of the patient's teeth relative to one another and to the gingiva. The FTA data can be obtained directly (e.g., generated by the operator) or may be received from an external source (e.g., the FTA data may be generated by a third party and provided to an operator for design of an appropriate orthodontic appliance).

In some embodiments, the FTA data can be obtained by manipulating the OTA data to virtually move the patient's teeth. Suitable software, such as iROK Digital Dentistry Studio, can be used by an operator to move the teeth to a desired FTA. In some embodiments, virtual movement of the teeth relative to the OTA also results in movement of the gingiva relative to the OTA in order to maintain the natural look of the gingiva and more accurately reflect the orientation and position of the gingiva when the teeth are at the FTA. This movement of the gingiva can be achieved using gingiva morphing or other suitable technique.

In some embodiments, the FTA can reflect changes to the patient's teeth that may occur as part of the treatment process. For example, an operator may extract one or more teeth of the patient, due to lack of space for all the teeth to fit in the arch (or other reasons), as part of the treatment. In that event, the extracted teeth can be excluded from the FTA data. If the operator decides that the teeth need to become smaller due to a lack of space, then interproximal reduction (IPR) may be performed on the patient. In this case, stripping and reducing the size of the teeth in the FTA can be performed so as to match the IPR done by the operator.

In some embodiments, a proposed FTA can be developed by an operator (e.g., independently or based in whole or in part on input from a treating orthodontist) and then sent to a treating orthodontist for review and comment. If the treating orthodontist has comments, she can provide input to the operator (e.g., written notes, proposed manipulation of one or more teeth or securing members, etc.) that can be transmitted electronically or otherwise. The operator may then revise the FTA and send a revised proposed FTA back to the treating orthodontist for further review and comment. This iterative process may repeat until the treating orthodontist approves the proposed FTA, and the resulting digital model 1100.

Additionally or alternatively, an FTA digital model (e.g., as depicted in FIG. 8) can be manipulated to have digital models of securing members 900 coupled to the teeth at appropriate locations. In some embodiments, the relative position of each securing member relative to its respective tooth may be obtained or derived from the digital model 1000 (FIG. 10), in which the securing members are attached to the teeth in the OTA. In some embodiments, the securing members may be first positioned on the teeth in the FTA to generate the digital model 1100 (FIG. 11), and this model may in turn be used to generate the digital model 1000 (FIG. 10), for example by manipulating the digital model 1100 to move the teeth to the OTA.

Referring back to FIG. 5, the process 500 continues at block 512 with determining the displacements of individual teeth or groups or teeth between the OTA and the FTA. For example, the displacement of each tooth between the OTA and FTA can be described using six degrees of freedom (e.g., translation along X, Y, and Z axes, and rotation around the same three axes; or alternatively translation along mesiodistal, buccolingual, and/or occlusogingival directions, and rotation in the form of buccolingual root torque, mesiodistal angulation, and/or mesial out-in rotation). In some embodiments, these values can be determined by calculating the difference between the location of each tooth in the FTA data and the OTA data. This can be performed for each tooth in each jaw to generate a dataset that includes the required displacement along six degrees of freedom for each tooth.

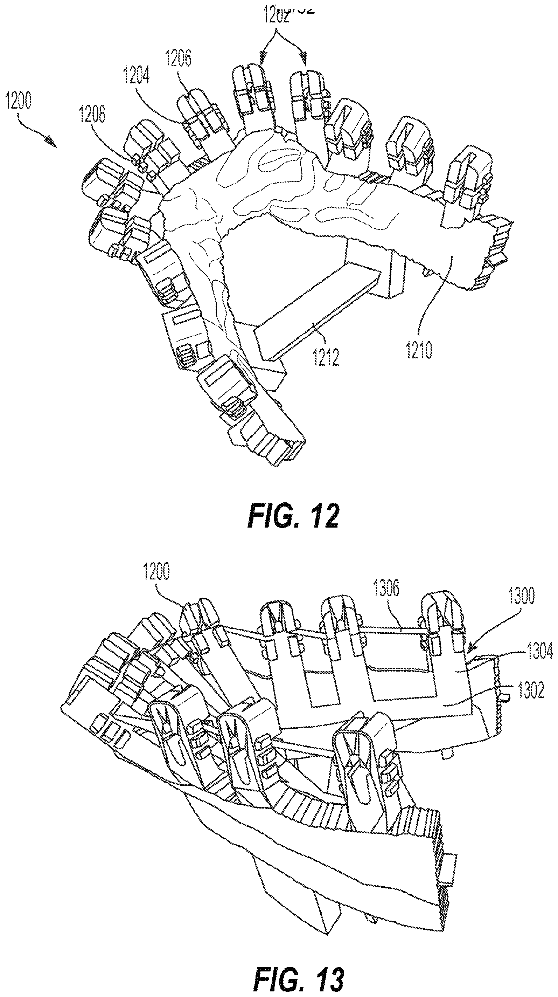

The process 500 continues at block 514 with obtaining a heat treatment fixture digital model. FIG. 12 illustrates an example fixture digital model 1200, which can be generated by manipulating the digital model 700 (FIG. 7) of the OTA, the digital model 800 (FIG. 8) of the FTA, the digital model 1000 (FIG. 10) of the OTA with securing members attached, and/or the digital model 1100 (FIG. 11) of the FTA with securing members attached. For example, the digital model(s) 700, 800, 1000, 1100 can be manipulated to generate a digital representation of a fixture (e.g., a heat treatment fixture) for use in manufacturing an appliance. The digital model(s) 700, 800, 1000, 1100 can be manipulated in a number of ways to generate suitable fixture data. In some embodiments, such manipulation can be performed using suitable software, e.g. MeshMixer by Audodesk^{®}.

In some examples, the securing members in the digital model(s) 1000, 1100 can be modified or substituted with appropriate securing portions 1202 that are each configured to couple to arms of an appliance and to facilitate temporary fastening of the appliance to the fixture. Additionally or alternatively, the securing portions 1202 can be added to the digital models 700, 800. For example, bracket-like securing members can be replaced with securing portions 1202 that include both horizontal channels 1204 configured to mate with attachment portions 140 of an appliance 100 as well as vertical channels 1206. A plurality of protrusions 1208 can be disposed along one or more side surfaces of the securing portions 1202. Together, the channels 1204 and 1206 and the protrusions 1208 can provide structures that are configured to receive ligature wire or other fastener therethrough. For example, an operator can couple an appliance 100 to the fixture and then wind ligature wire through the horizontal channels 1204 and within the space between adjacent protrusions 1208 to hold the appliance 100 in place against the fixture. Additionally or alternatively, the horizontal channels 1204 can be configured to mate with attachment portions 140 of the appliance 100, for example being sufficiently deep (e.g., deeper than corresponding channels of the securing members 900 of the digital model(s) 1000, 1100) to both receive the attachment portions 140 therein and to receive ligature wire or other fastener therethrough. In some embodiments, the vertical channels 1206 can be configured to mate with part of the attachment portions 140 of the appliance 100, such that a single attachment portion 140 can be partially received within a horizontal channel 1204 and partially received within a vertical channel 1206. The protrusions 1208 may additionally define grooves or recesses configured to receive the ligature wire or other elongate fastener. The fixture model 1200 can also define through-channels or apertures within each securing portion 1202. These through-channels can allow a pushing tool to be inserted from the back of the securing portion 1202 (e.g., through the buccal surface of the fixture model 1200) to push an attachment portion 40 away from the securing portion 1202 after the heat treatment has been completed and the ligature wire or other fastener has been removed.

Additionally or alternatively, the digital model(s) 700, 800, 1000, 1100 can be manipulated to alter the shape or configuration of the gingiva to generate the fixture model 1200. When an appliance is installed, a patient may suffer considerable discomfort if any portion of the appliance impinges on the gingiva. Accordingly, it can be desirable to design an appliance that rests close to the patient's gingiva without impinging upon it. In some embodiments, this can be achieved by enlarging the gingiva of the digital model(s) 700, 800, 1000, 1100 to generate the fixture model 1200. For example, the lingual surface of the gingiva in the digital model(s) 700, 800, 1000, 1100 can be expanded (e.g., moved more lingually) by a predetermined amount (e.g., less than about 1.5 mm, less than about 1.4 mm, less than about 1.3 mm, less than about 1.2 mm, less than about 1.1 mm, less than about 1.0 mm, less than about 0.9 mm, less than about 0.8 mm, less than about 0.7 mm, less than about 0.6 mm, less than about 0.5 mm, less than about 0.4 mess, less than about 0.3 mm, less than about 0.2 mm, or less than about 0.1 mm). As such, when an appliance is generated using the surface of the fixture data (e.g., the appliance 100 can be shaped to substantially correspond to a portion of the lingual surface of the fixture model 1200, as described in more detail below), the appliance can be sized and configured to rest a short distance away from the patient's gingiva without impinging thereon.

With continued reference to block 514, the digital model(s) 700, 800 without securing members attached and/or the digital model(s) 1000, 1100 with securing members attached can be manipulated to remove the teeth or other structural elements not needed for heat treating the appliance, and/or to add structural features to reinforce the fixture for sufficient rigidity during the heat treatment process. For example, as shown in FIG. 12, the fixture model 1200 does not include any teeth, but retains at least a portion of the gingival surface 1210. Additionally, the fixture model 1200 includes a stabilizing crossbar 1212 that can enhance the rigidity of the resulting fixture. Various other modifications to the digital model(s) 700, 800, 1000, 1100 can be made to achieve the desired heat treatment fixture model 1200.

Referring back to FIG. 5, the process 500 continues at block 516 with obtaining an appliance template digital model. FIG. 13 illustrates an example of an appliance template digital model 1300, shown here in a configuration mated with the fixture model 1200.

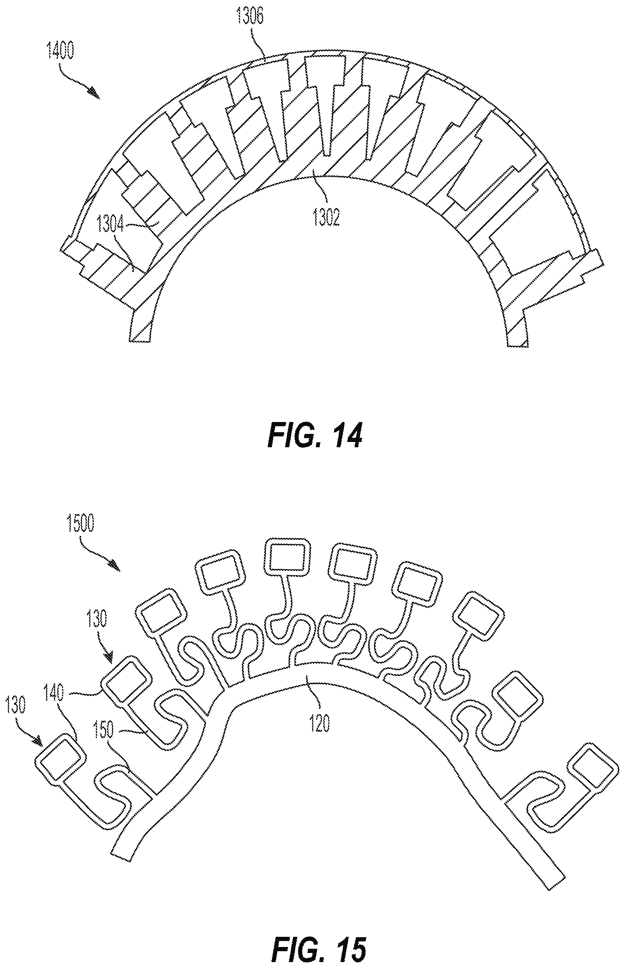

The model 1300 defines an anchor portion 1302, arm portions 1304, and an attachment bar portion 1306. These components can take the form of a genericized template for an appliance that is later customized for a particular patient (as described in more detail below with respect to FIG. 15). For example, the anchor portion 1302 can correspond to the anchor 120 of the completed appliance, and the arm portions 1304 can serve as placeholders for the arms 130 of the completed appliance. The attachment bar portion 1306 takes the form of a continuous strip connecting each of the arms 130. As shown in FIG. 13, the arm portion 1306 can be configured to be received within the channels 1204 of the securing portions 1202 of the fixture model 1200. The attachment bar portion 1306 can correspond in part to portions of the attachment portions 140 of the arms 130 of the completed appliance.

In various embodiments, the appliance template digital model 1300 can be generated using surface data of the fixture model 1200. For example, the appliance template digital model 1300 can be configured to substantially correspond to the surface of the fixture model 1200, such as the anchor portion 1302 corresponding to a contour of the fixture model 1200 that is derived from data characterizing the patient's gingiva. As noted previously, the treatment fixture model 1200 can be modified with respect to the OTA model 1100 by, among other things, enlarging the gingiva. As such, when the anchor portion 1302 contacts the gingival portion of the fixture model 1200, the anchor portion 1302 may be positioned so as to be slightly spaced apart from the actual gingiva as characterized in the OTA model 700. In some embodiments, the appliance template model 1300 can have no thickness dimension, instead corresponding to a three-dimensional surface following a contour of the fixture model 1200. In some embodiments, the appliance template model 1300 can have at least some thickness.

In block 518, the appliance template digital model 1300 can be flattened or otherwise manipulated to generate a planar appliance template model 1400 (FIG. 14). The planar template model 1400 can reflect 2-dimensional or substantially planar data corresponding to or at least derived from the contoured appliance template model 1300. For example, the appliance template digital model 1300 (FIG. 13) can be converted into the planar appliance template model 1400 (FIG. 14) by flattening, planarizing, or otherwise converting the digital model 1300 to generate the planar appliance template model 1400. Such conversion may be carried out using a processor system and appropriate software such as, but not limited to ExactFlat^{®}, Solidworks^{®}, Autodesk^{®} Inventor, Creo^{®}, or other suitable software.

At block 520, the planar appliance digital model is obtained. An example of a planar appliance model 1500 is shown in FIG. 15. In this stage, the particular shape and configuration of the arms of the appliance can be determined, such as by modifying or substituting portions or components of the planar template model 1400 (FIG. 14). For example, the particular dimensions, geometry, and material properties of arms of the appliance can be selected so as to apply the necessary force and/or torque to achieve the desired displacement determined at block 512. In some embodiments, a pre-populated library of arm designs can be used to select an appropriate design and configuration to achieve the desired displacement. In some embodiments, the arm designs in the pre-populated library can be analyzed using finite element analysis (FEA) or other techniques to determine the spring force such arms would apply when deflected by particular amounts (e.g., the amount of deflection between the FTA (when the arm is at rest) and the OTA). In some embodiments, fully or partially automated selection of particular arm designs can be reviewed and/or modified by an operator based on relevant criteria. For example, if the proposed arm designs include overlapping or otherwise interfering arms, the operator may manually adjust the shape and/or configuration of the arms.

Based on the determined displacement, the required forces and/or torques required to move each tooth from the OTA to the FTA can be determined. The forces required to move teeth are generally in the range of centiNewtons, and distances moved are typically in the range of millimeters. The amount of moment (Newton-millimeter) acting to rotate a tooth can be found by multiplying the magnitude of the applied force by the force arm. In general, the displacement can be a 3D tooth movement that combines both translational and rotational motion.

The forces and/or torques required to achieve the FTA may depend on the patient's anatomy, for example the size of the particular tooth being moved, the anatomy of the root, etc. The forces and/or torques may also depend on other physiological parameters (e.g., bone density, biological determinants, sex, ethnicity, jaw (maxilla or mandible), mechanical properties of surrounding tissues (lips, tongue, gingiva, and bone) around the moving tooth, etc.). The particular force and/or torque applied to a given tooth will also depend on the particular positioning of the securing member (e.g., bracket). For example, a securing member positioned further off a center-of-resistance of a tooth will generate more torque under a given applied force than a securing member that is positioned nearer to a center-of-resistance of the tooth. Based on the desired displacement (e.g., along six degrees of freedom), the patient's anatomy, and the location of the securing member, a particular arm configuration can be selected to generate the desired force and/or torque on the subject tooth, so as to move the tooth from the OTA to the FTA. By determining appropriate thickness, widths, shapes, and configurations of the arms and other components of the orthodontic appliance, an appliance configuration that applies forces and torques to the appropriate teeth to move the teeth to the FTA is determined.

In particular examples, the design of the appliance may be performed by an operator, with the processor system and appropriate design software such as, but not limited to CAD software such as, but not limited to Solidworks^{®}, Autodesk^{®} Inventor, Creo^{®}, or the like. FEA software such as, but not limited to Abaqus, Ansys, etc. may be employed to design the springs and arms in order to apply the desired or optimal force to the teeth. For example, such software and processing systems may be employed to design and alter the thickness, cut width, length, as well as the overall design of each arm based at least in part on the movement of the tooth to which the arm is connected.

In some examples, if a tooth needs to be displaced by a longer distance or the tooth is smaller (e.g. lower incisors), the arm 130 may be designed such that it is more flexible. In some embodiments, the selection or design of the arms 130 can account for variation in the rate of teeth movement based on direction. It is known that the rate of tooth movement when a given force is applied to the tooth is different depending on the direction of movement. For example, extrusion is the fastest movement for a given force, intrusion is the slowest, and mesiodistal and buccolingual movements are somewhere in between these two extremes. In one example, if a tooth moves 2 mm per month occlusally and 1 mm per month distally under the same applied force, the tooth will not move in a straight line as the occlusal movement will be more rapid than the distal movement. The occlusal movement will finish first, and then the tooth will move in a straight line from there in the distal direction until that motion is complete. It may be desired to move the tooth in a particular trajectory, and so the force applied distally can be different from the force applied occlusally. For example, it may be desired to move the tooth in a straight line, and so the distal force would have to be greater than the occlusal force in order to result in a straight trajectory from OTA to FTA.

In some embodiments, the arms 130 can be designed to impart less force on some or all of the teeth because of periodontal problems such as bone resorption, root resorption or attachment loss. The ability to customize the force or torque (or both) applied to each tooth can provide significant advantages over traditional orthodontics. In particular examples, the computer-aided procedure employs an algorithm for selecting or configuring an arm or other feature of an appliance, for example, from one or more predefined sets of options or one or more ranges of options. Thus, for example, a set of options or a range of options may be predefined for one or more parameters associated with an arm or other feature.

The one or more parameters associated with an arm 130 may include, but are not limited to, the overall length of the arm, the shape or configuration of the biasing portion 150, the shape or configuration of the attachment portion 140, the width dimension of one or more sections of the arm 130, the thickness dimension of one or more sections of the arm 130, or the like.

Obtaining the planar appliance digital model 1500 can also include determining the shape and configuration of the anchor 120. For example, the anchor 120 can be selected so as to substantially conform to the patient's gingiva without impinging thereon. The thickness, depth, or other properties of the anchor 120 can also be selected to provide sufficient rigidity against the forces generated by the arms. In some embodiments, the anchor 120 design can be automatically generated (e.g., by being automatically generated to substantially conform to the patient's gingiva or other location in the FTA model (e.g., model 1100) or the OTA model (e.g., model 700 or 1000). In some embodiments, an operator may manually select or revise the design and configuration of the anchor as desired.

Although in the illustrated embodiment, the specific features of the arms 130 are selected while the appliance model is in a substantially planar or 2D form, in other embodiments the appliance features can be selected and configured based on a digital model that is contoured to correspond to a patient's anatomy. For example, the 3D appliance template model 1300 (FIG. 13) can be modified to select particular arms 130, anchor 120, or any aspects thereof to achieve the desired appliance. In some embodiments, the template is omitted altogether, and a customized appliance model is generated based on the OTA model and/or the FTA model without the use of an intervening template model.

In some embodiments, the planar appliance model 1500 can be 2D, such that the model defines no thickness of the appliance. Such a model can be used, for example, to cut an appliance out of a sheet of material. In such cases, the thickness can be determined by selecting the sheet of material and by polishing, etching, grinding, deposition, or other techniques used to modify a final thickness of the appliance. In some embodiments, the planar appliance model 1500 can define a thickness dimension while remaining substantially planar or flat. For example, the planar appliance model 1500 can define a thickness of the appliance which may be uniform or may vary across some or all of the anchor 120 and arms 130.

In some embodiments, a 3D or contoured appliance model can be generated, for example by manipulating the planar appliance model 1500 into a curved or contoured configuration. In some embodiments, the 3D appliance model can correspond to the appliance mounted to the teeth in the OTA (e.g., by manipulating the planar appliance model 1500 using position data of the securing members 900 in the OTA model 1000 (FIG. 10), or by manipulating the planar appliance model 1500 using position data of the securing members 900 in the FTA model 1100 (FIG. 11)).

With reference to blocks 516, 518, and 520 together, in some examples a computer-aided procedure can be used to select or determine the shape and configuration of the arms, anchor, and/or any other features of an appliance. The procedure may be configured to select one (or more than one) arm, securing member, anchor, or parameter thereof, or any other aspect of the appliance based on one or more input data. For example, input data may include, but is not limited to, a type of a tooth (e.g., molar, canine, incisor, etc.) or a size of a tooth. A larger tooth (such as a molar) may require larger arms or larger, wider or thicker loop or curved features for providing a greater force, than for a smaller tooth (such as an incisor). Additionally or alternatively, input data may include the size of the periodontal ligament (PDL) of one or more teeth. The size of the PDL may be obtained by any suitable process including, but not limited to, CBCT scan or other imaging technique. Other input data may include, but is not limited to, the number or direction of forces to be applied to a tooth or teeth in a three-dimensional space. For example, a desired tooth movement direction may require one or more shapes or configurations of arms that differ from the shapes or configurations required for a different tooth movement direction. Other input data may include but is not limited to, the number or direction of rotational forces (or torque) to be applied to a tooth or teeth. For example, a desired tooth movement in a rotational direction may require one or more shapes or configurations of arms that differ from the shapes or configurations required for a different tooth movement direction. Additionally, in some embodiments two or more arms can be attached to a single tooth, either with each arm coupled to a separate securing member, or with two arms coupled to the same securing member. In such instances, the input data can include a number of arms and/or securing members coupled to each tooth, or alternatively the number of arms and/or securing members can be generated as output data.

In some embodiments, this computer-aided procedure can include an algorithm that includes, as input, (but is not limited to) one or more values representing one or more of: (a) up to three translational and up to three rotational movements from an OTA to an ITA or FTA, or from an ITA to another ITA or FTA; (b) the surface of periodontal ligament (PDL) or the area of the root of a or each tooth; (c) bone density of the patient; (d) biological determinants for example, obtained from saliva, gingival fluid (GCF), blood, urine, mucosa, or other sources; (e) gender of the patient; (f) ethnicity of the patient; (g) the jaw (maxilla or mandible) for which the appliance is to be installed; (i) the number of teeth on which the appliance is to be installed; and (j) mechanical properties of the tissue (lips, tongue, gingiva) and bone around the teeth to be moved. In various embodiments, one or more of such inputs can affect the forces (e.g., magnitude, direction, point of contact) required to move each tooth from the OTA to or toward the FTA.

In other examples, other suitable input data may be employed. The computer-aided process employs a computer programed or configured with suitable non-transient software, hardware, firmware, or combinations thereof, to generate an output (such as one or more selected arm configurations, anchor configurations, or securing member configurations), based on the one or more input data.

An output generated by the computer-aided procedure, based on such input, can include, but is not limited to one or more of: (a) a design of an arm; (b) a width or cut-width of one or more of such arms; (c) a thickness dimension of any portion of the appliance of the entire appliance; (d) mechanical properties of such arms including but not limited to amount of flexibility, or a magnitude of bias force or resilience; (e) a design of an anchor; (f) a width or thickness of the anchor; (g) connection locations between the arms and the anchor; and/or (h) transformational temperature of the nitinol (or other material) in one or more (or each) section of the appliance. As noted previously, in some embodiments the output can include particular configurations selected from among a pre-populated library of anchors and/or arms. For example, based on the inputs, a desired force (e.g., magnitude and direction) can be determined for each tooth. Based on the desired force, an appropriate anchor member and/or arm configuration can be selected that provides the desired force or a suitable approximation thereof. In some embodiments, the configuration of the appliance (including any of the outputs listed above) can be generated independently of any pre-populated library. In some embodiments, generating the output can include analyzing provisional selections or designs using finite element analysis (FEA) or other techniques to determine performance parameters, for example, the spring force such arms would apply when deflected by particular amounts (e.g., the amount of deflection between the FTA (when the arm is at rest) and the OTA).

In particular examples, computer-aided processes can be employed to make customized appliances, for each given patient. In other examples, appliances may be made in a plurality of predefined sizes, shapes, configurations, or the like, based on a population group. Accordingly, a different semi-customized size, shape or configuration would be configured to fit each different selected portion of the population group. In that manner, a more limited number of different appliance sizes, shapes and configurations may be made to accommodate a relatively large portion of the population.

Based on the determined shape and configuration of the arms and the anchor, the full appliance shape data can be generated. In some embodiments, the appliance shape data can take the form of 3D data (e.g., the appliance in its shape-set form following heat treatment or other suitable setting technique) or planar or substantially 2D data (e.g., the appliance in its laid-flat form, for example as cut out from a sheet of material).

At block 522, an appliance can be fabricated (e.g., based on the planar appliance digital model 1500 (block 520). And at block 524, a heat treatment fixture can be fabricated (e.g., based on the heat treatment fixture digital model 1200 (block 514). Fabrication of the heat treatment fixture and the appliance are described in more detail below.

In some embodiments, generating the full appliance shape data can include obtaining a heat treatment fixture model (e.g., as described below with respect to FIG. 12), and generating a preliminary appliance model based on the heat treatment fixture model. For example, the preliminary appliance model can conform to at least a portion of a lingual surface of the heat treatment fixture model. The preliminary appliance model can then be modified to include the determined arms and anchor, to have a determined thickness profile, etc. The modified appliance model may then be flattened for use in fabrication as described below.

### Methods of Fabricating Orthodontic Appliances

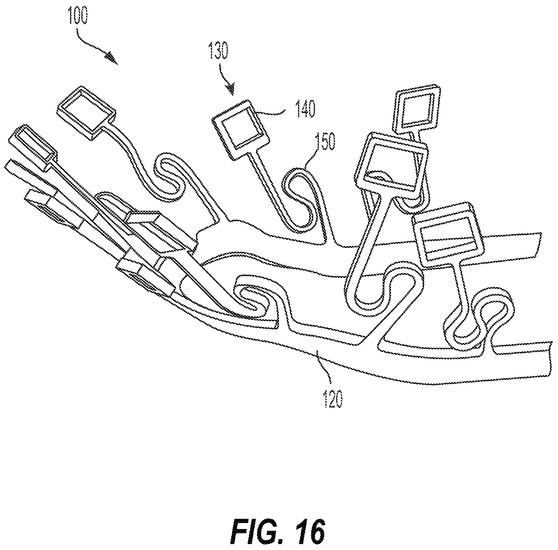

As noted above, one or more digital models can be generated that characterize or define an appliance (e.g., the planar appliance digital model 1500, or a contoured appliance digital model). In various embodiments, one or more such digital models can be used to fabricate an appliance for use in a patient. FIG. 16 illustrates an example of an appliance 100 fabricated using one or more of the digital models described herein. Certain example fabrication processes are described below. However, one of skill in the art will appreciate that any suitable fabrication process may be used to manufacture appliances (or components thereof) as disclosed herein.

In some embodiments, an orthodontic appliance 100 can be fabricated using a planar digital appliance model (e.g., the planar appliance digital model 1500). For example, the planar appliance digital model can include planar or substantially 2D shape data. The planar shape data can be provided to a suitable fabrication device (such as, but not limited to one or more machines that perform cutting, laser cutting, milling, chemical etching, wire electrical discharge machining (EDM), water jetting, punching (stamping), etc.) for cutting a flat sheet of material into a member having a shape corresponding to the planar appliance digital model 1500. The member may be cut from a flat sheet of any suitable material, such as, but not limited to Nitinol, stainless steel, cobalt chrome, or another type of metal, a polymer, a superelastic material, etc. The sheet of material can have a thickness selected to achieve the desired material properties of the resulting member. In various embodiments, the thickness of the sheet of material can be uniform or can vary (e.g., along a gradient, being thinned at particular regions using etching, grinding, etc., or thickened at particular regions using deposition, etc.). In some examples, the sheet can have a thickness of between about 0.1 mm and about 1.0 mm, between about 0.2 mm and about 0.9 mm, between about 0.3 mm and about 0.8 mm, between about 0.4 mm and about 0.7 mm, or about 0.5 mm. In some embodiments, the sheet can have a thickness of less than about 1.5 mm, less than about 1.4 mm, less than about 1.3 mm, less than about 1.2 mm, less than about 1.1 mm, less than about 1.0 mm, less than about 0.9 mm, less than about 0.8 mm, less than about 0.7 mm, less than about 0.6 mm, less than about 0.5 mm, less than about 0.4 mm, less than about 0.3 mm, less than about 0.2 mm, or less than about 0.1 mm.

Next, the cut member can be bent from its substantially planar form into a contoured arrangement. FIG. 16 illustrates an example of a completed appliance 100 resulting from such bending of a planar member. As illustrated, and as described elsewhere herein, the appliance 100 can include an anchor 120 and a plurality of arms 130 extending away from the anchor 120. Each arm 130 can include an attachment portion 40 configured to mate with a securing member adhered to a patient's tooth, and a biasing portion 150 disposed between the attachment portion 40 and the anchor 120. When the appliance 100 is installed in the patient's mouth, each of the arms 130 can connect to a different one of the teeth to be moved and exerts a specific force on its respective tooth, thereby allowing an operator to move each tooth independently.

In some embodiments, the planar member, after being cut from a sheet or otherwise formed, may be bent or otherwise manipulated into a shape or contour corresponding or substantially corresponding to the FTA configuration. For example, the member can be a shape cut from a flat sheet of Nitinol or other suitable material and assume a generally planar configuration. The member can be bent into a desired 3D or contoured configuration, for example corresponding to the contoured appliance digital model 1600. In certain examples, one or more fixtures are configured for use in bending the planar member into the desired 3D shape. In such examples, after cutting the planar member, the planar member can be fixed on or between one or more fixtures and bent or otherwise manipulated to form a desired 3D shape. In some embodiments, either before or after cutting the member from the sheet, the thickness of the member can be modified at least in some portions to achieve desired material properties. For example, the thickness of the member can be reduced in at least some regions using grinding, chemical etching, photoetching, electrical discharge machining, or any other suitable material removal process. The thickness of the member can be increased in at least some regions using thin film deposition, electroplating, or any other suitable additive technique. In some embodiments, the planar member can be formed using 3D printing or other technique instead of or in addition to cutting the planar member from a sheet of material. 3D printing may provide certain advantages, for example ease of controlling the thickness of different portions of the appliance. In some embodiments, the planar member can be formed by 3D printing metal, a polymer, or any other suitable material amendable to additive manufacturing by 3D printing.

In some embodiments, the appliance can be shape set into the desired contoured or 3D configuration (e.g., corresponding to the OTA, the FTA, the heat treatment fixture, etc.). One or more shape setting procedures, such as, but not limited to heat treatment, may be applied to the appliance while held in the desired 3D shape, during or after the bending operation, to set the desired 3D shape. A shape setting procedure involving a heat treatment may include rapid cooling, following heating of the member during or after bending. Additional details regarding example heat treatment and associated fixtures are described below.

By employing a cut planar member, instead of a traditional single-diameter wire, a greater variety of resulting 3D shapes may be made, as compared to shapes made by bending single-diameter wire. The cut planar member may have designed or varying widths and lengths that, when bent into a desired shape, can result in portions of the 3D appliance having variances in thickness, width and length dimensions. In this manner, the planar member can be cut into a shape that provides a desired thickness, width and length of biasing portions, arms, or other components of the appliance. A larger variety of shapes may be provided by bending a custom cut planar member, as compared to bending a single-diameter wire.

In some examples, the entire appliance (including arms and anchor) is fabricated by bending the cut planar member into the desired 3D shaped member. In other examples, additional components may be attached to the 3D shape, for example, after bending. Such additional components may include, but are not limited to attachment portions 40, biasing portions 150, arms 130, etc. Such additional components may be attached to the 3D shaped member by any suitable attachment mechanism including, but not limited to, adhesive material, welding, friction fitting, etc.

In some embodiments, the appliance can be 3D printed directly into the desired contoured or 3D shaped configuration. In some embodiments, the 3D shaped member can be 3D printed, for example using any suitable material. In cases in which the appliance is 3D printed using Nitinol, there may be no need for a shape-setting process (e.g., heat treatment). Additionally, 3D printing may allow the use of different geometries (e.g., a cross-sectional shape of the anchor member may be oval, rather than rectangular, which may increase patient comfort on both the gingival-facing and lingual-facing sides of the anchor).

### Methods of Shape-Setting Orthodontic Appliances

As noted previously, in some embodiments a heat treatment fixture model (e.g., the heat treatment fixture model 1200 (FIG. 12)) can be used to generate an appliance digital model. For example, the planar appliance digital model 1500 can be obtained based at least in part on the heat treatment fixture model 1200. The heat treatment fixture model 1200 may also be used to manufacture a heat treatment fixture, which is then used to shape-set the appliance (e.g., a planar member cut from a sheet of material can be formed into the desired 3D shape by use of the heat treatment fixture).

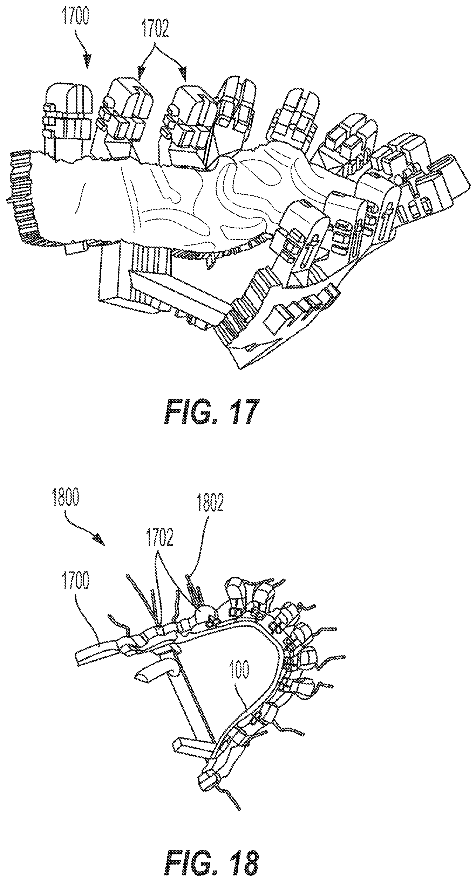

FIG. 17 illustrates an example of a heat treatment fixture 1700. The fixture 1700 can be manufactured based on the heat treatment fixture digital model (e.g., the fixture digital model 1200 (FIG. 12)). For example, the digital model or associated data can be provided to a fabricating system to produce a physical model based on the fixture model. In one example, the fixture data can be used to 3D print a model of the fixture in wax. The wax model may then be used to investment cast the fixture in brass or other suitable material. In some embodiments, the fixture can be 3D printed directly in brass or other suitable material (e.g., stainless steel, bronze, a ceramic or other material that tolerates high temperatures required for heat treatment). As shown in FIG. 17, the fixture 1700 can include securing portions 1702 configured to mate with attachment portions 40 of an appliance 100.

In some embodiments, the fabricated fixture may be used to heat set an appliance. For example, as shown in FIG. 18, a combined assembly 1800 can include an appliance 100 that has been bent or otherwise manipulated into shape against a surface of the heat treatment fixture 1700. The appliance 100 can be coupled to the fixture 1700 by placing attachment portions of the arms into the securing portions 1702 of the fixture. Ligature wires 1802 or other suitable fasteners can be wrapped around the appliance 100 at a plurality of positions to secure the appliance 100 with respect to the fixture 1700. Next, heat can be applied to heat set the appliance 100, after which the appliance 100 can be removed from the fixture 1700.

One example, of a heat treatment procedure can include heating the appliance 100 to a selected temperature (such as, but not limited to 525 degrees centigrade) for a selected period of time (such as, but not limited to 20 minutes), followed by rapid cooling. The rapid cooling can be achieved by any suitable cooling procedure such as, but not limited to water quench or air-cooling. In other examples, the time and temperature for heat treatment can be different than those discussed above, for example, based upon the specific treatment plan. For example, heat treatment temperatures can be within a range from 200 degrees centigrade to 700 degrees centigrade and the time of heat treatment can be a time in the range up to about one hundred and twenty minutes. In particular examples, the heat treatment procedure may be carried out in an air or vacuum furnace, salt bath, fluidized sand bed or other suitable system. After completing the heat treatment, the appliance has a desired 3D shape and configuration (e.g., corresponding substantially to the heat treatment fixture and/or to the desired FTA). In other examples, other suitable heat-treating procedures may be employed including, but not limited to resistive heating or heating by running a current though the metal of the appliance structure.

One or more additional post processing operations may be provided on the 3D shaped article, including, but not limited to abrasive grit blasting, shot peening, polishing, chemical etching, electropolishing, electroplating, coating, ultrasonic cleansing, sterilizing or other cleaning or decontamination procedures.

In examples in which the appliance is made of multiple components, some (or each) of the components of the appliance may be made according to methods described above, and then connected together to form the desired 3D appliance configuration. In these or other examples, the appliance (or some or each component of the appliance) may be made in other suitable methods including, but not limited to: directly printing of metal, first printing of a wax member and then investment casting the wax member into a metal or other material, printing of elastomeric material or other polymer, cutting or machining out of solid material, or cutting the components out of a sheet of metal and shape setting into the desired 3D configuration.

As discussed herein, one or more heat treatment fixtures may be configured for use in bending a cut planar member into a desired 3D shape configuration. In particular examples, one or more heat treatment fixture is provided (such as, but not limited to, custom made) for each jaw of a patient. For example, the heat treatment fixtures may be customized in shape and configuration for each patient and can be made in any suitable manner, including molding, machining, direct metal printing of stainless steel or other suitable metals, 3D printing of a suitable material, such as, but not limited to stainless steel via powder bed fusion, or a steel/copper mix via binder jetting, as well as first printing the configuration in wax and then investment casting the wax into various metals. In various examples described herein, the heat treatment fixtures may be configured of material that is sufficiently resistant to the temperature of the heat treatment. In particular examples, one or more robots may be employed with or without the one or more heat treatment fixtures, for bending the cut planar member into a desired 3D shape configuration.

In some embodiments, a single shape-setting step may be completed to deform the member from its planar configuration to its desired 3D configuration. However, in certain embodiments the shape setting may include two or more shape-setting steps (e.g., two or more heat treatment processes, potentially using two or more different heat treatment fixtures). In such cases, the amount of deformation imparted to the appliance within each shape-setting step may be limited, with each subsequent shape-setting step moving the appliance further toward the desired 3D configuration.

The completed appliances can then be sent (optionally along with bonding trays and/or securing members) to the treating clinician. To install the appliances, the orthodontist can clean the lingual side of the patient's teeth to prepare them for bonding (e.g., with pumice powder). The surface of the teeth can then be sandblasted (e.g., with 50-micron aluminum oxide). The securing members can then be attached using a bonding tray as described elsewhere herein.

After the appliances are fabricated and the securing members are attached to the teeth, each arm can be coupled to its corresponding securing member element to install the appliance. Once installed, the appliance imparts forces and torques on the teeth, to move the teeth to the desired FTA. After treatment is completed (e.g., OTA to FTA, OTA to ITA, ITA to ITA, or ITA to FTA) the arms may sit passively in the securing members and force will no longer be applied to the teeth. Alternatively, any remaining force applied by the arms may fall below a threshold for causing further displacement of the teeth.

The patient can return for a check-up appointment (e.g., at approximately 2-3 months), and if the treatment is advancing as planned, nothing is done until the patient returns at a planned time for appliance removal. At this stage the securing members may be removed. If treatment is not progressing as planned, the appliance may be removed, the patient's mouth rescanned, and a new appliance can be device designed and installed based on a modified treatment plan.

### IV. Use of Finite Element Analysis for Design of Orthodontic Appliances and Treatment Fixtures

As noted previously, in some embodiments, designing and/or fabricating an orthodontic appliance (or components thereof) or a heat treatment fixture (or components thereof) can include using computer-aided or computer-automated analyses. In some embodiments, such computer-aided analysis can include obtaining one or more digital models and performing a finite element analysis (FEA) using one or more such models. For example, digital models can be obtained that characterize or represent the patient's teeth, gingiva, maxilla, mandible, and/or other anatomical structures of the oral cavity (e.g., whether in the OTA, ITA, or FTA), an orthodontic appliance (e.g., in planar form, in 3D pre-installation form, in a deformed configuration, etc.), and/or a heat treatment fixture. As described in more detail below, FEA can be used to evaluate the design and configuration of an orthodontic appliance and/or a heat treatment fixture prior to fabricating the appliance and/or heat treatment fixture. As such, the designs may be corrected, improved, or otherwise modified based on the evaluation before proceeding to fabrication, thereby reducing costly errors and improving device designs.

As noted previously, orthodontic appliances of the present technology may have a planar form corresponding to a flattened or substantially two-dimensional (2D) configuration, a pre-installation form corresponding to a substantially three-dimensional (3D) configuration of the appliance after manufacturing (e.g., the shape of the appliance after heat treatment), and/or an installed form corresponding to a substantially 3D configuration of the appliance at the start of treatment once installed in the patient's mouth (e.g., with the appliance coupled to the patient's teeth in an OTA or ITA). According to some embodiments, the pre-installation form of an appliance can be created by coupling an appliance in planar form to a heat treatment fixture and heat treating the appliance and fixture to form a 3D, contoured shape of the appliance, as previously described. In some embodiments, a pre-installation form of the appliance can be created by any suitable process including, for example, 3D printing an appliance, mechanically deforming an appliance, etc. In some embodiments, a pre-installation form of the appliance can substantially correspond to the patient's teeth in the OTA, the FTA, and/or the heat treatment fixture. For example, the pre-installation form of the appliance can be formed by heat treating the appliance while the appliance is coupled to the heat treatment fixture. In some embodiments, when the appliance is coupled to the heat treatment fixture the anchor substantially conforms to a gingival surface of the heat treatment fixture and the arms substantially conform to securing portions of the heat treatment fixture. As previously noted, these and other embodiments the gingival surface of the heat treatment fixture can be derived from and/or substantially correspond to the patient's gingiva in OTA or in FTA. Each form of the appliance may be virtually represented as a unique digital model. For example, the appliance in the planar form may be virtually represented as a planar appliance digital model.

In some cases, it may be beneficial to evaluate an intended appliance design prior to fabricating a physical appliance based on the intended appliance design to assess how the physical appliance would perform during treatment. For example, because the pre-installation form of the appliance is based at least in part on a desired FTA, the position of one or more portions of the appliance may shift relative to the gingiva once the physical appliance is installed in the patient's mouth (e.g., with the patient's teeth in the OTA or an ITA). As a result, one or more shifted positions of the physical appliance may cause pain for the patient that may reduce treatment compliance and/or satisfaction. The anchor member of the appliance, for example, may be intended to sit adjacent to and slightly spaced apart from the patient's gingiva throughout treatment. In the installed form, the anchor member may sit too far away from the gingiva and irritate the tongue, or the anchor member may sit too close to the gingiva and apply painful pressure to the gingiva. Thus, one or more systems and methods of the present technology may evaluate the position of the appliance relative to the local anatomy once installed in the patient's mouth, such as the position of the anchor relative to the gingiva. Based on the evaluation, one or more parameters of the heat treatment fixture or the appliance can be modified.

Additionally, when the physical appliance is installed in the patient's mouth, the appliance is deformed from the pre-installation form to the installed form and large strain may develop in certain portions of the appliance (e.g., the arms). If strain in the appliance exceeds an elastic limit of the appliance material, plastic deformation may occur and alter the force applied by the appliance. Thus, one or more systems and methods of the present technology may evaluate potential plastic deformation of the appliance and, based on the evaluation, modify one or more parameters of the appliance, such as the geometry of the arms, the geometry of the 3D pre-installation form, and/or the locations of the securing members on the teeth.

Orthodontic appliances of the present invention can be configured apply a force and/or moment to a patient's tooth to move the tooth from an original position (e.g., OTA or ITA) to another position (e.g., ITA or FTA). As previously described, various parameters of an orthodontic appliance design such as arm geometry, anchor geometry, material properties, etc. can be selected and adjusted based on an intended force and/or moment to be applied to a tooth. In some cases, it may be beneficial to evaluate the forces and/or moments an intended appliance design will apply to a patient's teeth before physically manufacturing the appliance to determine whether a physical appliance based on the intended appliance design will perform as intended. Thus, one or more systems and methods of the present technology may evaluate forces and/or moments applied to the patient's teeth and/or an intended appliance design and, based on the evaluation, modify one or more parameters of the appliance and/or heat treatment fixture.

To address the foregoing challenges, prior to fabricating the physical appliance and installing the physical appliance in the patient's mouth, one or more processes may be performed to evaluate an intended appliance design by virtually deforming a digital model of the appliance in one form to produce a digital model of the appliance in another form. For example, a digital model of the appliance in a pre-installation form may be deformed to obtain a digital model of the appliance in an installed form. An output of the virtual deformation can be evaluated to assess whether the physical appliance will function as intended, and based on the evaluation of the output, the intended appliance design can be modified, or a final appliance design can be obtained.

Some or all of the analyses described herein can be performed using suitable computing devices (e.g., computing device 404 described previously). The processes can be performed on one computing device or cluster of computing devices working in concert, or various processes can be performed by remote or distributed computing devices, with different steps being performed by different entities and/or different computing devices. For example, some or all of the analysis processes described herein can be performed in a distributed computing environment in which tasks or modules are performed by remote processing devices, which are linked through a communication network (e.g., a wireless communication network, a wired communication network, a cellular communication network, the Internet, a short-range radio network (e.g., via Bluetooth)). In various embodiments, some or all of the processes described herein can be performed automatically. According to some embodiments, some of the processes described herein may rely at least in part on one or more inputs from a human operator such as a clinician or technician.

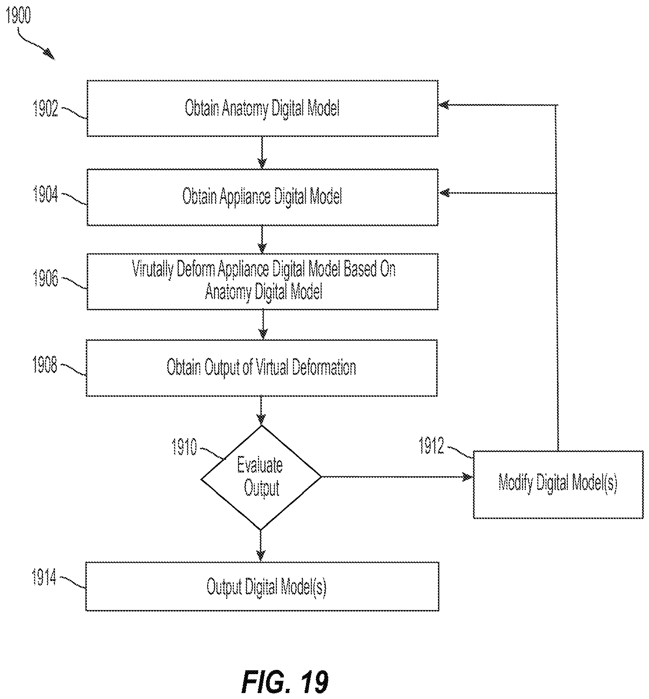

FIG. 19 is a flow diagram of a process 1900 for determining a design of an orthodontic appliance. In some embodiments, the process 1900 may include obtaining an anatomy digital model (process portion 1902) representing or characterizing the geometry of the patient's teeth and/or gingiva in an arrangement. The arrangement may be an original tooth arrangement (OTA), an intermediate tooth arrangement (ITA), or a final tooth arrangement (FTA). In some embodiments the anatomy digital model may be a modified representation of the patient's teeth and/or gingiva. For example, the anatomy digital model may represent a heat treatment fixture based on an OTA and/or a desired FTA, wherein the heat treatment fixture includes modifications to the OTA and/or FTA such as increased thickness of the gingival surface or the addition of structural features, as described previously herein. The process 1900 may include obtaining an appliance digital model (process portion 1904) representing the orthodontic appliance in a specific form. For example, the appliance digital model may be a planar appliance digital model that represents the orthodontic appliance in a substantially flattened or 2D configuration.

The process 1900 may continue at process potion 1906 with virtually deforming the appliance digital model based on the anatomy digital model. The process 1900 may perform the virtual deformation 1906 by finite element analysis (FEA), finite difference methodology, finite volume methodology, or any other suitable numerical methodology. For example, virtually deforming the appliance digital model 1906 may include performing an FEA with the appliance digital model and the anatomy digital model to deform the appliance digital model based on a difference in position between a portion of the appliance digital model and a portion of the anatomy digital model. The process 1900 may obtain an output of the virtual deformation at process portion 1908 and may evaluate the output in process portion 1910. The output may comprise the virtually deformed appliance digital model, the anatomy digital model, and/or data produced by the virtual deformation such as a displacement, force, strain, stress, or relative position. Evaluating the output may comprise performing a quantitative comparison of the output to a predetermined threshold or parameter. In some embodiments, evaluating the output may comprise performing a qualitative evaluation of the output. For example, a human operator can visually inspect the output. Based on the evaluation of the output 1910, the process 1900 may continue with modifying one or more of the previously obtained digital models (process portion 1912). For example, the process 1900 may determine that a strain in the appliance digital model exceeds a predetermined threshold and modify the geometry of one or more portions of the appliance digital model to reduce the strain. Based on the evaluation of the output 1910, the process 1900 may continue to process portion 1914 and output one or more previously obtained digital models. The digital model(s) output by process portion 1914 may be used to fabricate a physical appliance and/or fixture, as previously described.

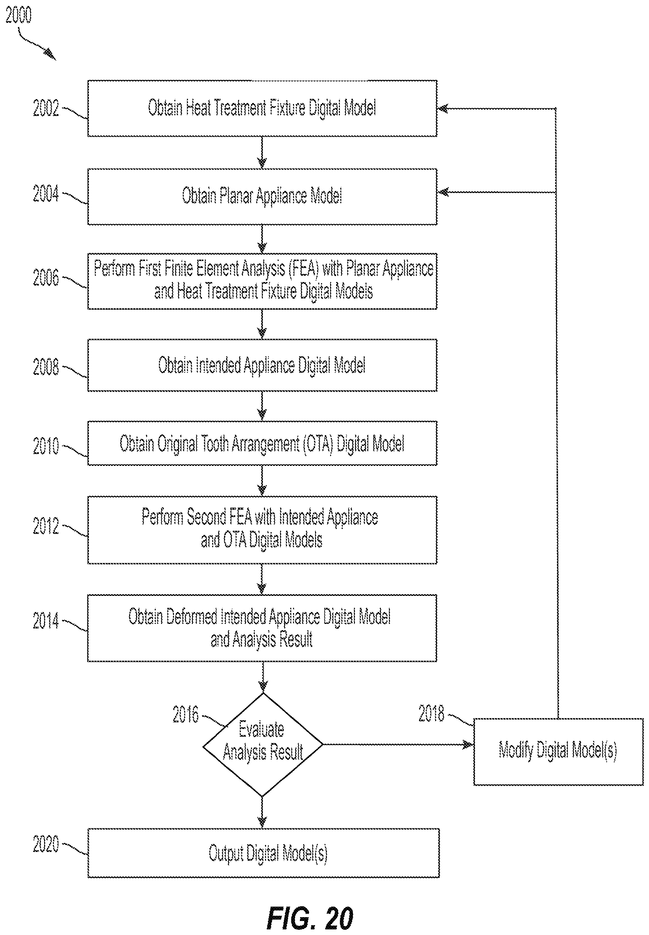

FIG. 20 illustrates an example process 2000 for evaluating an orthodontic appliance design. In some embodiments, each of the process portions of the process 2000 can be executed automatically or manually, by human operator, for example. The process 2000 may begin at process portion 2002 with obtaining a heat treatment fixture digital model 1200. An example of a heat treatment fixture model 1200 is shown in FIG. 12, described above. As previously described, the heat treatment fixture digital model 1200 may correspond to and/or be derived from an OTA and/or a desired FTA with certain modifications (e.g., enlarging the gingiva). At process portion 2004, a planar appliance digital model 1500 may be obtained. An example of a planar appliance digital model 1500 is shown in FIG. 15, described above. As previously described, the planar appliance digital model 1500 may have a substantially flattened or 2D configuration and may virtually represent the appliance design comprising an anchor and/or a plurality of arms. In some embodiments, the planar appliance digital model 1500 can include a thickness dimension, which can be uniform over the appliance or may vary over different portions of the appliance. The process 2000 may continue at process portion 2006 with performing a first FEA with the planar appliance digital model 1500 based on the heat treatment fixture digital model 1200. For example, the first FEA can generate an intended appliance digital model, in which the planar appliance digital model 1500 has been deformed based on a feature (e.g., securing portions 1202, gingival surface 1210) of the heat treatment fixture model 1200, resulting in a contoured or 3D appliance, with a shape and configuration similar to the completed appliance 10 (e.g., as shown in FIG. 16). In some embodiments, the process 2000 may perform the first FEA using suitable commercial FEA software (e.g. Abaqus, Ansys) and/or suitable proprietary FEA software.

Although some embodiments describe using the heat treatment fixture digital model 1200 to generate a 3D or contoured configuration of the appliance digital model, in some embodiments an FTA digital model (e.g., FTA models 800 or 1100 described above) can be used. For example, a planar appliance digital model 1500 can be deformed to conform to a surface of an FTA digital model, without the need for the heat treatment fixture digital model 1200.

In some embodiments, performing the first FEA in process portion 2006 may include meshing one or more of the digital models, wherein meshing comprises discretizing a digital model into a plurality of finite elements and a plurality of nodes. Meshing may be performed manually, such as by human operator, and/or automatically using suitable software. Suitable software may include commercial meshing software (e.g. Hypermesh^{®}), commercial FEA software with meshing capabilities (e.g. Abaqus), and/or proprietary meshing software. The finite elements may have a dimensionality based on geometry of the digital model, including, but not limited to, 2D (e.g., triangular, quadrilateral) or 3D (e.g., tetrahedral, quadrilateral) elements. For example, the finite elements for the planar appliance digital model 1500 may comprise hexahedral 3D elements. Element parameters (e.g., element type, element order, number of integration points, hourglass control) may be selected to control the accuracy and stability of the FEA. In some embodiments, performing the FEA may include meshfree techniques such as element-free Galerkin process, generalized-strain mesh-free formulation, isogeometric analysis, or the process of external approximations.

Performing the first FEA in process portion 2006 may include assigning material properties (e.g., Young's modulus, Poisson's ratio, density) to the planar appliance digital model 1500 and the heat treatment fixture digital model 1200. For example, material properties for nitinol may be assigned to the planar appliance digital model 1500, such as a Young's modulus between about 28 GPa and 83 GPa. In some embodiments, the heat treatment fixture model 1200 may be represented as a deformable component with material properties for brass, such as a Young's modulus between about 100 GPa and 130 GPa. In some embodiments, the heat treatment fixture digital model 1200 may be represented as a rigid component such that the heat treatment fixture digital model 1200 does not deform during the first FEA. The heat treatment fixture digital model 1200 may be represented as a rigid component by assigning an artificially large Young's modulus to the heat treatment fixture digital model 1200. The process 2000 may obtain the material properties from a database and/or the material properties may be entered manually.

In some embodiments, performing the FEA (process portion 2006) may include defining a contact interaction between at least one portion of the appliance digital model 1500 and at least one portion of the heat treatment fixture digital model 1200. Defining the contact interaction may include creating a first contact surface by selecting digital nodes, elements, and/or surfaces of the planar appliance digital model 1500. Another contact surface may be created by selecting digital nodes, elements, and/or surfaces of the heat treatment fixture digital model 1200. Defining the contact interaction may further comprise defining a contact formulation to govern the contact interaction between the contact surfaces. The type of contact formulation (e.g., bonded, frictional, frictionless, etc.) may be selected from a database of contact formulations and/or or the contact formulation may be entered manually. The process may further comprise entering relevant parameters of the contact formulation including, but not limited to, a coefficient of friction, a penalty contact stiffness, and/or a nodal search distance. For example, in some embodiments a bonded contact interaction can be defined between an attachment portion 140 of the appliance digital model 1500 and a horizontal channel 1204, a vertical channel 1206, and/or a securing portion 1202 of the heat treatment fixture digital model 1200. In some embodiments, a sliding contact interaction can be defined between an attachment portion 140 of the appliance digital model 1500 and a securing portion 1202 of the heat treatment fixture digital model 1200. For example, a sliding contact interaction can be defined to simulate interplay between an attachment portion and a securing member.

Performing the FEA in process portion 2006 may include assigning boundary conditions to at least one of the digital models. In some embodiments, the boundary conditions may include a constraint to prevent translation and/or rotation of one or more portions of one or more digital models. For example, the boundary conditions may include a constraint of the heat treatment fixture digital model 1200 and one or more attachment portions 140 of the planar appliance digital model 1500. In addition or alternatively, the boundary conditions may include a non-zero force, moment, displacement, and/or rotation. For example, to virtually deform the planar appliance digital model 1500 into a contoured or 3D digital model representing the pre-installation form of the appliance, a non-zero displacement may be applied to a portion of the anchor member 20 of the planar appliance digital model 1500. The non-zero displacement can correspond to a distance between the anchor member 20 and the distal-gingival surface 1210 of the heat treatment fixture digital model 1200 when the attachment portions 140 of the planar appliance digital model 1500 are within the securing portions 1202 of the heat treatment fixture digital model 1200. In some embodiments, the planar appliance digital model 1500 can be virtually deformed into a contoured or 3D digital model representing the pre-installation form of the appliance by applying a non-zero displacement to an attachment portion 140 of the planar appliance digital model 1500 such that the attachment portion 140 is positioned within a corresponding securing portion 1202 of the heat treatment fixture digital model 1200. In addition, or alternatively, a non-zero displacement can be applied to an attachment portion 140 and/or arm 130 of the planar appliance digital model 1500 such that the attachment portion 140 and/or arm 130 is tangent to a base plane of a corresponding securing portion 1202 of the heat treatment fixture digital model 1200.

In some embodiments, performing the FEA in process portion 2006 may include defining one or more analysis parameters such as analysis type (e.g., static or dynamic), geometric linearity, integration scheme (e.g., implicit, explicit), simulation duration, incrementation size, and/or incrementation control. Performing the FEA may include running the FEA until an exit condition is reached. For example, running the FEA may include applying a non-zero displacement to the planar appliance digital model 1500, wherein the exit condition is reached once the entire magnitude of the non-zero displacement has been applied.

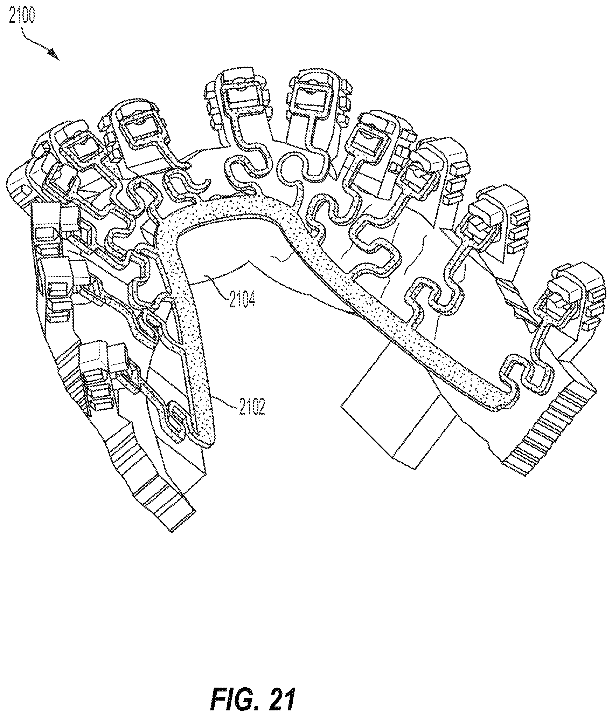

Referring back to FIG. 20, the process 2000 may continue at process portion 2008 with obtaining an intended appliance digital model 2102 virtually representing the appliance in a pre-installation form. As depicted in FIG. 21, the digital model 2100 resulting from the first FEA (process portion 2006) can include an intended appliance digital model 2102 representing a pre-installation form of the appliance in a contoured configuration and mated with a heat treatment fixture model 2104. The intended appliance digital model 2102 obtained in process portion 2008 can be obtained from the first FEA performed in process portion 2006 of the process 2000. In some embodiments, an intended appliance digital model 2102 representing a contoured or 3D configuration of the appliance after manufacturing can be obtained without performing a first FEA. For example, an intended appliance digital model 2102 can be obtained directly from CAD software such as Solidworks^{®}, Autodesk^{®} Inventor, Autodesk^{®} MeshMixer, Creo^{®}, etc. In addition, or alternatively, an intended appliance digital model 2102 can be obtained from a scan of a physical representation of an appliance such as a fabricated appliance, an appliance mold, etc. At process portion 2010 an OTA digital model may be obtained that virtually represents the patient's teeth in an original arrangement. For example, the OTA digital model 1000 with securing members attached thereto can be used, as described above with respect to FIG. 10. In some embodiments, the OTA digital model can comprise a modified representation of the patient's teeth and/or gingiva. For example, the OTA digital model can have similar features as the heat treatment fixture 1200 (e.g., securing portions 1202 with horizontal channels 1204 and/or vertical channels 1206, stabilizing crossbar 1212) in place of or in addition to a virtual representation of the patient's actual teeth and/or gingiva. For example, in some embodiments, securing portions in the positions of a patient's teeth in an original arrangement can replace a virtual representation of the patient's actual teeth in an OTA digital model. According to some embodiments, the OTA digital model can comprise a dataset comprising position data characterizing the spatial coordinates of a patient's teeth in an original arrangement.

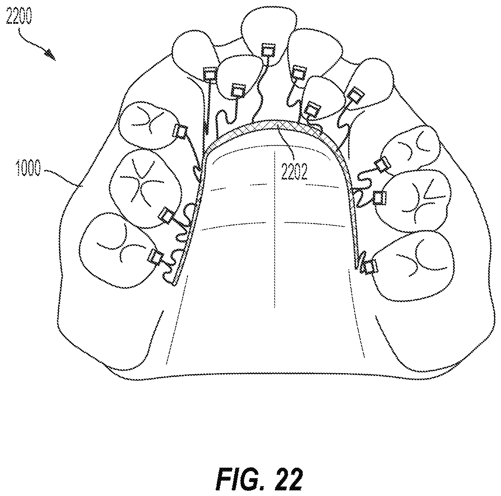

Referring back to FIG. 20, the process 2000 may continue with performing a second FEA with the intended appliance digital model 2102 and the OTA digital model (process portion 2012) to produce a deformed intended appliance digital model representing the appliance in an installed form. FIG. 22 illustrates an example of a digital model 2200 that includes a deformed intended appliance digital model 2202 that is mated with an OTA digital model 1000. As shown in FIG. 22, the deformed intended appliance digital model 2202 can virtually represent the appliance in an installed form in the patient's mouth (e.g., in the OTA or ITA). For example, via the second FEA, the intended appliance digital model 2102 (e.g., characterizing the appliance in a pre-installation form) can be virtually deformed into an installed form in which the appliance is mated to a patient's teeth in the OTA (or ITA), as reflected in the OTA digital model 1000. This virtual deformation can produce the deformed intended appliance digital model 2202, which can effectively model the real-world behavior of a fabricated appliance when installed within a patient's mouth. As such, evaluation of the deformed intended appliance digital model 2202 allows a human operator and/or an automated process to assess and/or predict operation and behavior of the appliance when installed within the patient's mouth.

In some embodiments, performing the second FEA can include discretizing the digital model(s), assigning material properties, defining any contact interactions, assigning boundary conditions, defining any analysis parameters, and/or running the FEA until an exit condition is reached as previously described. For example, assigning boundary conditions to perform the second FEA may include determining a displacement of each tooth between the FTA and OTA. As previously described, the displacement of a tooth can be defined using six degrees of freedom by calculating the difference between the location of each tooth in the FTA data and the OTA data. Assigning the boundary conditions can include assigning the displacement of each tooth between the FTA and OTA to a corresponding attachment portion of the intended appliance digital model 2102. Assigning the boundary conditions may comprise assigning constraints to prevent rotation and/or translation of the OTA digital model 1000 and/or one or more portions of the intended appliance digital model 2102.

Referring back to FIG. 20, at process portion 2014 the process 2000 may obtain the deformed intended appliance digital model 2202 (FIG. 22), and/or an analysis result. The deformed intended appliance digital model 2202 and/or the analysis result can be obtained from the second FEA. The deformed intended appliance digital model 2202 may virtually represent the appliance in an installed form once it has been installed into the patient's mouth (e.g., with the appliance coupled to the patient's teeth in an OTA or ITA). The analysis result can comprise output data from the second FEA. For example, the analysis result may be a measure of position, displacement, rotation, force, moment, stress, or strain in one or more of the digital models used in the second FEA. The process 2000 may continue at process portion 2016 with evaluating the analysis result. In some embodiments, evaluating the analysis result includes comparing the analysis result to one or more predetermined thresholds. Based on the evaluation of the analysis result, the process 2000 may continue to process portion 2018 and modify the planar appliance digital model 1500 and/or the heat treatment fixture digital model 1200.

In various embodiments, modifying the appliance digital model (e.g., the planar appliance digital model 1500, or a 3D appliance digital model) can include modifying the particular shape and/or configuration of an anchor and/or arms of the appliance, the geometry of the 3D pre-installation form of the appliance, and/or the locations of the securing members on the teeth. For example, features of the arm(s) that can be modified include but are not limited to, the overall length of the arm, the shape or configuration of the biasing portion, the shape or configuration of the bracket connector, the width dimension of one or more sections of the arm, the thickness dimension of one or more sections of the arm 130, or the like. Features of the anchor that can be modified include, but are not limited to the shape, length, thickness, depth, or other properties of the anchor. In some embodiments, a human operator may manually select or revise the design and configuration of the anchor and/or arms as desired. In some embodiments, one or more of the arms can be replaced based on a pre-populated library of arm designs. In some embodiments, fully or partially automated modification of the appliance digital model or the heat treatment fixture digital model can be reviewed and/or modified by an operator based on relevant criteria.

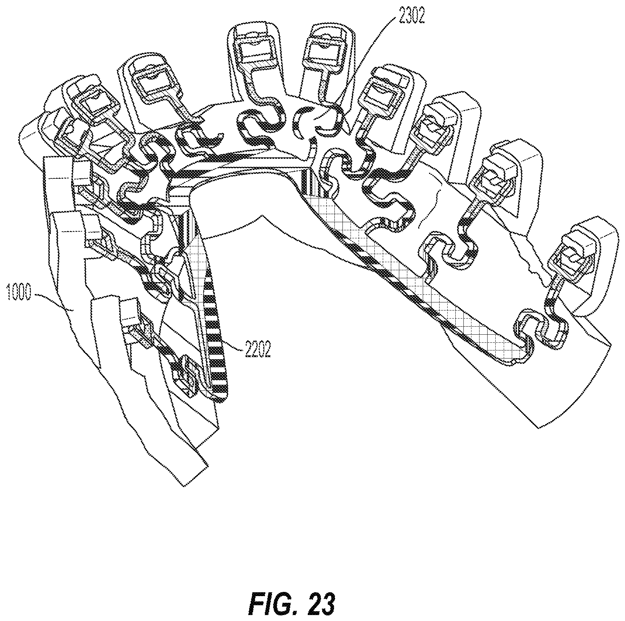

FIG. 23 illustrates an example of an analysis result of a deformed intended appliance digital model 2202 in a configuration mated to a patient's teeth, as reflected in the OTA digital model 1000. The analysis result can include a measure of strain in the appliance digital model 2202. The measure of strain may comprise, for example, a single maximum strain in the appliance, a volume of elements exceeding a strain threshold, and/or an average strain of a portion of the appliance. As depicted in FIG. 23, the measure of strain may be displayed by the process 2000 as a heat map superimposed over the appliance digital model 2202. Such a heat map (or other graphical representation) can visually indicate the strain at different regions of the appliance digital model 2202. In some embodiments, the measure of strain may be a number and/or a set of numbers. The process 2000 may compare the measure of strain to a predetermined maximum strain threshold in process portion 2016. In some embodiments, the predetermined maximum strain may be an elastic limit of the appliance material. For example, the predetermined maximum strain for nitinol may between about 4% to about 10%. If the measure of strain exceeds the predetermined maximum strain threshold, the process 2000 may proceed to process portion 2018 and modify the planar appliance digital model 1500. Modifying the planar appliance digital model may include, for example, increasing the thickness of one or more portions of the appliance, selecting a different geometry of an arm or anchor portion of the appliance, etc.

In some embodiments, the analysis result may comprise a force and/or moment in order to evaluate a force and/or moment the appliance applies to a patient's tooth. For example, the analysis result can be a reaction force and/or moment measured at a portion of the anchor of the appliance digital model 2202, a securing member of an OTA digital model 1000, a tooth of the OTA digital model, or any other suitable location. The location the force and/or moment is measured from can be based, at least in part, on the boundary conditions assigned in the second FEA. In some embodiments, evaluating the analysis result (process portion 2016) comprises comparing the force and/or moment to a predetermined value. In some embodiments, the predetermined value may correspond to an intended force and/or moment. A difference between the measured and intended force and/or moment can be obtained and evaluated to determine if a physical appliance based on the intended appliance design will sufficiently apply the intended force and/or moment and perform as intended. In some embodiments, the predetermined value can be a safety threshold corresponding to a maximum allowable force for the appliance and/or the patient's anatomy. According to some embodiments, the predetermined value is a minimum force and/or moment, a range of allowable forces and/or moments, or any other suitable metric. Based on the comparison of the force and/or moment to the predetermined value, the process 2000 may modify one or more parameters of the planar appliance digital model 1500, the heat treatment fixture digital model 1200, the intended appliance digital model 2100, or another suitable digital model.

Another example of an analysis result includes identifying portions of the appliance that may impinge on a patient's gingiva. For example, as shown in FIG. 23, in region 2302, a portion of the appliance digital model 2202 has penetrated beneath a gingival surface of the OTA digital model 1000. This may occur as a result of deformation of the model from the intended appliance digital model 2102 to the deformed appliance digital model 2202 described previously. The intersection shown in region 2302 can indicate an area at which a real-world fabricated appliance is at risk of contacting the patient's gingiva when installed. Such contact can be uncomfortable and irritate the patient's gingiva. Accordingly, as a result of identifying such a contact point, the appliance design may be modified (e.g., by modifying the planar appliance digital model 1500), the pre-installation form of the appliance may be modified (e.g., by modifying the heat treatment fixture model 1200), or any other suitable modifications, corrections, or compensations may be made.

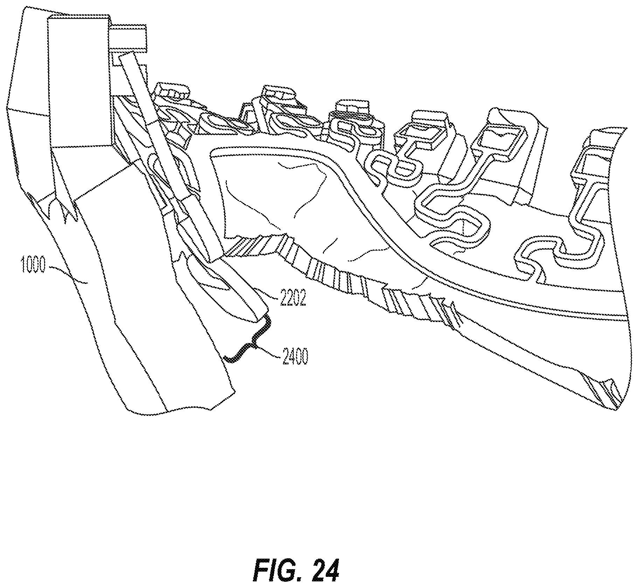

FIG. 24 illustrates another example of an analysis result based on assessment of the relative positions of the deformed appliance digital model 2202 and the OTA digital model 1000. For example, as shown in FIG. 24, a portion of the appliance digital model 2202 is spaced apart from a gingival surface of the OTA digital model 1000 by a local distance 2400 due to a shape set form of the appliance. Too large of a gap between the appliance the patient's gingiva can irritate the patient's tongue and cause pain and/or discomfort for the patient. Therefore, in some embodiments, the analysis can include determining whether a local distance 2400 is greater than a predetermined maximum distance threshold. In the example shown in FIG. 24, the analysis result can comprise a local distance 2400 between a portion of the deformed intended appliance digital model 2202 and a portion of the lingual surface of the patient's gingiva of the OTA digital model 1000 that exceeds a predetermined maximum distance threshold. In some examples, the maximum distance threshold for a local distance between a portion of the deformed intended appliance digital model and a portion of the lingual surface of the patient's gingiva may be between about 0 mm and about 5 mm. If the local distance 2400 is greater than the maximum distance threshold, the process 2000 may modify one or more digital models (process portion 2018) to thereby modify the relative positions of the appliance in the installed form and the patient's gingiva. For example, the thickness of the gingival surface of the heat treatment fixture digital model 1200 can be increased and/or decreased at one or more locations. The process 2000 may repeat with the modified digital model(s) to determine if the local distance 2400 falls below the maximum distance threshold and whether the modified digital model(s) are more favorable design(s).

It may be favorable to space an anchor 20 of an appliance apart from a patient's gingiva to minimize irritation of the patient's gingiva due to the appliance. Consequently, in some embodiments, the analysis result can comprise a local distance 2400 between a portion of the deformed intended appliance digital model 2202 and a portion of the lingual surface of the patient's gingiva of the OTA digital model 1000 that is less than a predetermined minimum distance threshold. In some examples, the minimum threshold may be between about 0.00 mm and 0.5 mm. If the local distance 2400 is less than the minimum distance threshold, the process 2000 may modify one or more digital model(s) (process portion 2018). For example, the thickness of the gingival surface of the heat treatment fixture digital model 1200 may be increased and/or decreased at one or more locations. Such a modification may alter the pre-installation form of the appliance. The process 2000 may repeat with the modified digital model(s) to determine if the local distance falls above the minimum threshold.

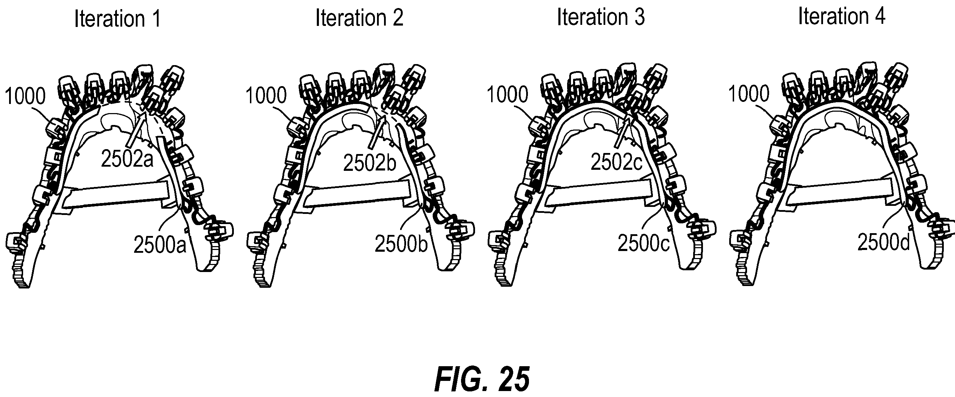

According to some embodiments, the process 2000 can iteratively repeat until a favorable appliance design is obtained. For example, FIG. 25 depicts four deformed intended appliance digital models 2500a, 2500b, 2500c, and 2500d mated to an OTA digital model 1000 representing the patient's teeth in an original arrangement. A first appliance digital model 2500a has penetrated a gingival surface of the OTA digital model 1000 in a first intersecting region 2502a as a result of the second FEA. The process 2000 can modify one or more digital model(s) based on this analysis result (process portion 2018) and repeat process portions 2002 through 2016 with the modified digital model(s) until a finalized appliance design is obtained. For example, FIG. 25 shows a second appliance digital model 2500b that penetrates a gingival surface of the OTA digital model 1000 to a lesser extent than the first appliance digital model 2500a, forming a second intersection region 2502b that is smaller than the first intersection region 2502a. A third appliance digital model 2022c forms a third intersection region 2502c that is smaller than the first and second intersection regions 2502a, 2502b. A fourth appliance digital model 2502d depicted in FIG. 25 does not penetrate a gingival surface of the OTA digital model 1000 and may be a favorable appliance design. Based on the favorable fourth appliance digital model 2502d, the process 2000 can stop iteratively repeating and select a finalized appliance design. In some embodiments, a human operator can select a finalized appliance design. In some embodiments, a finalized appliance design can be selected automatically and/or by a human operator based on a quantitative metric such as, but not limited to, a change in an analysis result between iterations, a comparison of an analysis result to a predetermined threshold or parameter, etc. In addition, or alternatively, the process 2000 may stop repeating and select a finalized appliance design if a predetermined maximum number of iterations has been reached.

In some embodiments, the heat treatment fixture digital model 1200 can be modified based on a final appliance design. For example, the gingival surface 1210 of the heat treatment fixture 1200 can be modified such that the lingual-gingival surface 1210 of the heat treatment fixture 1200 is tangent to the gingival-facing surface of the appliance when attachment portions of the appliance are positioned within and tangent to a base plane of securing portions 1202 of the heat treatment fixture 1200.

Upon selection of a final appliance design and/or a final heat treatment fixture design, the process 2000 can continue to process portion 2020 and output the planar appliance digital model 1500, the heat treatment fixture digital model 1200, and/or the intended appliance digital model 2102. Based on the output in process portion 2020, the appliance and/or the heat treatment fixture can be fabricated, for example using any of the techniques described previously herein.

### IV. Selected Devices, Systems, and Methods for Manufacturing Orthodontic Appliances based on Overcorrection and/or Compensation Parameters

As previously described, the manufacturing process to create an orthodontic device (e.g., an orthodontic appliance or fixture) according to embodiments of the present technology can include obtaining data corresponding to an OTA of a patient, and then using the data to develop an FTA model in which the patient's teeth are in an optimal position. The FTA model can be used as a basis for creating a fixture (e.g., a heat treatment fixture) that generally corresponds to the FTA, but with one or more modifications (as discussed elsewhere herein). The fixture can then be used to form a 3D configuration of the appliance (e.g., a curved or contoured configuration of the appliance able to urge teeth from the OTA toward the FTA when installed in a patient's mouth). For example, as described elsewhere herein, a substantially planar configuration of the appliance may be manipulated and/or disposed over the fixture and then heat treated on the fixture such that the appliance assumes a 3D shape that generally conforms to the fixture.

Manufacturing the appliance in such a manner should enable the appliance to precisely replicate the FTA described above and, when installed, reposition a patient's teeth from the OTA to the desired FTA. However, in practice, certain factors may cause there to be a discrepancy between the desired FTA and the actual final arrangement of the patient's teeth after repositioning via the appliance. As described in more detail below, this discrepancy can be due to: (a) implementation considerations (e.g., a minimum threshold force needed to move the patient's teeth, and/or free play or tolerance between the appliance and securing member), (b) material properties of the appliance (e.g., plastic deformation, hysteresis, etc.), (c) irregularities associated with the manufacturing process, and/or (d) expected teeth movement (e.g., relapse) after repositioning. To mitigate these issues, embodiments of the present technology can account for these discrepancies and modify design parameters (e.g., via overcorrection or compensation) of the fixture and/or appliance during or prior to manufacturing thereof.

A person of ordinary skill in the art will recognize that while embodiments of the present technology related to overcorrection or compensation are described below as individual parameters or factors, any of the factors described may be combined in a single embodiment. For example, design or manufacturing of an appliance and/or fixture may consider both the minimum threshold force needed to move the patient's teeth as well as irregularities associated with the manufacturing process.

### A. Considerations Related to Orthodontic Device Implementation

As previously described, orthodontic appliances of the present technology are generally designed and manufactured based at least in part on the forces (e.g., load/moment/magnitude and/or direction) needed to reposition a patient's teeth (e.g., individual teeth) from the OTA to a desired or optimal FTA. In some embodiments, these appliances may consider external factors acting thereon (e.g., the minimum threshold force needed to move a patient's teeth and/or the free play between the appliance and securing members), which in turn affect the necessary force(s) that the appliance and/or one or more portions thereof must provide on the patient's teeth to cause the desired repositioning to the FTA.

### 1. Minimum Threshold Force to Move a Patient's Teeth

As previously described, appliances of the present technology are configured to move a patient's teeth from the OTA along a path to a determined FTA. More specifically, individual arms of the appliance are configured to move a respective patient's tooth along a respective path from an original position to a respective final position. The force applied to a patient's teeth via the appliance, or in some embodiments the force applied to a patient's tooth via a corresponding arm of the appliance, is generally highest at or near the OTA, when the appliance is in a loaded or stressed state, and decreases as the patient's teeth approach the FTA, when the appliance is in an unloaded or unstressed state. Accordingly, when the patient's teeth approach the FTA, the appliance will generally be applying some minimal force to the teeth. However, due to various external factors, such as the root of a particular tooth or positioning of a tooth within the gingiva, there can be a minimum threshold force that must be overcome to move each tooth. That is, a force applied via an arm of the appliance on the tooth that is less than the minimum threshold force will not move the tooth. Therefore, if an appliance in its unloaded state is manufactured to resemble or otherwise correspond to the FTA without considering this minimum threshold, movement of the patient's teeth may cease prior to actually reaching the FTA.

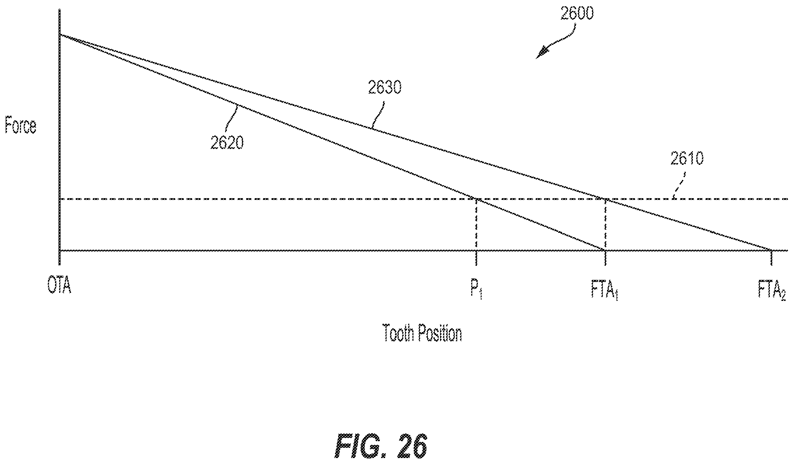

To further illustrate this point, FIG. 26 is a plot 2600 showing the relationship between force applied to a patient's teeth on the y-axis, and positioning of the patient's teeth on the x-axis. As shown in FIG. 26, line 2610 corresponds to a minimum threshold force needed to move a patient's teeth, line 2620 corresponds to varying forces applied, e.g., via a first appliance, to the patient's teeth during movement from the OTA to a first final tooth arrangement (FTA₁), and line 2630 corresponds to varying forces applied, e.g., via a second appliance, to the patient's teeth during movement from the OTA to a second final tooth arrangement (FTA₂). In some embodiments, the minimum threshold force may be at least about 5 grams-force (GF), 10 GF, 15 GF, 20 GF, 25 GF, or 50 GF. The first appliance has an unloaded or unstressed state that corresponds to the first final tooth arrangement (FTA₁), which is an optimal tooth arrangement determined for the patient, and the second appliance has an unloaded state that corresponds to the second final tooth arrangement (FTA₂) different than the first final tooth arrangement (FTA₁).

As shown in FIG. 26, lines 2620, 2630 indicate a generally linear relationship between force applied to the patient's teeth and positioning thereof. However, a person of ordinary skill in the art will appreciate that in some embodiments the relationship between the applied force and positioning of the patient's teeth may be non-linear (e.g., exponential, logarithmic, etc.). Additionally or alternatively, in some embodiments the relationship between force applied to the patient's teeth and positioning thereof can be linear, non-linear, and/or constant depending on the strain of the appliance of portions thereof (e.g., the arm(s) of the appliance). For example, with regard to an appliance or arm comprising nitinol, the force applied to the patient's teeth via the nitinol appliance may be constant or nearly constant during a first portion of teeth movement and have a linear of non-linear relationship to the position of the patient's teeth during a second, different portion of teeth movement.

As indicated by line 2620 of FIG. 26, the first appliance (manufactured to have an unloaded configuration corresponding to the first final tooth arrangement (FTA₁)) will cause the patient's teeth to reposition from the OTA along a path toward the first final tooth arrangement (FTA₁). Such an appliance, when implanted within a patient's mouth and secured to securing members adhered to the patient's teeth (as previously described), will transition from a loaded configuration generally corresponding to the OTA toward an unloaded configuration generally corresponding to the first final tooth arrangement (FTA₁). However, due to the minimum threshold force (T_{MIN}) needed to move the patient's teeth (as shown by line 2610), the first appliance will be unable to move the patient's teeth all the way to the first final tooth arrangement (FTA₁), and instead will move the patient's teeth only until the force provided via the appliance is equal to the minimum threshold force (T_{MIN}), as represented by position (P₁) in FIG. 26.

Embodiments of the present technology can mitigate the above described issues by considering the minimum threshold force (T_{MIN}) when designing the orthodontic appliance. In some embodiments, an appliance may be designed and/or manufactured to have a second final tooth arrangement (FTA₂) in its unloaded configuration that is different that the first final tooth arrangement (FTA₁). When implanted within a patient's mouth and secured to securing members adhered to the patient's teeth, the second appliance is configured to reposition the patient's teeth from the OTA toward and/or to the first final tooth arrangement (FTA₁). In such embodiments, the second appliance is designed to provide the minimum threshold force (T_{MIN}) on the patient's teeth when the second appliance, which has an unloaded configuration corresponding to the second final tooth arrangement (FTA₂), assumes a configuration generally corresponding to the first final tooth arrangement (FTA₁). As shown in FIG. 26, the second appliance can be manufactured to have an unloaded configuration generally corresponding to the second final tooth arrangement (FTA₂). When implanted within a patient's mouth and secured to corresponding securing members, the second appliance will cause the patient's teeth to reposition from the OTA along a path toward the second final tooth arrangement (FTA₂), as indicated by line 2630. Due to the minimum threshold force (T_{MIN}), movement of the patient's teeth via the second appliance ceases when the second appliance generally assumes the first final tooth arrangement (FTA₁) and is providing a force on the patient's teeth approximately equal to the minimum threshold force (T_{MIN}). As shown in FIG. 26, such an appliance is configured to apply a nonzero force on the patient's teeth when they become repositioned to the first tooth arrangement (FTA₁). The nonzero force may be (i) at least about 5 GF, 10 GF, 15 GF, 20 GF, 25 GF, or 50 GF, and/or (ii) no more than 500 GF, 400 GF, 300 GF, 250 GF, 100 GF, or 50 GF.

The above description regarding the minimum threshold force applies to the appliance and patient's teeth generally, but the same or similar principles also apply to individual arms of the appliance and individual teeth of the patient. For example, each arm of the appliance may be configured to move a corresponding patient tooth such that the force provided via the arm is equal to the minimum threshold force (T_{MIN}) when the position of the arm generally corresponds to that of a corresponding arm in the first final tooth arrangement (FTA₁). Moreover, the minimum threshold needed to move a particular tooth may be slightly different from other teeth, e.g., depending on the type of tooth (e.g., molar or incisor), the position of the tooth (e.g., relative to the adjacent gingival surface), and/or other factors. As such, the distinct minimum threshold force for individual teeth may each be accounted for when designing the corresponding portions (e.g., arms, biasing portions, attachment portions, etc.) of the appliance.

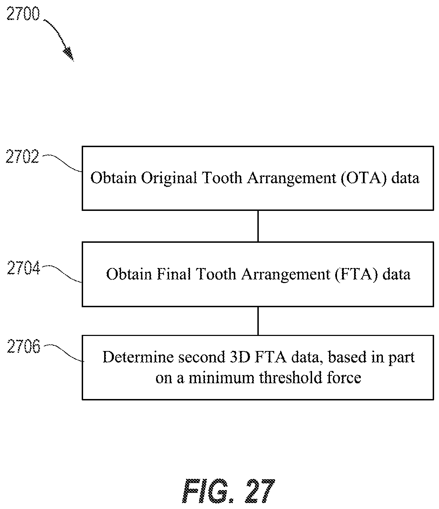

FIG. 27 is a flow diagram of a method 2700 for determining a dataset associated with an arrangement of an orthodontic device, in accordance with embodiments of the present technology. The method 2700 includes obtaining data (e.g., a first input) corresponding to an OTA of a patient (process portion 2702), and obtaining data (e.g., a second input) corresponding to a first FTA of the patient (process portion 2704). As described elsewhere herein, the OTA can be based on a scan of the patient's teeth, and the FTA can be determined and/or provided by an operator (e.g., a clinician, orthodontist, or technician) based on the OTA and a desired optimal positioning of the teeth.

The method 2700 can further include determining data (e.g., a third input) corresponding to a second FTA (different than the first FTA), based in part on a minimum threshold force needed to move at least one tooth of the patient (process portion 2706). The minimum threshold force may be a predetermined parameter, in that the minimum threshold force is known or can be determined prior to manufacturing of the device. In some embodiments, the minimum threshold force may correspond to a modification applied generally to the appliance (e.g., the same modification is applied to each individual arm), or a plurality of distinct modifications applied to each individual arm of the appliance. Additionally or alternatively, the minimum threshold may be determined based on factors common to all patients generally or on factors unique to a particular patient. For example, in some embodiments the minimum threshold considered may be based on the general anatomy of human teeth, e.g., with molars or larger teeth having a greater minimum threshold than that of incisors or smaller teeth. As another example, in some embodiments the minimum threshold considered may be based on the patient's particular gingiva (e.g., the gingival surface) surrounding individual ones of the patient's teeth.

In some embodiments, the method 2700 may omit process portion 2706 and only include a single FTA that considers the minimum threshold force. In such embodiments, the method 2700 may include obtaining first data corresponding to an OTA of a patient, and providing second data corresponding to an FTA of the patient, where the second data is based at least in part on a minimum threshold force. In some embodiments, the method 2700 can further comprise manufacturing the fixture and/or the appliance according to at least the data corresponding to the second FTA. Such manufacturing of the fixture and/or the appliance can correspond to the manufacturing processes described elsewhere herein.

### 2. Free Play Between the Appliance and Securing Member

As previously described, appliances of the present technology are configured to move a patient's teeth from the OTA along a path to a determined FTA. More specifically, individual arms of the appliance are configured to move a respective patient's tooth along a respective path from an original position to a respective final position. As also previously described, the individual arms are attached to a corresponding securing member (e.g., a bracket) adhered to individual teeth of the patient. Accordingly, the force applied via the individual arms of the appliance is provided to the corresponding securing member, and therein to the corresponding individual patient's tooth to cause repositioning. In this regard, because the securing members are a separate component from the appliance, there will often be some free play (e.g., gap, wiggle, or misfit, for example due to manufacturing tolerances) between each individual arm and the corresponding securing member. In some embodiments, the free play is the same for each individual arm and corresponding securing member. Moreover, in some embodiments the free play is different for at least one of the individual arms and corresponding securing member relative to other individual arms and corresponding securing members. As a result of the free play, the force provided via the individual arm may not be entirely transferred to the corresponding tooth because a portion of the force is lost via the free play. For example, if an individual arm is configured to move the corresponding tooth a given distance in a particular direction (e.g., the mesial, distal, occlusal, gingival, buccal, and/or lingual direction) and/or a given angle of rotation about a particular axis (e.g., about the mesiodistal axis, occlusogingival axis, and/or buccolingual axis), the free play can prevent the corresponding tooth from moving the full distance and/or the full angle of rotation.

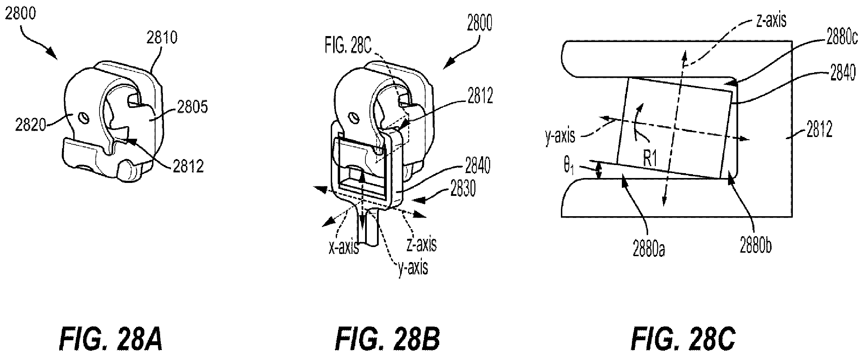

FIG. 28A is a perspective view of a securing member 2800, and FIG. 28B is a perspective view of a portion of an arm 2830 of an orthodontic appliance coupled to the securing member 2800 shown in FIG. 28A, in accordance with embodiments of the present technology. As shown in FIG. 28A, the securing member 2800 includes (i) a body region 2805 having a back side or surface 2810 to be attached to a patient's tooth, (ii) a slot or recess 2812 within the body region 2805 and configured to receive a portion of an orthodontic appliance or arm, and (iii) a moveable clip portion 2820 coupled to the body region 2805 configured to secure the portion of the appliance or arm 2830 when positioned within the slot 2812. The slot 2812 can form a three-sided or U-shaped opening. As shown in FIG. 28B, the arm 2830, or more particularly an attachment portion 2840 of the arm 2830, is disposed within the slot 2812. As also shown in FIG. 28B, the x-axis may generally correspond to the buccolingual axis, the y-axis may generally correspond to the occlusogingival axis, and the z-axis may generally correspond to the mesiodistal axis.

FIG. 28C is an enlarged cross-sectional side view of the securing member 2800 and arm 2830 shown in FIG. 28B, and is meant to further illustrate the previously described issue associated with the free play between the securing member 2800 and attachment portion 2840. As shown in FIG. 28C, the attachment portion 2840 is disposed within the slot 2812, but one or more gaps 2880 (individual gaps identified as 2880a-c) exist between the attachment portion 2840 and corresponding adjacent surfaces of the slot 2812. As such, rotation of the attachment portion 2840, e.g., in a first direction (R₁) causes the attachment portion 2840 to rotate relative to the slot 2812, and thus relative to the securing member 2800. That is, the initial rotation of the attachment portion 2840, as indicated by (θ₁), is not translated to the securing member 2800 and/or the corresponding tooth of the patient. Such a translation issue may occur (e.g., simultaneously occur) in one or more directions (e.g., the mesial, distal, occlusal, gingival, buccal, and/or lingual directions) and/or about one or more axes (e.g., the mesiodistal axis, occlusogingival axis, and/or buccolingual axis). For example, free play between the attachment portion and securing member may allow some rotation of an attachment portion relative to the securing member about the mesiodistal axis, the occlusogingival axis, and/or the buccolingual axis. As a result, such rotation would not be translated to the corresponding tooth because of the gap between the attachment portion and corresponding securing member. As another example, free play between the attachment portion and securing member may allow some initial movement of the attachment portion relative to the securing member along the mesiodistal axis, the occlusogingival axis, and/or the buccolingual axis . As a result, such movement would not be translated to the corresponding tooth because of the gap between the attachment portion and corresponding securing member.

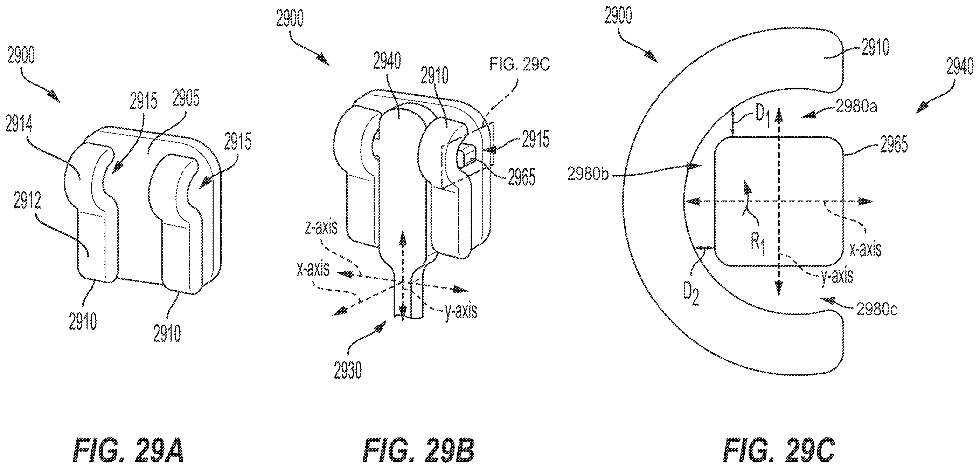

FIG. 29A is a perspective view of another securing member 2900 configured in accordance with embodiments of the present technology, and is another example of the above-described concepts regarding free play between an arm of an appliance and a securing member. As shown in FIG. 29A, the securing member 2900 includes (i) a body region 2905 having a first, back side or surface to be attached to a patient's tooth and a second, opposing side or surface, and (ii) one or more coupling arms 2910 attached to the second side of the body region 2905. Each coupling arm 2910 can include a first, elongate portion 2912 fixed to the body region 2905, and a second, coupling portion 2914 extending from the first portion 2912 and that is partially spaced apart from the body region 2905. The coupling portion 2914 can define a slot or opening 2915 configured to receive and partially surround a portion of an orthodontic appliance or arm (as shown in FIG. 29B). In some embodiments, the securing member 2900 may be a commercially-available 2D^{®} Lingual Bracket manufactured by Bernhard Foerster GmbH.

FIG. 29B is a perspective view of a portion of an arm 2930 of an orthodontic appliance coupled to the securing member 2900 shown in FIG. 29A. As shown in FIG. 29B, the arm 2930 includes an attachment portion or end portion 2940 having a region or extension 2965 disposed within the slot 2915. As also shown in FIG. 29B, when the arm 2930 and securing member 2900 are installed within a patient's mouth, the x-axis may generally correspond to the buccolingual axis, the y-axis may generally correspond to the occlusogingival axis, and the z-axis may generally correspond to the mesiodistal axis.

FIG. 29C is an enlarged side view of the securing member 2900 and portion of the attachment portion 2940 shown in FIG. 29B, and is meant to further illustrate the previously described issue associated with the free play between the securing member 2900 and arm 2930. As shown in FIG. 29C, the region 2965 of the attachment portion 2940 is disposed adj acent the securing member 2900 such that one or more gaps 2980 (individual gaps identified as 2980a, 2980b, 2980c) exist between the region 2965 and corresponding adjacent surfaces of the coupling arm 2910 of the securing member 2900. As such, free play between the attachment portion 2940 and securing member 2900 may allow some movement of the region 2965 relative to the coupling arm 2910 along the mesiodistal axis, the occlusogingival axis, and/or the buccolingual axis. For example, as shown in FIG. 29C, free play between the attachment portion 2940 and securing member 2900 may allow movement of the region 2965 relative to the coupling arm 2910 by a distance (D₁) along the y-axis and/or a distance (D₂) along the x-axis. As a result, such movement would not be translated to the corresponding tooth because of the one or more gaps 2980. As another example, rotation of the region 2965 in a first direction (R₁) can cause the region 2965 to rotate relative to the coupling arm 2910, and thus the securing member 2900. That is, the initial rotation of the region 2965 may not be translated to the securing member 2900 and/or the corresponding tooth of the patient. Such a translation issue may occur (e.g., simultaneously occur) in one or more directions (e.g., the mesial, distal, occlusal, gingival, buccal, and/or lingual directions) and/or about one or more axes (e.g., the mesiodistal axis, occlusogingival axis, and/or buccolingual axis). For example, free play between the attachment portion 2940 and securing member 2900 may allow some rotation of the attachment portion 2940 relative to the securing member 2900 about the mesiodistal axis, the occlusogingival axis, and/or the buccolingual axis. As a result, such rotation would not be translated to the corresponding tooth because of the gap between the attachment portion 2940 and corresponding securing member 2900.

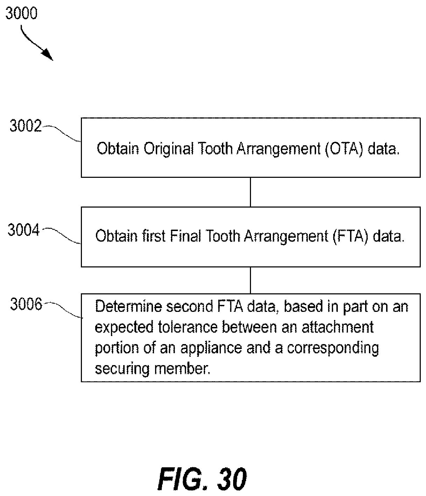

Embodiments of the present technology can mitigate this issue (as described with reference to FIGS. 28A-29C) associated with free play between the attachment portion and securing member by considering free play when designing the orthodontic device. FIG. 30 is a flow diagram of a method 3000 for generating design parameters and/or manufacturing an orthodontic appliance or related fixture, in accordance with embodiments of the present technology. The method 3000 includes obtaining data corresponding to an OTA of a patient (process portion 3002), and obtaining data corresponding to a first FTA of the patient (process portion 3004). As described elsewhere herein, the OTA can be based on a scan of the patient's teeth, and the FTA can be determined and/or provided by the operator based on the OTA and a desired optimal positioning of the teeth.

The method 3000 can further include determining data corresponding to a second FTA (different than the first FTA), based in part on an expected free play between an attachment portion of an appliance and a corresponding securing member (process portion 3006). In some embodiments, the expected free play may be a predetermined parameter (e.g., based on the attachment portion and securing member used), in that the expected free play is known or can be determined prior to manufacturing of the appliance. In some embodiments, the expected free play may correspond to a dimension or angle that causes the design (e.g., shape, thickness, type of spring, etc.) of the appliance or portions thereof (e.g., the arms, biasing portions, attachment portions, etc.) to be modified. For example, if the expected free play between an attachment portion and securing member is 15° in a first direction (e.g., a direction about the mesiodistal axis, occlusogingival axis, and/or buccolingual axis) and the total rotation in the first direction required for a particular tooth (e.g., from the OTA to the first FTA) is 45°, then the arm (e.g., the attachment portion) of the appliance may be designed to rotate 60° in the first direction. In doing so, the arm or attachment portion, when coupled to the corresponding tooth via the corresponding securing member, will rotate 15° relative to the corresponding securing member, and then will rotate 45° along with the corresponding securing member and corresponding tooth, as desired. As previously described, the free play may be adjusted in multiple directions and/or about multiple axes simultaneously for an individual arm. Additionally or alternatively, the free play for each arm of the appliance may be uniquely adjusted relative to the other arms.

In some embodiments, the method 3000 may omit process portion 3006 and only include a single FTA that considers the expected free play. In such embodiments, the method 3000 may include receiving first data corresponding to an OTA of a patient, and providing second data corresponding to an FTA of the patient, where the second data is based at least in part on the expected free play between an attachment portion of an appliance and a corresponding securing member or portion thereof.

In some embodiments, the method 3000 can further comprise manufacturing the fixture and/or the appliance according to at least the data corresponding to the second FTA. Such manufacturing of the fixture and/or the appliance can correspond to the manufacturing processes described elsewhere herein.

### B. Accounting for Material Properties of the Appliance

Appliances of the present technology are configured to move a patient's teeth from the OTA along a path to a determined and optimal FTA. As previously described, an appliance may be manufactured to have a configuration that in its unloaded or unstressed state generally corresponds to the FTA of the patient's teeth. The appliance is implanted within a patient's mouth and individual arms of the appliance are coupled to corresponding securing members adhered to the patient's teeth. As the individual arms are coupled to the corresponding securing member on the patient's teeth in the OTA, the appliance assumes a loaded or stressed configuration. In this loaded configuration, the appliance is often in its most stressed state and thus is most likely, if at all, to experience plastic deformation. If plastic deformation occurs, the individual arm may not transition from the OTA to the FTA along the desired path and/or may be unable to provide the necessary force upon the corresponding tooth. More generally, plastic deformation will limit treatment efficacy of the patient's teeth and prevent or inhibit the teeth from reaching the FTA.

Embodiments of the present technology can mitigate these issues by considering plastic deformation, or more particularly avoiding plastic deformation, when designing the orthodontic appliance and/or fixture. As previously described, embodiments of the present technology may determine the path of a patient's teeth from the OTA to the FTA. As such, the path of the appliance from a first configuration generally corresponding to the OTA to a second configuration generally corresponding to the FTA is also known. Based on the expected path of the individual arms of the appliance and the material(s) used to form the appliance (e.g., the arms, biasing portions, attachment portion, etc.), embodiments of the present technology can determine, and if necessary avoid, the appliance's yield strength at which plastic deformation occurs for each arm. For example, embodiments of the present technology may be able to simulate the stress to be experienced by individual arms of an appliance when in the first configuration generally corresponding to the OTA, or any other configuration between the OTA and FTA. If the stress experienced by one of the arms in any such a configuration is expected to be above the yield strength for the material of the arm, embodiments of the present technology may then adjust one or more parameters of the arm such that the yield strength is not exceeded. In some embodiments, altering one or more parameters of the arm can include altering the shape, configuration, and/or dimension (e.g., length, width, and/or thickness) of any portion of the appliance (e.g., the anchor, arms, and/or biasing portions). Altering one or more of these parameters can increase the yield strength of the arm to be greater than the highest stress expected to be experienced. As but one example, certain biasing portions (e.g., spring designs) can experience a greater stress than other biasing portions. Accordingly, if movement of an arm from the OTA to the FTA is determined to cause the yield strength of the arm to be exceeded, embodiments of the present technology may alter the biasing portion of the arm to increase its yield strength and thereby avoid plastic deformation.

Additionally or alternatively to altering a portion of the appliance in response to determining that a yield strength may be exceeded, embodiments of the present technology may alter the path of a patient's tooth from the OTA such that the yield strength of the appliance is not exceeded along the path. That is, if moving a patient's tooth from an OTA to an FTA along a first path will result in yield strength being exceeded, embodiments of the present technology may instead alter the appliance, or more particularly the corresponding arm of the appliance, such that the patient's tooth is moved from the OTA to the FTA along a second path, different than the first path, which will result in the yield strength not being exceeded.

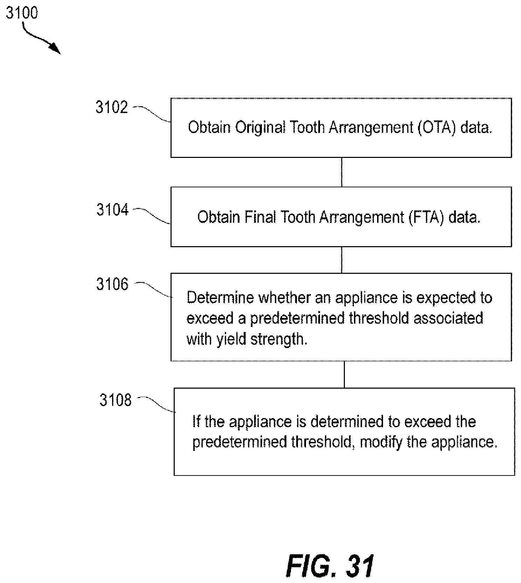

FIG. 31 is a flow diagram of a method 3100 for generating design parameters and/or manufacturing an orthodontic appliance or related fixture, in accordance with embodiments of the present technology. The method 3100 includes obtaining data corresponding to an OTA of a patient (process portion 3102), and obtaining data corresponding to an FTA of the patient (process portion 3104). As described elsewhere herein, the OTA can be based on a scan of the patient's teeth, and the FTA can be determined and/or provided by the operator based on the OTA and a desired positioning of the teeth.

The method 3100 can further include determining whether an appliance is expected to exceed a predetermined threshold associated with yield strength (process portion 3106). In some embodiments, process portion 3106 can include determining whether an appliance configured to transition from a first configuration (e.g., corresponding to the OTA) toward a second configuration (e.g., corresponding to the FTA) is expected to exceed a yield strength of the appliance. Additionally or alternatively, determining whether the appliance is expected to exceed the yield strength can include determining whether any portion of the appliance (e.g., individual arms, biasing portions, attachment portions, etc.) is expected to exceed the yield strength. As such, embodiments of the present technology may determine the stress experienced by the appliance or any portion thereof when in the first configuration (e.g., at the OTA), the second configuration (e.g., at the FTA), and/or a plurality of discrete points along the path between the first and second configurations (e.g., at intermediate tooth arrangements (ITA)).

In some embodiments, determining whether the appliance is expected to exceed the yield strength can be based on hysteresis behavior, e.g., of the material(s) forming the appliance. With regard to the present technology, hysteresis can alter the path taken by an arm of the appliance depending on whether the arm is experiencing compression or tension along its path from the OTA to the FTA. For example, an arm comprising Nitinol or nickel-titanium alloy may follow a different stress-strain curve in compression than the arm would in tension. Accordingly, in addition to or in lieu of the determining the expected stress of the appliance or any portion thereof at discrete points between and including the OTA and FTA, embodiments of the present technology may consider the configuration of the appliance or any portion thereof prior to the appliance assuming these discrete points.

In some embodiments, the method 3100 can include, if the appliance is expected to exceed the predetermined threshold, modifying the appliance such that the yield strength is not exceeded (process portion 3108). Modifying the appliance in such a manner can include altering (i) the shape, configuration, and/or dimension (e.g., length, width, and/or thickness) of the appliance (e.g., the anchor, arms, and/or biasing portions), and/or (ii) the material of the arm. Altering one or more of these parameters can increase the yield strength of the arm to be greater than the highest stress expected to be experienced, thereby ensuring the appliance (or any portion thereof) is not plastically deformed in a manner that limits treatment efficacy of the patient's teeth. As an example, if it is determined that an appliance would exceed a predetermined threshold, a single biasing portion (e.g., spring) of the appliance could be replaced with two or more lower load biasing portions. Such a replacement may be performed for each arm of the appliance that is expected to exceed the predetermined threshold.

In some embodiments, the method 3100 can further comprise manufacturing the fixture and/or the appliance. Such manufacturing of the fixture and/or the appliance can correspond to the manufacturing processes described elsewhere herein.

### C. Accounting for Manufacturing Irregularities

As previously described, the 3D configuration of the orthodontic appliance can be created by bending a substantially planar configuration of the appliance to assume the 3D configuration that generally corresponds to the FTA. In some embodiments, as described elsewhere herein, this bending is accomplished by attaching (e.g., via ligature wire) a substantially planar configuration of the appliance to a heat treatment fixture that generally corresponds to the FTA (potentially with slight modifications, as previously described), and then heat treating the substantially planar configuration such that the appliance assumes and remains in the 3D configuration after heat treatment. In some embodiments, the appliance is made at least in part from a superelastic material (e.g., Nitinol). In such embodiments, the heat treatment process previously described may be relatively mild to ensure the superelastic material after heat treatment substantially maintains its elastic properties. However, as a result of such mild heat treatment, the appliance in the 3D configuration or portions thereof can tend to retract partially back toward the previous substantially planar configuration after the heat treatment process is complete and the appliance is detached from the fixture. For example, individual arms of the appliance in the 3D configuration may move in a direction (e.g., a labial, buccal, gingival, occlusal, mesial, and/or distal direction) or about an axis (e.g., a mesiodistal axis, occlusogingival axis, and/or buccolingual axis) after the appliance is detached from the fixture after heat treatment. As a result, the heat treated 3D configuration of the appliance may not precisely correspond to the shape of the fixture, or more generally, the FTA. Such a discrepancy may cause individual arms of the appliance to apply a force (e.g., a direction and/or magnitude) different than the intended force and thus prevent the patient's teeth from reaching the desired FTA.

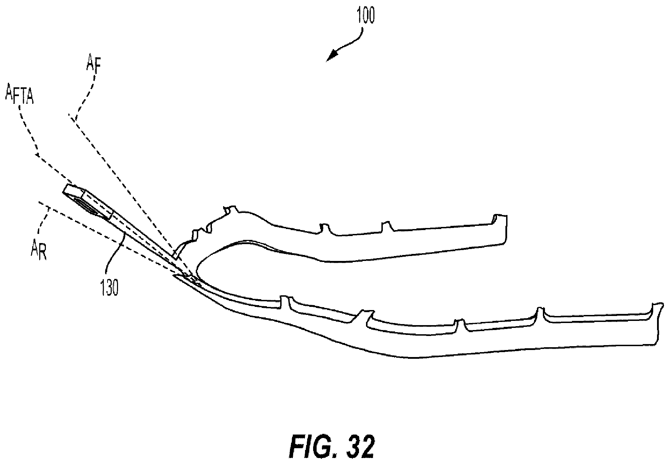

FIG. 32 is a side perspective view of an orthodontic appliance 100 in accordance with embodiments of the present technology, and is meant to further illustrate the issue regarding an appliance retracting after heat treatment. For illustrative purposes, only a single arm 130 of the appliance 100 is shown, but a person of ordinary skill in the art will appreciate that the principles described herein can apply to any arm 130 of the appliance (e.g., the appliance 100 shown in FIG. 16). As shown in FIG. 32, the arm 130 extends along an axis (A_{FTA}), which corresponds to the FTA of the patient's teeth. As previously described, due at least in part to the material of the appliance, when the appliance is heat treated over the fixture and detached therefrom, the appliance tends to retract to a previous position other than that of the FTA. As shown in FIG. 32, axis (A_{R}) corresponds to the arrangement the appliance would have after retracting, e.g., from the axis (A_{FTA}). That is, if the fixture was heat set while the arm 130 was positioned along axis A_{FTA}, the arm 130 of the resulting appliance after retraction would be positioned along axis (A_{R}), which is deflected away from the axis (A_{FTA}) in which it was heat set and/or toward a more planar configuration. Such an arm, or appliance generally, would be spaced apart from the axis (A_{FTA}) and thus, when coupled to a securing member adhered to a patient's tooth, would provide a force different than that intended and thus prevent the patient's teeth from reaching the FTA.

Embodiments of the present technology can mitigate this issue by considering the material properties of the appliance and irregularities associated with heat treatment, or more generally the manufacturing process, when designing the orthodontic appliance and/or fixture. For example, embodiments of the present technology may design and/manufacture an appliance to have a configuration that retracts after heat treatment to have a configuration generally corresponding to the FTA. As shown in FIG. 32, axis (A_{F}) corresponds to the arrangement of the fixture and/or the appliance after heat treatment and before the appliance is detached from the fixture. After heat treatment and after being detached from the fixture, the arm 130 of the appliance 100 may generally retract from the axis (A_{F}) to the axis (A_{FTA}), which corresponds to the FTA of the patient's teeth. Accordingly, the axis (A_{F}) can correspond to a position that enables the arm 130 of the appliance 100 to have an arrangement corresponding to the FTA for the corresponding tooth after the appliance has been heat treated, while also maintaining the desirable elastic properties of the material, e.g., to reposition the patient's teeth to the FTA. Stated differently, if a fixture is designed to have an arrangement corresponding to the axis (A_{F}), the resulting appliance formed via the fixture after the expected retraction can have an arrangement corresponding to or positioned along the axis (A_{FTA}). In some embodiments, the amount of retraction from the axis (A_{F}) to the axis (A_{FTA}) may be the same or different than the amount of retraction from the axis (A_{FTA}) to the axis (A_{R}). Accordingly, this varying amount of retraction can be considered during the manufacturing process, e.g., when designing the fixture.

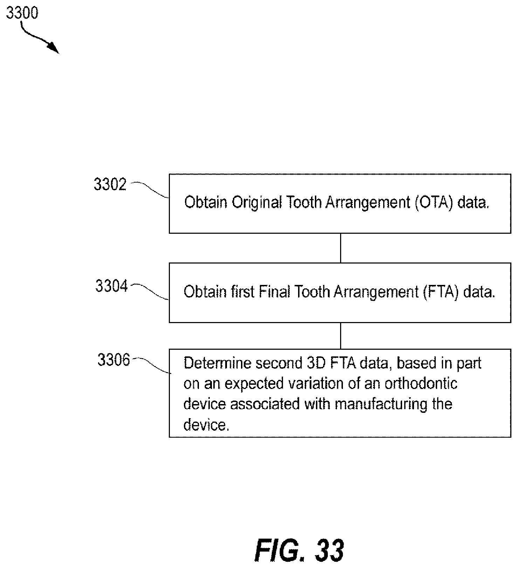

FIG. 33 is a flow diagram of a method 3300 for generating design parameters and/or manufacturing an orthodontic appliance or related fixture, in accordance with embodiments of the present technology. The method 3300 includes obtaining data corresponding to an OTA of a patient (process portion 3302), and obtaining data corresponding to a first FTA of the patient (process portion 3304). As described elsewhere herein, the OTA can be based on a scan of the patient's teeth, and the FTA can be determined and/or provided by the operator based on the OTA and a desired positioning of the teeth.

The method 3300 can further include determining data corresponding to a second FTA (different than the first FTA), based on an expected variation of an orthodontic device associated with manufacturing the device (process portion 3306). The expected variation can correspond to the expected different position or arrangement of the retracted appliance after heat treatment (as previously described) relative to the position or arrangement of the FTA. For example, if the position of a particular arm of the retracted appliance is spaced apart (e.g., in a lingual, occlusal, and/or distal direction) from the position of the corresponding arm in the FTA, then the expected variation, and therein the data corresponding to the second FTA, may correspond to the positional difference between the arm in the retracted position and the arm in the second FTA position. The expected variation may be a predetermined parameter, in that the expected variation is known or can be determined prior to manufacturing of the appliance. In some embodiments, the expected variation may be based on one or more factors including (i) the shape, configuration, and/or dimension (e.g., length, width, and/or thickness) of the appliance (e.g., the anchor, arms, and/or biasing portions), (ii) the material(s) of the appliance, (iii) the type of heat treatment applied or expected to be applied (e.g., maximum temperature of the heat treatment, elapsed time of heat treatment, etc.), and/or (iv) other aspects of the particular patient's dentition. In some embodiments, the expected variation may be unique to each arm of the appliance. As such, the expected variation may correspond to different values or modifications made to each arm (e.g., each biasing portion, attachment portion, etc.).

In some embodiments, the method 3300 may omit process portion 3306 and only include a single FTA that considers the expected variation of the appliance. In such embodiments, the method 3300 may include receiving first data corresponding to an OTA of a patient, and providing second data corresponding to an FTA of the patient, where the second data is based in part on the expected variation of the appliance or fixture associated with manufacturing, as described above.

In some embodiments, the method 3300 can further comprise manufacturing the fixture and/or the appliance according to at least the data corresponding to the second FTA. Such manufacturing of the fixture and/or the appliance can correspond to the manufacturing processes described elsewhere herein.

### D. Accounting for Expected Teeth Movement After Repositioning

Appliances of the present technology are configured to reposition a patient's teeth from the OTA along a path to a determined and optimal FTA. After reaching the FTA, a patient's teeth may experience orthodontic relapse and move toward their previous position (e.g., the OTA) and thus away from their optimal position. For example, the patient's teeth may generally move in a partial buccal direction and/or a partial occlusal direction after the teeth are repositioned via the appliance to the FTA. As such, the patient's teeth after relapse may no longer resemble the FTA. Retainers or other devices may be used to prevent such relapse, however for multiple reasons (e.g., lack of patient compliance) these devices are often ineffective.

Embodiments of the present technology can mitigate these issues by considering orthodontic relapse when designing the orthodontic appliance and/or fixture. As previously described, the 3D configuration of the orthodontic appliance can be created by bending a substantially planar configuration of the appliance to assume a 3D configuration that generally corresponds to the FTA. In some embodiments, this bending is accomplished by attaching a substantially planar configuration of the appliance to a fixture that generally corresponds to the FTA (with slight modifications, as previously described), and then heat treating the substantially planar configuration such that the appliance assumes and remains in the 3D configuration. In order to account for orthodontic relapse after repositioning a patient's teeth to the FTA, embodiments of the present technology can determine the amount of relapse expected to occur, and alter the design of the appliance and/or fixture accordingly.

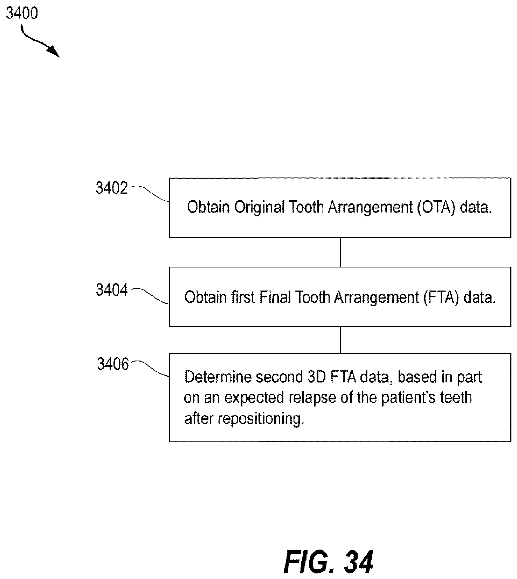

FIG. 34 is a flow diagram of a method 3400 for generating design parameters and/or manufacturing an orthodontic appliance or related fixture, in accordance with embodiments of the present technology. The method 3400 includes obtaining data corresponding to an OTA of a patient (process portion 3402), and obtaining data corresponding to a first FTA of the patient (process portion 3404). As described elsewhere herein, the OTA can be based on a scan of the patient's teeth, and the FTA can be determined and/or provided by the operator based on the OTA and a desired optimal positioning of the teeth.

The method 3400 can further include determining data corresponding to a second FTA (different than the first FTA), based in part on an expected relapse of the patient's teeth after repositioning, e.g., to the first FTA and/or second FTA (process portion 3406). The second FTA may correspond to a tooth arrangement wherein the expected relapse causes the patient's teeth to transition from the second FTA to the first FTA, which is the optimal tooth arrangement for the patient. As a result, in some embodiments an appliance having a configuration generally corresponding to the second FTA may have individual arms with positions that are spaced apart in a particular direction (e.g., a labial, buccal, gingival, occlusal, mesial, and/or distal direction) and/or about a particular axis (e.g., a mesiodistal, occlusogingival, and/or buccolingual) from the positions of corresponding individual arms of an appliance having a configuration generally corresponding to the first FTA.

In some embodiments, the expected relapse may be a predetermined parameter, in that the expected relapse is known or can be determined prior to manufacturing the appliance and/or fixture. Determining the expected relapse can be based on the second FTA, the first FTA, the OTA, and/or other factors specific to the patient's dentition. Additionally or alternatively, the expected relapse may differ for each individual tooth. For example, smaller teeth (e.g., incisors) may experience more relapse than larger teeth (e.g., molars). As such, individual portions of the appliance (e.g., the anchor, arms, biasing portions, attachment portions, etc.) and/or the fixture corresponding to individual teeth may be adjusted differently and distinctly based on the expected relapse for that particular portion. For example, modifications made to the smaller teeth, which are expected to experience more relapse, may be smaller than those made to the larger teeth, which are expected to experience less relapse.

In some embodiments, the method 3400 may omit process portion 3406 and only include a single FTA that considers the expected relapse of the appliance. In such embodiments, the method 3400 may include receiving first data corresponding to an OTA of a patient, and providing second data corresponding to an FTA of the patient, where the second data is based in part on the expected relapse of the patient's teeth after repositioning.

In some embodiments, the method 3400 can further comprise manufacturing the fixture and/or the appliance according to at least the data corresponding to the second FTA. Such manufacturing of the fixture and/or the appliance can correspond to the manufacturing processes described elsewhere herein.

Any of the processes detailed herein can be used with any of the other processes detailed herein. For example, any of the processes described with respect to FIGS. 19-25 can be used with any of the processes described with respect to FIGS. 26-34.

### Conclusion

Although many of the embodiments are described above primarily with respect to systems, devices, and methods for orthodontic appliances positioned on a lingual side of a patient's teeth, the technology is applicable to other applications and/or other approaches, such as orthodontic appliances positioned on a facial side of the patient's teeth. Moreover, other embodiments in addition to those described herein are within the scope of the technology. Additionally, several other embodiments of the technology can have different configurations, components, or procedures than those described herein. A person of ordinary skill in the art, therefore, will accordingly understand that the technology can have other embodiments with additional elements, or the technology can have other embodiments without several of the features shown and described above with reference to FIGS. 1A-34.

The descriptions of embodiments of the technology are not intended to be exhaustive or to limit the technology to the precise form disclosed above. Where the context permits, singular or plural terms may also include the plural or singular term, respectively. For example, embodiments described herein as using multiple coupling arms may just as well be modified to include fewer (e.g., one) or more (e.g., three) coupling arms. Although specific embodiments of, and examples for, the technology are described above for illustrative purposes, various equivalent modifications are possible within the scope of the technology, as those skilled in the relevant art will recognize. For example, while steps are presented in a given order, alternative embodiments may perform steps in a different order. The various embodiments described herein may also be combined to provide further embodiments.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

### The invention will now be defined by reference to the following clauses

1. A method for designing an orthodontic appliance for repositioning a tooth of a patient, the method comprising:
   obtaining an appliance digital model characterizing the orthodontic appliance in an initial configuration;
   obtaining a fixture digital model characterizing a fixture for setting a shape of the appliance; and
   performing a finite element analysis (FEA) to virtually deform the appliance digital model based on the fixture digital model.
2. The method of Clause 1, wherein the fixture digital model comprises:
   a gingival portion having a shape substantially corresponding to a surface of the patient's gingiva; and
   at least one securing portion carried by the gingival portion and configured to retain a portion of the appliance.
3. The method of Clause 1, wherein performing the FEA comprises causing at least one portion of the appliance digital model to substantially conform to the fixture digital model.
4. The method of Clause 1, wherein the appliance comprises an anchor and an arm extending away from the anchor, the arm comprising a proximal portion at the anchor and a distal portion configured to be secured to an orthodontic bracket, and wherein performing the FEA comprises positioning a distal portion of an arm of the appliance digital model at or within the securing portion of the fixture digital model.
5. The method of Clause 1, wherein the appliance comprises an anchor and an arm extending away from the anchor, and wherein performing the FEA comprises applying a non-zero displacement to an anchor of the appliance digital model.
6. The method of Clause 1, wherein the appliance is substantially planar in the initial configuration.
7. A method for designing an orthodontic appliance for repositioning a tooth of a patient, the method comprising:
   obtaining an appliance digital model characterizing the orthodontic appliance in a pre-installation configuration;
   obtaining an anatomy digital model characterizing a patient's teeth and gingiva in an original arrangement; and
   performing an FEA to virtually deform the appliance digital model based on the anatomy digital model.
8. The method of Clause 7, the appliance comprises an anchor and an arm extending away from the anchor, the arm comprising a proximal portion at the anchor and a distal portion configured to be secured to an orthodontic bracket, and wherein performing the FEA comprises causing the distal portion of the arm to be positioned at or adjacent to one of the patient's teeth.
9. The method of Clause 7, wherein the appliance has a substantially three-dimensional (3D) shape in the pre-installation configuration.
10. The method of Clause 7, further comprising evaluating the deformed appliance digital model.
11. The method of Clause 10, wherein evaluating the deformed appliance digital model comprises determining whether the deformed appliance digital model impinges on the gingiva or is spaced apart from the gingiva by greater than a predetermined threshold.
12. The method of Clause 10, wherein evaluating the deformed configuration comprises determining whether any portion of the deformed appliance digital model exceeds an elastic strain limit.
13. The method of Clause 10, wherein evaluating the deformed configuration comprises determining a difference between a force and/or moment applied to the teeth by the deformed appliance and an intended force and/or moment.
14. The method of Clause 10, further comprising, based on the evaluation, modifying the appliance digital model, wherein modifying the appliance digital model comprises changing at least one of a shape of an arm of the appliance, a shape of an anchor of the appliance, or a shape of the appliance in the pre-installation configuration.
15. A method for designing an orthodontic appliance for repositioning a tooth of a patient, the method comprising:
   obtaining a preliminary appliance digital model virtually representing the appliance in a preliminary configuration;
   obtaining a heat treatment fixture digital model, the heat treatment fixture digital model characterizing a geometry of a heat treatment fixture for shape-setting an appliance, wherein the heat treatment fixture comprises a gingival surface having a shape substantially corresponding to a shape of a gingival surface of the patient and a securing portion configured to releasably retain a portion of the appliance;
   performing a first FEA to virtually deform the preliminary appliance digital model based on the heat treatment fixture digital model;
   obtaining an intended appliance digital model virtually representing the appliance in a three-dimensional configuration with a geometry based at least in part on the heat treatment fixture digital model;
   obtaining an original tooth arrangement (OTA) digital model virtually representing a patient's teeth and gingiva in an original arrangement;
   performing a second FEA to virtually deform the intended appliance digital model based on the OTA digital model; and
   obtaining a deformed intended appliance digital model and an analysis result.
16. The method of Clause 15, wherein the appliance is substantially planar in the preliminary configuration.
17. The method of Clause 15, wherein performing the first FEA comprises:
   discretizing at least one of the preliminary appliance digital model and the heat treatment fixture digital model into a plurality of finite elements and a plurality of nodes;
   assigning material properties to at least one of the preliminary appliance digital model and the heat treatment fixture digital model;
   defining a contact interaction between the preliminary appliance digital model and the heat treatment fixture digital model;
   assigning boundary conditions to at least one of the preliminary appliance digital model and the heat treatment fixture digital model;
   defining an analysis parameter; and
   running the FEA until an exit condition is reached.
18. The method of Clause 17, wherein assigning the boundary conditions includes at least one of assigning a non-zero displacement a portion of the planar appliance digital model or defining a relationship between an orientation of a portion of the planar appliance digital model and a base plane of a securing portion of the heat treatment fixture.
19. The method of Clause 15, wherein performing the second FEA comprises:
   discretizing at least one of the intended appliance digital model and the OTA digital model into a plurality of finite elements and a plurality of nodes;
   assigning material properties to at least one of the intended appliance digital model and the OTA digital model;
   defining a contact interaction between the intended appliance digital model and the OTA digital model;
   assigning boundary conditions to at least one of the intended appliance digital model and the OTA digital model;
   defining an analysis parameter; and
   running the FEA until an exit condition is reached.
20. The method of Clause 19, wherein assigning the boundary conditions comprises assigning a displacement to a portion of the intended appliance digital model, the displacement based at least in part on a movement of the patient's tooth from the original arrangement to a desired final arrangement.
21. The method of Clause 20, wherein the analysis result comprises at least one of a strain in the deformed intended appliance digital model or a distance between the deformed intended appliance digital model and the gingival surface of the patient.
22. The method of Clause 20, wherein the orthodontic appliance comprises an anchor and at least one arm extending away from the anchor, the arm comprising a proximal portion at the anchor and a distal portion configured to be secured to an orthodontic bracket.
23. The method of Clause 22, wherein performing the first FEA causes the anchor of the appliance to be positioned at or adjacent to the gingival surface of the heat treatment fixture digital model.
24. The method of Clause 22, wherein performing the second FEA causes the distal portion of the arm of the appliance to be positioned at or adjacent to one of the patient's teeth.
25. A method for designing an orthodontic appliance for repositioning a tooth of a patient, the method comprising:
   obtaining an OTA digital model of a patient's teeth and gingiva in an original arrangement, the OTA digital model comprising original position data of a tooth to be repositioned by the orthodontic appliance when installed in the patient's mouth;
   obtaining an FTA digital model characterizing the patient's teeth and gingiva in a desired final arrangement, the FTA digital model comprising final position data of the tooth;
   determining displacement data characterizing a displacement between the original position data of the tooth and the final position data of the tooth;
   obtaining a heat treatment fixture digital model based on at least one of the OTA digital model or the FTA digital model;
   obtaining a 3D template digital model based on the heat treatment fixture digital model;
   obtaining a planar template digital model, wherein the planar template digital model is a substantially planar configuration of the 3D template digital model;
   obtaining a planar appliance digital model based on the planar template digital model;
   obtaining an intended appliance digital model, wherein the intended appliance digital model characterizes the orthodontic appliance in 3D configuration based on the heat treatment fixture digital model;
   performing an FEA on the OTA and intended appliance digital models to deform the intended appliance digital model based on the displacement data; and evaluating an analysis result of the virtual deformation.
26. The method of Clause 25, wherein the displacement data comprises three translations and three rotations.
27. The method of Clause 25, further comprising modifying the heat treatment fixture digital model based on the intended appliance digital model.
28. The method of Clause 27, wherein modifying the heat treatment fixture digital model comprises defining a tangent relationship between a gingival surface of the heat treatment fixture digital model and a gingival-facing surface of the intended appliance digital model.
29. The method of Clause 25, further comprising manufacturing at least one of the planar template digital model, the heat treatment fixture digital model, or the intended appliance digital model.
30. The method of Clause 25, wherein the orthodontic appliance comprises an anchor and an arm extending away from the anchor, the arm comprising a proximal portion at the anchor and a distal portion configured to be secured to an orthodontic bracket.

## Claims

1. A method for designing an orthodontic appliance for repositioning a tooth of a patient, the method comprising:
obtaining an appliance digital model characterizing the orthodontic appliance in a pre-installation configuration;
obtaining an anatomy digital model characterizing a patient's teeth and gingiva in an original or intermediate arrangement; and
performing an FEA to virtually deform the appliance digital model based on the anatomy digital model.

2. The method of Claim 1, wherein the appliance digital model comprises an anchor and an arm extending away from the anchor, the arm comprising a proximal portion at the anchor and a distal portion configured to be secured to an orthodontic bracket, and wherein performing the FEA comprises causing the distal portion of the arm to be positioned at or adjacent to one of the patient's teeth.

3. The method of Claim 1, wherein the anatomy digital model comprises position data of the tooth to be repositioned by the orthodontic appliance when installed in the patient's mouth.

4. The method of Claim 3, wherein the data is first position data and the method further comprises:
obtaining a desired digital model characterizing the patient's teeth and gingiva in a desired second configuration, the desired digital model comprising second position data of the tooth,
determining displacement data characterizing a displacement between the first position data of the tooth and the second position data of the tooth, and
wherein performing the FEA is based on the displacement data.

5. The method of any one of Claims 1 to 4, further comprising obtaining a deformed intended appliance digital model from the FEA, wherein the deformed intended appliance digital model virtually represents the orthodontic appliance once it has been installed in a patient's mouth with the teeth and gingiva in the original or intermediate arrangement.

6. The method of any one of Claims 1 to 5, further comprising modifying a fixture digital model based on an analysis result of the FEA virtual deformation.

7. The method of any one of Claims 1 to 6, further comprising modifying the appliance digital model based on an analysis result of the FEA virtual deformation.

8. The method of Claim 7, wherein modifying the appliance digital model comprises modifying a shape and/or configuration of an anchor and/or arms of the appliance and/or a geometry of the pre-installation configuration of the appliance.

9. The method of any one of Claims 1 to 8, further comprising obtaining an analysis result based on the FEA, wherein the analysis result is a measure of strain in the appliance digital model.

10. The method of any one of Claims 1 to 9, further comprising obtaining an analysis result based on the FEA, wherein the analysis result is identification of portions of the orthodontic appliance that may impinge on a patient's gingiva.

11. The method of any one of Claims 1 to 10, further comprising obtaining an analysis result based on the FEA, wherein the analysis result is a local distance between a portion of the appliance digital model and a portion of the lingual surface of the patient's gingiva in the original or intermediate arrangement, as represented virtually in the anatomy digital model, that exceeds a predetermined threshold.

12. The method of any one of Claims 1 to 11, further comprising obtaining an analysis result based on the FEA, wherein the analysis result is a local distance between a portion of the appliance digital model and a portion of the lingual surface of the patient's gingiva in the original or intermediate arrangement, as represented virtually in the anatomy digital model, that is less than a predetermined threshold.

13. The method of Claim 11 or Claim 12, further comprising increasing or decreasing a thickness of a gingiva of the anatomy digital model based on the analysis result.

14. The method of any one of Claims 1 to 13, wherein the FEA virtual deformation models the real-world behavior of an othodontic appliance fabricated based on the appliance digital model when the orthodontic appliance is installed in a patient's mouth with the teeth in the original or intermediate arrangement.

15. The method of any one of Claims 1 to 14, wherein the anatomy digital model includes securing portions in the positions of a patient's teeth.
